# EUROPEAN PATENT APPLICATION

(11) **EP 4 825 879 A1**
(43) Date of publication of application: **21.10.2026**
(21) Application number: 25747766.1
(22) Date of filing: 23.01.2025
(51) Int. Cl.: C07K 19/00, A61K 39/395, C12N 15/62, A61K 38/20, A61K 38/17, A61K 47/68, A61P 35/00, A61P 31/00, A61P 37/02

(54) **BISPECIFIC ANTIBODY-CYTOKINE FUSION PROTEIN AND USE THEREOF**

(30) Priority: 31.01.2024 CN 202410134944
(71) Applicant: Sichuan Huiyu Pharmaceutical Co., Ltd., Neijiang, Sichuan 641000 (CN); Sichuan Huiyu Seacross Pharmaceutical Technology Co., Ltd., Chengdu, Sichuan 610200 (CN)
(72) Inventor: ZHENG, Xuan, Neijiang, Sichuan 641000 (CN); DING, Zhao, Neijiang, Sichuan 641000 (CN); WEI, Tao, Neijiang, Sichuan 641000 (CN); CHEN, Yan, Neijiang, Sichuan 641000 (CN); DONG, Guibin, Neijiang, Sichuan 641000 (CN); LI, Chunxia, Neijiang, Sichuan 641000 (CN); ZHAN, Jinxi, Neijiang, Sichuan 641000 (CN); YANG, Liu, Neijiang, Sichuan 641000 (CN)
(74) Representative: Murgitroyd & Company
(86) International application number: PCT/CN2025/074454
(87) International publication number: WO 2025/162198

(57) **Abstract**

Provided is a bispecific antibody-cytokine fusion protein. Specifically provided is a bispecific antibody-cytokine fusion protein targeting to TIGIT and PD-1 and having immune effector cell activation effect. The bispecific antibody-cytokine fusion protein has good medication safety and anti-tumor effect. Also provided is the use of the bispecific antibody-cytokine fusion protein in disease treatment.

## Description

The present application claims priority to Chinese Patent Application No. 2024101349441, filed on January 31, 2024. The contents of the above Chinese patent application are incorporated herein by reference in their entirety.

### TECHNICAL FIELD

The present disclosure relates to a bispecific antibody-cytokine fusion protein. In particular, the present disclosure provides a bispecific antibody-cytokine fusion protein targeting TIGIT and PD-1 with immune effector cell activation, and the use thereof in the treatment of a disease.

### BACKGROUND

T cell immunoreceptor with Ig and ITIM domains (TIGIT) is composed of an extracellular immunoglobulin variable (IgV) domain, a type I transmembrane domain, and an intracellular domain containing a classical immunoreceptor tyrosine-based inhibitory motif (ITIM) and an immunoglobulin tyrosine tail (ITT) motif. Currently, TIGIT signaling has been primarily studied in NK cells. These studies have shown that the ITT and ITIM motifs are the main motifs responsible for TIGIT function. Through these two motifs, TIGIT can recruit GRB2 and further recruit SHIP1, thereby inhibiting downstream PI3K and MAPK signaling pathways. TIGIT can also bind to β-arrestin to recruit SHIP1, blocking TRAF6 ubiquitination and NF-κB activation. TIGIT can inhibit lymphocytes through three distinct mechanisms of action: 1) TIGIT can signal through the ITIM and/or ITT motifs in its intracellular tail upon binding to poliovirus receptor (PVR). 2) TIGIT can induce PVR signaling in adjacent dendritic cells or tumor cells by binding to PVR. 3) TIGIT can inhibit CD226 signaling by binding to PVR with higher affinity or by disrupting CD226 homodimerization. All three mechanisms of action for the target are immunosuppressive. Previous preclinical and clinical studies on the TIGIT target have shown that the anti-tumor efficacy of TIGIT alone cannot surpass the currently widely used mature regimens targeting PD-1 or PD-1 combined with CTLA-4, indicating that it is necessary to select appropriate targets to combine with TIGIT.

Current studies on CD8+ T cells have shown that the higher the number of inhibitory receptors expressed, the higher the degree of CD8+ T cell exhaustion. How to overcome immune cell exhaustion is a key issue in the development of immunotherapy drugs. Current clinical studies have shown that TIGIT antibody therapy has essentially no efficacy in patients who are resistant to anti-PD1 therapy, indicating that TIGIT antibody alone cannot solve the problem of resistance to anti-PD1 therapy. However, multiple studies have shown that TIGIT and its ligand CD155 are upregulated in tumor tissues after PD-1 treatment, indicating a correlation with resistance to anti-PD1 therapy.

In addition to correlation with immune exhaustion, how to continuously provide active effector T cells and NK cells is another challenge related to how long patients can sustain a treatment response. Multiple studies have demonstrated that IL-15 can activate NK cells to exert anti-tumor effects and can modulate the expression of TIGIT and CD226. Studies in soft tissue sarcoma (STS) have shown that intratumoral NK and T cells exhibit significantly elevated activation and exhaustion markers, while IL-15 promotes activation signaling and the expression of exhaustion markers in NK and T cells. Moreover, *in vitro* experiments have shown that the combination of IL-15 with TIGIT exhibits significantly enhanced killing capacity against STS. Clinical studies have shown that the combination of the IL-15 recombinant protein N-803 with PD-1 can restore responsiveness in patients with immunotherapy-refractory tumors and overcome resistance to immunosuppressants.

Therefore, the present disclosure provides a TIGIT/PD-1/IL-15 bispecific antibody-cytokine fusion protein with a specific structural design, which is expected to enhance the synergistic effects of immune cells, expand immunomodulatory functions through multiple mechanisms, overcome resistance caused by tumor cell immune escape, thereby improving the efficacy of tumor immunotherapy.

### SUMMARY

In view of the foregoing issues, the present disclosure provides a bispecific antibody-cytokine fusion protein targeting TIGIT and PD-1 and comprising an IL-15/IL-15Rα complex with immune effector cell activation. The bispecific antibody-cytokine fusion protein exhibits good medication safety and anti-tumor efficacy. The following aspects are thus provided.

### Fusion protein

In a first aspect, the present disclosure relates to a fusion protein, comprising:
a first antigen-binding domain targeting TIGIT,
a second antigen-binding domain targeting PD-1,
an IL-15 polypeptide or a functional fragment thereof, and
an IL-15Rα polypeptide or a functional fragment thereof, wherein:
   (i) the first antigen-binding domain comprises a heavy chain variable region (VH) and a light chain variable region (VL) selected from any one of the following groups, wherein
   (i-a) a VH comprising the following three CDRs: CDR-H1 comprising the sequence as set forth in SEQ ID NO: 3, CDR-H2 comprising the sequence as set forth in SEQ ID NO: 4, and CDR-H3 comprising the sequence as set forth in SEQ ID NO: 5; and
   a VL comprising the following three CDRs: CDR-L1 comprising the sequence as set forth in SEQ ID NO: 6, CDR-L2 comprising the sequence as set forth in SEQ ID NO: 7, and CDR-L3 comprising the sequence as set forth in SEQ ID NO: 8;
      or
   (i-b) a VH comprising the following three CDRs: CDR-H1 comprising the sequence as set forth in SEQ ID NO: 11, CDR-H2 comprising the sequence as set forth in SEQ ID NO: 12, and CDR-H3 comprising the sequence as set forth in SEQ ID NO: 13; and
   a VL comprising the following three CDRs: CDR-L1 comprising the sequence as set forth in SEQ ID NO: 14, CDR-L2 comprising the sequence as set forth in SEQ ID NO: 15, and CDR-L3 comprising the sequence as set forth in SEQ ID NO: 16;
      or
   (i-c) a VH comprising the following three CDRs: CDR-H1 comprising the sequence as set forth in SEQ ID NO: 19, CDR-H2 comprising the sequence as set forth in SEQ ID NO: 20, and CDR-H3 comprising the sequence as set forth in SEQ ID NO: 21; and
   a VL comprising the following three CDRs: CDR-L1 comprising the sequence as set forth in SEQ ID NO: 22, CDR-L2 comprising the sequence as set forth in SEQ ID NO: 23, and CDR-L3 comprising the sequence as set forth in SEQ ID NO: 24;
   and/or, (ii) the second antigen-binding domain comprises a VH and a VL selected from any one of the following groups, wherein
   (ii-a) a VH comprising the following three CDRs: CDR-H1 comprising the sequence as set forth in SEQ ID NO: 27, CDR-H2 comprising the sequence as set forth in SEQ ID NO: 28, and CDR-H3 comprising the sequence as set forth in SEQ ID NO: 29; and
   a VL comprising the following three CDRs: CDR-L1 comprising the sequence as set forth in SEQ ID NO: 30, CDR-L2 comprising the sequence as set forth in SEQ ID NO: 31, and CDR-L3 comprising the sequence as set forth in SEQ ID NO: 32;
      or
   (ii-b) a VH comprising the following three CDRs: CDR-H1 comprising the sequence as set forth in SEQ ID NO: 35, CDR-H2 comprising the sequence as set forth in SEQ ID NO: 36, and CDR-H3 comprising the sequence as set forth in SEQ ID NO: 37; and
   a VL comprising the following three CDRs: CDR-L1 comprising the sequence as set forth in SEQ ID NO: 38, CDR-L2 comprising the sequence as set forth in SEQ ID NO: 39, and CDR-L3 comprising the sequence as set forth in SEQ ID NO: 40.

In certain embodiments, in the fusion protein, wherein,
(i) the first antigen-binding domain comprises a VH and a VL selected from any one of the following groups, wherein
   (i-a) a VH comprising the sequence as set forth in SEQ ID NO: 1 and a VL comprising the sequence as set forth in SEQ ID NO: 2;
   (i-b) a VH comprising the sequence as set forth in SEQ ID NO: 9 and a VL comprising the sequence as set forth in SEQ ID NO: 10;
   (i-c) a VH comprising the sequence as set forth in SEQ ID NO: 17 and a VL comprising the sequence as set forth in SEQ ID NO: 18;
   (i-d) a VH comprising the sequence as set forth in SEQ ID NO: 41 and a VL comprising the sequence as set forth in SEQ ID NO: 42;
   (i-e) a VH comprising the sequence as set forth in SEQ ID NO: 43 and a VL comprising the sequence as set forth in SEQ ID NO: 44;
   (i-f) a VH comprising the sequence as set forth in SEQ ID NO: 45 and a VL comprising the sequence as set forth in SEQ ID NO: 46;
   (i-g) a VH comprising the sequence as set forth in SEQ ID NO: 45 and a VL comprising the sequence as set forth in SEQ ID NO: 47;
   (i-h) a VH comprising the sequence as set forth in SEQ ID NO: 48 and a VL comprising the sequence as set forth in SEQ ID NO: 49;
   (i-i) a VH comprising the sequence as set forth in SEQ ID NO: 48 and a VL comprising the sequence as set forth in SEQ ID NO: 50;
      and/or,
   (ii) the second antigen-binding domain comprises a VH and a VL selected from any one of the following groups, wherein,
   (ii-a) a VH comprising the sequence as set forth in SEQ ID NO: 25 and a VL comprising the sequence as set forth in SEQ ID NO: 26;
   (ii-b) a VH comprising the sequence as set forth in SEQ ID NO: 33 and a VL comprising the sequence as set forth in SEQ ID NO: 34;
   (ii-c) a VH comprising the sequence as set forth in SEQ ID NO: 54 and a VL comprising the sequence as set forth in SEQ ID NO: 55;
   (ii-d) a VH comprising the sequence as set forth in SEQ ID NO: 56 and a VL comprising the sequence as set forth in SEQ ID NO: 57;
   (ii-e) a VH comprising the sequence as set forth in SEQ ID NO: 58 and a VL comprising the sequence as set forth in SEQ ID NO: 59;
   (ii-f) a VH comprising the sequence as set forth in SEQ ID NO: 56 and a VL comprising the sequence as set forth in SEQ ID NO: 59;
   (ii-g) a VH comprising the sequence as set forth in SEQ ID NO: 60 and a VL comprising the sequence as set forth in SEQ ID NO: 57;
   (ii-h) a VH comprising the sequence as set forth in SEQ ID NO: 60 and a VL comprising the sequence as set forth in SEQ ID NO: 61;
   (ii-i) a VH comprising the sequence as set forth in SEQ ID NO: 62 and a VL comprising the sequence as set forth in SEQ ID NO: 61;
   (ii-j) a VH comprising the sequence as set forth in SEQ ID NO: 51 and a VL comprising the sequence as set forth in SEQ ID NO: 52;
   (ii-k) a VH comprising the sequence as set forth in SEQ ID NO: 53 and a VL comprising the sequence as set forth in SEQ ID NO: 52.

In certain optional embodiments, for the VH and VL, compared to the VH and VL in any one of groups (i-a) to (i-i) and (ii-a) to (ii-k), the VH has at least 70%, at least 80%, at least 85%, at least 90%, at least 91%, at least 92%, at least 93%, at least 94%, at least 95%, at least 96%, at least 97%, at least 98%, or at least 99% sequence identity; and, the VL has at least 70%, at least 80%, at least 85%, at least 90%, at least 91%, at least 92%, at least 93%, at least 94%, at least 95%, at least 96%, at least 97%, at least 98%, or at least 99% sequence identity;
and/or,
(iii) the IL-15 polypeptide comprises the native amino acid sequence as set forth in SEQ ID NO: 63, or comprises an IL-15 variant that differs from the native amino acid sequence as set forth in SEQ ID NO: 63, wherein the IL-15 variant is still capable of maintaining the function of promoting effector cell activation; in certain preferred embodiments, the amino acid sequence of the IL-15 variant has at least one amino acid substitution, deletion, or addition (*e.g.*, 1, 2, 3, 4, or 5 amino acid substitutions, deletions, or additions) compared to the native amino acid sequence as set forth in SEQ ID NO: 63;
and/or, (iv) the IL-15Rα polypeptide comprises the native amino acid sequence as set forth in SEQ ID NO: 64, or comprises an IL-15Rα variant that differs from the native amino acid sequence as set forth in SEQ ID NO: 64, wherein the IL-15Rα variant is still capable of maintaining the function of promoting effector cell activation; in certain preferred embodiments, the amino acid sequence of the IL-15Rα variant has at least one amino acid substitution, deletion, or addition (*e.g.*, 1, 2, 3, 4, or 5 amino acid substitutions, deletions, or additions) compared to the native amino acid sequence as set forth in SEQ ID NO: 64; in certain preferred embodiments, the IL-15Rα variant comprises the sequence as set forth in SEQ ID NO: 75.

In a second aspect, the present disclosure relates to a fusion protein comprising: a first antigen-binding domain targeting TIGIT or PD-1, a second antigen-binding domain targeting TIGIT or PD-1, an IL-15 polypeptide or a functional fragment thereof, and an IL-15Rα polypeptide or a functional fragment thereof, wherein the first antigen-binding domain and the second antigen-binding domain respectively bind to different targets; the first antigen-binding domain is a Fab fragment; the second antigen-binding domain is a single-chain antibody; the IL-15 polypeptide and the IL-15Rα polypeptide are located on different peptide chains.

In certain preferred embodiments, the first antigen-binding domain is an antigen-binding domain targeting TIGIT; the second antigen-binding domain is an antigen-binding domain targeting PD-1.

In certain preferred embodiments, the single-chain antibody is an scFv.

In certain embodiments, the fusion protein further comprises an Fc domain, the Fc domain comprising a first monomer and a second monomer; wherein:
the N-terminus of the first monomer is optionally linked, via a linker, to one domain of the Fab fragment (*e.g.*, a heavy chain CH1 domain thereof), and the C-terminus of the first monomer is optionally linked, via a linker, to the IL-15 polypeptide or a functional fragment thereof, or to the IL-15Rα polypeptide or a functional fragment thereof; preferably, the N-terminus of the first monomer is optionally linked, via a linker, to the heavy chain CH1 domain of the Fab fragment, and the C-terminus of the first monomer is optionally linked, via a linker, to the IL-15 polypeptide or a functional fragment thereof; and
the N-terminus of the second monomer is optionally linked, via a linker, to another domain of the Fab fragment (*e.g.*, a light chain CL domain thereof), and the C-terminus of the second monomer is optionally linked, via a linker, to the IL-15Rα polypeptide or a functional fragment thereof, or to the IL-15 polypeptide or a functional fragment thereof; preferably, the N-terminus of the second monomer is optionally linked, via a linker, to the light chain CL domain of the Fab fragment, and the C-terminus of the second monomer is optionally linked, via a linker, to the IL-15Rα polypeptide or a functional fragment thereof.

In certain preferred embodiments, the Fc domain comprises a modification that promotes dimerization of the first monomer and the second monomer.

In certain preferred embodiments, the modification comprises a "knob" modification in one of the first monomer and the second monomer and a "hole" modification in the other of the first monomer and the second monomer, thereby forming a "knob-into-hole" modification.

In certain embodiments, in the fusion protein, wherein,
the IL-15 polypeptide comprises the amino acid sequence of the native IL-15 polypeptide of SEQ ID NO: 63, or comprises an IL-15 variant having an amino acid sequence different from that of the native IL-15 polypeptide, wherein the IL-15 variant is still capable of maintaining the function of the native IL-15 polypeptide in promoting effector cell activation; in certain preferred embodiments, the amino acid sequence of the IL-15 variant has at least one amino acid substitution, deletion, or addition (*e.g.*, 1, 2, 3, 4, or 5 amino acid substitutions, deletions, or additions) compared to the amino acid sequence of the native IL-15 polypeptide; and/or,
the IL-15Rα polypeptide comprises the amino acid sequence of the native IL-15Rα polypeptide of SEQ ID NO: 64, or comprises an IL-15Rα variant having an amino acid sequence different from that of the native IL-15Rα polypeptide, wherein the IL-15Rα variant is still capable of maintaining the function of the native IL-15Rα polypeptide in promoting effector cell activation; in certain preferred embodiments, the amino acid sequence of the IL-15Rα variant has at least one amino acid substitution, deletion, or addition (*e.g.*, 1, 2, 3, 4, or 5 amino acid substitutions, deletions, or additions) compared to the amino acid sequence of the native IL-15Rα polypeptide. In certain preferred embodiments, the IL-15Rα variant comprises the sequence as set forth in SEQ ID NO: 75.

In certain embodiments, the fusion protein comprises:
(i) a first peptide chain comprising a VH of the first antigen-binding domain, a heavy chain constant region 1 (CH1), an Fc domain monomer, an IL-15 polypeptide or an IL-15Rα polypeptide, and a single-chain antibody of the second antigen-binding domain; preferably, the CH1 is a human IgG1 heavy chain constant region CH1; preferably, the N-terminus of the Fc domain monomer is linked, via a first hinge region (*e.g.*, a hinge region comprising a PPCP peptide), to the C-terminus of the CH1, and the C-terminus of the Fc domain monomer is linked, via a linker (*e.g.*, a flexible peptide comprising (G4S)n), to the N-terminus of the IL-15 polypeptide or the IL-15Rα polypeptide; preferably, the C-terminus of the IL-15 polypeptide or the IL-15Rα polypeptide is linked, via a linker (*e.g.*, a flexible peptide comprising (G4S)n), to the N-terminus of the single-chain antibody; preferably, the single-chain antibody is an scFv;
   and
(ii) a second peptide chain comprising a VL of the first antigen-binding domain, a light chain constant region (CL), an Fc domain monomer, an IL-15 polypeptide or an IL-15Rα polypeptide, and a single-chain antibody of the second antigen-binding domain; preferably, the CL is a human kappa light chain constant region; preferably, the N-terminus of the Fc domain monomer is linked, via a second hinge region (*e.g.*, a hinge region comprising a PPCP peptide), to the C-terminus of the CL, and the C-terminus of the Fc domain monomer is linked, via a linker (*e.g.*, a flexible peptide comprising (G4S)n), to the N-terminus of the IL-15 polypeptide or the IL-15Rα polypeptide; preferably, the C-terminus of the IL-15 polypeptide or the IL-15Rα polypeptide is linked, via a linker (*e.g.*, a flexible peptide comprising (G4S)n), to the N-terminus of the single-chain antibody; preferably, the single-chain antibody is an scFv.

In certain preferred embodiments, the Fc domain monomer of the first peptide chain is capable of forming a dimer with the Fc domain monomer of the second peptide chain.

In certain preferred embodiments, the IL-15 polypeptide is located on the first peptide chain, and the IL-15Rα polypeptide is located on the second peptide chain.

In certain embodiments, in the fusion protein, the Fc domain monomer of the first peptide chain and the Fc domain monomer of the second peptide chain comprise a modification that promotes dimerization.

In certain preferred embodiments, the modification comprises an amino acid substitution in the CH3 domain of the Fc domain.

In certain preferred embodiments, the modification comprises a "knob" modification in one of the two Fc domains and a "hole" modification in the other of the two Fc domains, thereby forming a "knob-into-hole" modification.

In certain preferred embodiments, the Fc domain monomer of the first peptide chain comprises the amino acid sequence as set forth in SEQ ID NO: 71.

In certain preferred embodiments, the Fc domain monomer of the second peptide chain comprises the amino acid sequence as set forth in SEQ ID NO: 72.

In certain preferred embodiments, the two Fc domain monomers comprise the amino acid sequences as set forth in SEQ ID NOs: 71 and 72, respectively.

In certain preferred embodiments, the CH1 in the first peptide chain comprises the amino acid sequence as set forth in SEQ ID NO: 74, and/or the CL in the second peptide chain comprises the amino acid sequence as set forth in SEQ ID NO: 73.

In certain preferred embodiments, the first hinge region comprises the amino acid sequence as set forth in SEQ ID NO: 65, and the second hinge region comprises the amino acid sequence as set forth in SEQ ID NO: 66.

In certain preferred embodiments, in the first peptide chain and/or the second peptide chain, the linker connecting the C-terminus of the Fc domain monomer to the N-terminus of the IL-15 polypeptide or the IL-15Rα polypeptide comprises the sequence as set forth in SEQ ID NO: 67.

In certain preferred embodiments, in the first peptide chain and/or the second peptide chain, the linker connecting the C-terminus of the IL-15 polypeptide or the IL-15Rα polypeptide to the N-terminus of the single-chain antibody comprises the sequence as set forth in SEQ ID NO: 68.

In certain embodiments, in the fusion protein, the first antigen-binding domain targeting TIGIT comprises a VH and a VL selected from any one of the following groups, wherein,
(a) a VH comprising the following three CDRs: CDR-H1 comprising the sequence as set forth in SEQ ID NO: 3, CDR-H2 comprising the sequence as set forth in SEQ ID NO: 4, and CDR-H3 comprising the sequence as set forth in SEQ ID NO: 5; and
   a VL comprising the following three CDRs: CDR-L1 comprising the sequence as set forth in SEQ ID NO: 6, CDR-L2 comprising the sequence as set forth in SEQ ID NO: 7, and CDR-L3 comprising the sequence as set forth in SEQ ID NO: 8;
   or
(b) a VH comprising the following three CDRs: CDR-H1 comprising the sequence as set forth in SEQ ID NO: 11, CDR-H2 comprising the sequence as set forth in SEQ ID NO: 12, and CDR-H3 comprising the sequence as set forth in SEQ ID NO: 13; and
   a VL comprising the following three CDRs: CDR-L1 comprising the sequence as set forth in SEQ ID NO: 14, CDR-L2 comprising the sequence as set forth in SEQ ID NO: 15, and CDR-L3 comprising the sequence as set forth in SEQ ID NO: 16;
   or
(c) a VH comprising the following three CDRs: CDR-H1 comprising the sequence as set forth in SEQ ID NO: 19, CDR-H2 comprising the sequence as set forth in SEQ ID NO: 20, and CDR-H3 comprising the sequence as set forth in SEQ ID NO: 21; and

a VL comprising the following three CDRs: CDR-L1 comprising the sequence as set forth in SEQ ID NO: 22, CDR-L2 comprising the sequence as set forth in SEQ ID NO: 23, and CDR-L3 comprising the sequence as set forth in SEQ ID NO: 24;
preferably, the first antigen-binding domain targeting TIGIT comprises a VH and a VL selected from any one of the following groups, wherein
(i-a) a VH comprising the sequence as set forth in SEQ ID NO: 1 and a VL comprising the sequence as set forth in SEQ ID NO: 2;
(i-b) a VH comprising the sequence as set forth in SEQ ID NO: 9 and a VL comprising the sequence as set forth in SEQ ID NO: 10;
(i-c) a VH comprising the sequence as set forth in SEQ ID NO: 17 and a VL comprising the sequence as set forth in SEQ ID NO: 18;
(i-d) a VH comprising the sequence as set forth in SEQ ID NO: 41 and a VL comprising the sequence as set forth in SEQ ID NO: 42;
(i-e) a VH comprising the sequence as set forth in SEQ ID NO: 43 and a VL comprising the sequence as set forth in SEQ ID NO: 44;
(i-f) a VH comprising the sequence as set forth in SEQ ID NO: 45 and a VL comprising the sequence as set forth in SEQ ID NO: 46;
(i-g) a VH comprising the sequence as set forth in SEQ ID NO: 45 and a VL comprising the sequence as set forth in SEQ ID NO: 47;
(i-h) a VH comprising the sequence as set forth in SEQ ID NO: 48 and a VL comprising the sequence as set forth in SEQ ID NO: 49;
(i-i) a VH comprising the sequence as set forth in SEQ ID NO: 48 and a VL comprising the sequence as set forth in SEQ ID NO: 50;

In certain optional embodiments, for the VH and VL, compared to the VH and VL in any one of groups (i-a) to (i-i), the VH has at least 70%, at least 80%, at least 85%, at least 90%, at least 91%, at least 92%, at least 93%, at least 94%, at least 95%, at least 96%, at least 97%, at least 98%, or at least 99% sequence identity; and, the VL has at least 70%, at least 80%, at least 85%, at least 90%, at least 91%, at least 92%, at least 93%, at least 94%, at least 95%, at least 96%, at least 97%, at least 98%, or at least 99% sequence identity.

In certain embodiments, in the fusion protein, the second antigen-binding domain targeting PD-1 comprises a VH and a VL selected from any one of the following groups, wherein
(A) a VH comprising the following three CDRs: CDR-H1 comprising the sequence as set forth in SEQ ID NO: 27, CDR-H2 comprising the sequence as set forth in SEQ ID NO: 28, and CDR-H3 comprising the sequence as set forth in SEQ ID NO: 29; and
   a VL comprising the following three CDRs: CDR-L1 comprising the sequence as set forth in SEQ ID NO: 30, CDR-L2 comprising the sequence as set forth in SEQ ID NO: 31, and CDR-L3 comprising the sequence as set forth in SEQ ID NO: 32;
   or
(B) a VH comprising the following three CDRs: CDR-H1 comprising the sequence as set forth in SEQ ID NO: 35, CDR-H2 comprising the sequence as set forth in SEQ ID NO: 36, and CDR-H3 comprising the sequence as set forth in SEQ ID NO: 37; and

a VL comprising the following three CDRs: CDR-L1 comprising the sequence as set forth in SEQ ID NO: 38, CDR-L2 comprising the sequence as set forth in SEQ ID NO: 39, and CDR-L3 comprising the sequence as set forth in SEQ ID NO: 40;
preferably, the second antigen-binding domain targeting PD-1 comprises a VH and a VL selected from any one of the following groups, wherein
(ii-a) a VH comprising the sequence as set forth in SEQ ID NO: 25 and a VL comprising the sequence as set forth in SEQ ID NO: 26;
(ii-b) a VH comprising the sequence as set forth in SEQ ID NO: 33 and a VL comprising the sequence as set forth in SEQ ID NO: 34;
(ii-c) a VH comprising the sequence as set forth in SEQ ID NO: 54 and a VL comprising the sequence as set forth in SEQ ID NO: 55;
(ii-d) a VH comprising the sequence as set forth in SEQ ID NO: 56 and a VL comprising the sequence as set forth in SEQ ID NO: 57;
(ii-e) a VH comprising the sequence as set forth in SEQ ID NO: 58 and a VL comprising the sequence as set forth in SEQ ID NO: 59;
(ii-f) a VH comprising the sequence as set forth in SEQ ID NO: 56 and a VL comprising the sequence as set forth in SEQ ID NO: 59;
(ii-g) a VH comprising the sequence as set forth in SEQ ID NO: 60 and a VL comprising the sequence as set forth in SEQ ID NO: 57;
(ii-h) a VH comprising the sequence as set forth in SEQ ID NO: 60 and a VL comprising the sequence as set forth in SEQ ID NO: 61;
(ii-i) a VH comprising the sequence as set forth in SEQ ID NO: 62 and a VL comprising the sequence as set forth in SEQ ID NO: 61;
(ii-j) a VH comprising the sequence as set forth in SEQ ID NO: 51 and a VL comprising the sequence as set forth in SEQ ID NO: 52;
(ii-k) a VH comprising the sequence as set forth in SEQ ID NO: 53 and a VL comprising the sequence as set forth in SEQ ID NO: 52;

In certain optional embodiments, for the VH and VL, compared to the VH and VL in any one of groups (ii-a) to (ii-k), the VH has at least 70%, at least 80%, at least 85%, at least 90%, at least 91%, at least 92%, at least 93%, at least 94%, at least 95%, at least 96%, at least 97%, at least 98%, or at least 99% sequence identity; and, the VL has at least 70%, at least 80%, at least 85%, at least 90%, at least 91%, at least 92%, at least 93%, at least 94%, at least 95%, at least 96%, at least 97%, at least 98%, or at least 99% sequence identity.

In certain embodiments, the fusion protein comprises:
(i) a first peptide chain having a structure of [VH1]-[CH1]-[hinge region 1]-[Fc monomer 1]-[L1]-[IL-15]-[L1]-[VL2]-[L2]-[VH2]; and
(ii) a second peptide chain having a structure of [VL1]-[CL]-[hinge region 2]-[Fc monomer 2]-[L1]-[IL-15Rα]-[L1]-[VL2]-[L2]-[VH2];
wherein any one of the following is selected:
(1) the VH1 comprises the sequence as set forth in SEQ ID NO: 9; the VL1 comprises the sequence as set forth in SEQ ID NO: 10; the VH2 comprises the sequence as set forth in SEQ ID NO: 25; the VL2 comprises the sequence as set forth in SEQ ID NO: 26; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 64; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(2) the VH1 comprises the sequence as set forth in SEQ ID NO: 1; the VL1 comprises the sequence as set forth in SEQ ID NO: 2; the VH2 comprises the sequence as set forth in SEQ ID NO: 25; the VL2 comprises the sequence as set forth in SEQ ID NO: 26; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 64; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(3) the VH1 comprises the sequence as set forth in SEQ ID NO: 41; the VL1 comprises the sequence as set forth in SEQ ID NO: 42; the VH2 comprises the sequence as set forth in SEQ ID NO: 54; the VL2 comprises the sequence as set forth in SEQ ID NO: 55; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(4) the VH1 comprises the sequence as set forth in SEQ ID NO: 41; the VL1 comprises the sequence as set forth in SEQ ID NO: 42; the VH2 comprises the sequence as set forth in SEQ ID NO: 56; the VL2 comprises the sequence as set forth in SEQ ID NO: 57; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(5) the VH1 comprises the sequence as set forth in SEQ ID NO: 41; the VL1 comprises the sequence as set forth in SEQ ID NO: 42; the VH2 comprises the sequence as set forth in SEQ ID NO: 62; the VL2 comprises the sequence as set forth in SEQ ID NO: 61; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(6) the VH1 comprises the sequence as set forth in SEQ ID NO: 45; the VL1 comprises the sequence as set forth in SEQ ID NO: 47; the VH2 comprises the sequence as set forth in SEQ ID NO: 51; the VL2 comprises the sequence as set forth in SEQ ID NO: 52; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(7) the VH1 comprises the sequence as set forth in SEQ ID NO: 45; the VL1 comprises the sequence as set forth in SEQ ID NO: 47; the VH2 comprises the sequence as set forth in SEQ ID NO: 58; the VL2 comprises the sequence as set forth in SEQ ID NO: 59; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(8) the VH1 comprises the sequence as set forth in SEQ ID NO: 45; the VL1 comprises the sequence as set forth in SEQ ID NO: 47; the VH2 comprises the sequence as set forth in SEQ ID NO: 56; the VL2 comprises the sequence as set forth in SEQ ID NO: 57; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(9) the VH1 comprises the sequence as set forth in SEQ ID NO: 45; the VL1 comprises the sequence as set forth in SEQ ID NO: 47; the VH2 comprises the sequence as set forth in SEQ ID NO: 56; the VL2 comprises the sequence as set forth in SEQ ID NO: 59; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(10) the VH1 comprises the sequence as set forth in SEQ ID NO: 45; the VL1 comprises the sequence as set forth in SEQ ID NO: 46; the VH2 comprises the sequence as set forth in SEQ ID NO: 60; the VL2 comprises the sequence as set forth in SEQ ID NO: 57; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(11) the VH1 comprises the sequence as set forth in SEQ ID NO: 45; the VL1 comprises the sequence as set forth in SEQ ID NO: 47; the VH2 comprises the sequence as set forth in SEQ ID NO: 62; the VL2 comprises the sequence as set forth in SEQ ID NO: 61; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(12) the VH1 comprises the sequence as set forth in SEQ ID NO: 48; the VL1 comprises the sequence as set forth in SEQ ID NO: 50; the VH2 comprises the sequence as set forth in SEQ ID NO: 60; the VL2 comprises the sequence as set forth in SEQ ID NO: 57; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(13) the VH1 comprises the sequence as set forth in SEQ ID NO: 48; the VL1 comprises the sequence as set forth in SEQ ID NO: 50; the VH2 comprises the sequence as set forth in SEQ ID NO: 51; the VL2 comprises the sequence as set forth in SEQ ID NO: 52; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(14) the VH1 comprises the sequence as set forth in SEQ ID NO: 48; the VL1 comprises the sequence as set forth in SEQ ID NO: 49; the VH2 comprises the sequence as set forth in SEQ ID NO: 60; the VL2 comprises the sequence as set forth in SEQ ID NO: 61; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(15) the VH1 comprises the sequence as set forth in SEQ ID NO: 48; the VL1 comprises the sequence as set forth in SEQ ID NO: 49; the VH2 comprises the sequence as set forth in SEQ ID NO: 53; the VL2 comprises the sequence as set forth in SEQ ID NO: 52; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(16) the VH1 comprises the sequence as set forth in SEQ ID NO: 43; the VL1 comprises the sequence as set forth in SEQ ID NO: 44; the VH2 comprises the sequence as set forth in SEQ ID NO: 60; the VL2 comprises the sequence as set forth in SEQ ID NO: 57; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(17) the VH1 comprises the sequence as set forth in SEQ ID NO: 43; the VL1 comprises the sequence as set forth in SEQ ID NO: 44; the VH2 comprises the sequence as set forth in SEQ ID NO: 60; the VL2 comprises the sequence as set forth in SEQ ID NO: 61; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(18) the VH1 comprises the sequence as set forth in SEQ ID NO: 45; the VL1 comprises the sequence as set forth in SEQ ID NO: 46; the VH2 comprises the sequence as set forth in SEQ ID NO: 53; the VL2 comprises the sequence as set forth in SEQ ID NO: 52; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66.

### Preparation of fusion protein

The fusion protein according to any one of the foregoing aspects may be prepared by various methods known in the art, for example, by means of genetic engineering and recombinant technology. For example, DNA molecules encoding the fusion protein may be obtained by chemical synthesis or PCR amplification. The obtained DNA molecules are inserted into expression vectors, which are then transfected into host cells. Subsequently, the transfected host cells are cultured under specific conditions to express the fusion protein of the present disclosure.

In another aspect, the present disclosure provides an isolated nucleic acid molecule encoding:
the fusion protein according to the first or second aspect, or a polypeptide chain thereof.

In certain embodiments, the vector comprises nucleotide sequences encoding the respective peptide chains of the present disclosure, and the nucleotide sequences encoding the respective peptide chains are present in the same vector or in different vectors.

In another aspect, the present disclosure provides a host cell comprising the nucleic acid molecule or vector described above. Such host cells include, but are not limited to, prokaryotic cells such as bacterial cells (*e.g.*, *Escherichia coli* cells), and eukaryotic cells such as fungal cells (*e.g.*, yeast cells), insect cells, plant cells, and animal cells (*e.g.*, mammalian cells, such as mouse cells or human cells).

In another aspect, the present disclosure provides a method of preparing the fusion protein according to any one of the foregoing aspects, comprising culturing the host cell described above under conditions that allow protein expression, and collecting the fusion protein from the culture of the cultured host cell.

### Conjugate

The present disclosure provides a conjugate comprising the fusion protein or isolated nucleic acid molecule according to any one of the foregoing aspects, and a conjugation moiety linked thereto.

In certain embodiments, the conjugation moiety is selected from detectable labels, such as radioisotopes, fluorescent substances, luminescent substances, chromogenic substances, or enzymes.

In certain embodiments, the conjugation moiety is selected from therapeutic agents, such as cytotoxic agents, cytokines, toxins, radionuclides, immunostimulants, immunosuppressants, and other active substances that inhibit tumor cell growth or promote tumor cell apoptosis or necrosis.

### Pharmaceutical composition

The fusion protein or conjugate disclosed herein (also referred to as the active ingredient) may be incorporated into a pharmaceutical composition suitable for administration.

In another aspect, the present disclosure relates to a pharmaceutical composition comprising the fusion protein, isolated nucleic acid molecule, vector, host cell, or conjugate according to any one of the foregoing aspects, and a pharmaceutically acceptable carrier and/or excipient.

### Kit

The present disclosure provides a kit comprising the fusion protein, isolated nucleic acid molecule, vector, host cell, conjugate, or pharmaceutical composition according to any one of the foregoing aspects.

### Therapeutic use

In another aspect, the present disclosure relates to a method for the prevention and/or treatment and/or neoadjuvant treatment and/or adjuvant treatment of a disease in a subject, comprising administering to the subject in need thereof the fusion protein, isolated nucleic acid molecule, vector, host cell, conjugate, or pharmaceutical composition according to any one of the foregoing aspects. The present disclosure also relates to the use of the fusion protein, isolated nucleic acid molecule, vector, host cell, conjugate, or pharmaceutical composition according to any one of the foregoing aspects for the prevention and/or treatment and/or neoadjuvant treatment and/or adjuvant treatment of a disease, or in the manufacture of a medicament for the prevention and/or treatment and/or neoadjuvant treatment and/or adjuvant treatment of a disease.

In certain embodiments, the disease is a tumor. In certain embodiments, the tumor is a solid tumor or a hematological tumor. In certain embodiments, the solid tumor is melanoma, lung cancer, renal cancer, bladder cancer, head and neck tumor, gastric cancer, esophageal cancer, liver cancer, cervical cancer, breast cancer, or skin cancer.

### BRIEF DESCRIPTION OF THE DRAWINGS

FIG. 1A shows the assay results that the chimeric anti-TIGIT antibody 7TI-027 enhances IFN-γ secretion by SEA-activated PBMCs.
FIG. 1B shows the assay results that the chimeric anti-TIGIT antibodies 7TI-042 and 7TI-046 enhance IFN-γ secretion by SEA-activated PBMCs.
FIG. 2A shows the assay results that the chimeric anti-TIGIT antibody 7TI-027 enhances IL-2 secretion by SEA-activated PBMCs.
FIG. 2B shows the assay results that the chimeric anti-TIGIT antibodies 7TI-042 and 7TI-046 enhance IL-2 secretion by SEA-activated PBMCs.
FIG. 3A shows the assay results that the chimeric anti-PD-1 antibody 1KP1-1E10-F6-hz enhances IFN-γ secretion by SEA-activated PBMCs.
FIG. 3B shows the assay results that the chimeric anti-PD-1 antibody 1KP1-3A6-3C6-hz enhances IFN-γ secretion by SEA-activated PBMCs.
FIG. 4 shows the assay results that the chimeric anti-PD-1 antibody 1KP1-1E10-F6-hz enhances IL-2 secretion by SEA-activated PBMCs.
FIG. 5A is a schematic diagram of structure I of the TIGIT/PD-1/IL-15 bispecific antibody-cytokine fusion protein.

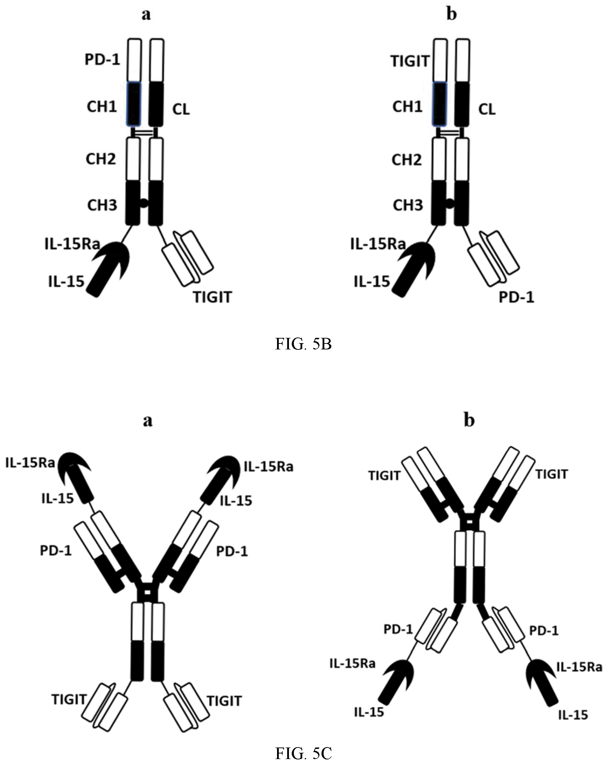

FIG. 5B is a schematic diagram of structure II of the TIGIT/PD-1/IL-15 bispecific antibody-cytokine fusion protein; wherein a of FIG. 5Ba shows that the N-terminus of chain 1 is the heavy chain variable region of the anti-PD-1 antibody; the N-terminus of chain 2 is the light chain variable region of the anti-PD-1 antibody, and the C-terminus of chain 2 is composed of the light chain variable region of the anti-TIGIT antibody linked to the heavy chain variable region of the anti-TIGIT antibody via a linker; b of FIG. 5B shows that the N-terminus of chain 1 is the heavy chain variable region of the anti-TIGIT antibody; the N-terminus of chain 2 is the light chain variable region of the anti-TIGIT antibody, and the C-terminus of chain 2 is composed of the light chain variable region of the anti-PD-1 antibody linked to the heavy chain variable region of the anti-PD-1 antibody via a linker.
FIG. 5C is a schematic diagram of structure III of the TIGIT/PD-1/IL-15 bispecific antibody-cytokine fusion protein; wherein a of FIG. 5C is a schematic diagram showing the IL-15/IL-15Rα complex linked to the N-terminus of the heavy chain variable region of the anti-PD-1 antibody via a linker; b of FIG. 5C is a schematic diagram showing the IL-15/IL-15Rα complex linked to the C-terminus of the heavy chain variable region of the anti-PD-1 antibody via a linker.
FIG. 5D is a schematic diagram of structure IV of the TIGIT/PD-1/IL-15 bispecific antibody-cytokine fusion protein.
FIG. 6A shows the activation effect of chimeric bispecific antibody-cytokine fusion proteins on Jurkat/NFAT-luc/PD1 reporter cells.
FIG. 6B shows the activation effect of chimeric bispecific antibody-cytokine fusion proteins on Jurkat-TIGIT-NFAT-Luc reporter cells.
FIG. 7A shows the proliferation-promoting effect of chimeric bispecific antibody-cytokine fusion proteins on CTLL-2 cells.
FIG. 7B shows the proliferation-promoting effect of chimeric bispecific antibody-cytokine fusion proteins on Mo7e cells.
FIG. 8A shows the effect of the chimeric bispecific antibody-cytokine fusion protein 7Y2-104 in promoting IL-2 secretion.
FIG. 8B shows the effect of the chimeric bispecific antibody-cytokine fusion protein 7Y2-102 in promoting IL-2 secretion.
FIG. 9A shows the effect of the chimeric bispecific antibody-cytokine fusion protein 7Y2-104 in promoting IFN-γ secretion.
FIG. 9B shows the effect of the chimeric bispecific antibody-cytokine fusion protein 7Y2-102 in promoting IFN-γ secretion.
FIG. 10 shows long-term toxicity survival curves of mice treated with chimeric bispecific antibody-cytokine fusion proteins.
FIG. 11 shows the activation effect of humanized bispecific antibody-cytokine fusion proteins on TIGIT/PD-1 dual-target effector cells.
FIG. 12A shows the proliferation-promoting effect of the humanized bispecific antibody-cytokine fusion proteins 7Y2-120, 7Y2-123, 7Y2-126, and 7Y2-127 on Mo7e cells.
FIG. 12B shows the proliferation-promoting effect of the humanized bispecific antibody-cytokine fusion proteins 7Y2-129, 7Y2-130, 7Y2-132, 7Y2-134, and 7Y2-136 on Mo7e cells.
FIG. 12C shows the proliferation-promoting effect of the humanized bispecific antibody-cytokine fusion proteins 7Y2-128, 7Y2-135, and 7Y2-137 on Mo7e cells.
FIG. 13 shows the assay results of the tumor inhibitory effects of humanized bispecific antibody-cytokine fusion proteins at different dosing levels. The significance level was set at p < 0.05; *, P < 0.05; **, P < 0.01; ***, P < 0.001; indicating statistically significant differences.
FIG. 14 shows the assay results of the effects of humanized bispecific antibody-cytokine fusion proteins at different dosing levels on mouse body weight.

### DETAILED DESCRIPTION OF THE PREFERRED EMBODIMENT

### Definition of terms

In the present disclosure, unless otherwise specified, all scientific and technical terms used herein have the meanings commonly understood by those skilled in the art. Additionally, to better understand the present disclosure, definitions and explanations of relevant terms are provided below.

When the terms "for example", "*e.g.*", "such as", "including", "comprising", or variations thereof are used herein, such terms shall not be considered limiting and shall be interpreted to mean "but not limited to" or "not limited to".

Unless otherwise indicated herein or clearly contradicted by context, the terms "a", "an", "the", and similar referents shall be interpreted as covering both singular and plural forms in the context of describing the present disclosure (especially in the context of the following claims).

As used herein, the terms "fusion protein" and "bispecific antibody-cytokine fusion protein" are used interchangeably and refer to the bispecific antibody-cytokine fusion protein targeting TIGIT and PD-1 and comprising an IL-15/IL-15Rα complex with immune effector cell activation provided by the present disclosure.

As used herein, the term "antigen-binding domain" refers to a domain capable of specifically binding to a target antigen. In specific embodiments, the antigen-binding domain may comprise an antigen-binding site derived from an antibody molecule that specifically binds to the target antigen; preferably, the antigen-binding site in the antibody molecule includes the heavy chain variable region (VH) and light chain variable region (VL) of the antibody.

As used herein, the term "antibody" refers to an immunoglobulin-derived molecule capable of specifically binding to a target antigen, wherein the immunoglobulin-derived molecule binds to the target antigen through at least one antigen-binding site located in its variable region. When referring to the term "antibody", unless the context clearly indicates otherwise, it includes not only intact antibodies but also antigen-binding fragments capable of specifically binding to a target antigen. An "intact antibody" typically consists of two pairs of polypeptide chains (each pair having one light chain (LC) and one heavy chain (HC)). Light chains of an antibody may be classified as κ (kappa) and λ (lambda) light chains. Heavy chains may be classified as µ, δ, γ, α, or ε, and the isotypes of antibodies are defined as IgM, IgD, IgG, IgA, and IgE, respectively. In the light and heavy chains, the variable and constant regions are linked by a "J" region of approximately 12 or more amino acids, and the heavy chain also comprises a "D" region of approximately 3 or more amino acids. Each heavy chain consists of a heavy chain variable region (VH) and a heavy chain constant region (CH). The heavy chain constant region consists of three domains (CH1, CH2, and CH3). Each light chain consists of a light chain variable region (VL) and a light chain constant region (CL). The light chain constant region consists of one domain CL. The constant domains are not directly involved in antibody-antigen binding, but exhibit various effector functions, such as being capable of mediating the binding of immunoglobulins to host tissues or factors, including various cells of the immune system (*e.g.*, effector cells) and the first component of the classical complement system (C1q). The VH and VL regions may be further subdivided into regions of high variability, known as complementarity-determining regions (CDRs), among which more conserved regions called framework regions (FRs) are interspersed. Each V_{H} and V_{L} consists of three CDRs and four FRs arranged in the following order: FR1, CDR1, FR2, CDR2, FR3, CDR3, and FR4 from the N-terminus to the C-terminus. The variable regions (VH and VL) of each heavy chain/light chain pair form an antigen-binding site.

As used herein, the term "complementarity-determining region" or "CDR" refers to the amino acid residues in the variable region of an antibody responsible for antigen binding. Each variable region of the heavy and light chains contains three CDRs, designated CDR1, CDR2, and CDR3. The precise boundaries of these CDRs may be defined according to various numbering systems known in the art, for example, defined according to the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196: 901-917; Chothia et al. (1989) Nature 342: 878-883), the IMGT numbering system (Lefranc et al., Dev. Comparat. Immunol. 27: 55-77, 2003), the AbM numbering system (Martin ACR, Cheetham JC, Rees AR (1989) Modelling antibody hypervariable loops: A combined algorithm. Proc Natl Acad Sci USA 86: 9268-9272), or the Contact numbering system (MacCallum, R. M., Martin, A. C. R., & Thornton, J. M. (1996). Antibody-antigen Interactions: Contact Analysis and Binding Site Topography. Journal of Molecular Biology, 262(5), 732-745). For a given antibody, those skilled in the art will readily identify the CDR defined according to each numbering system. Moreover, the correspondence between different numbering systems is well known to those skilled in the art.

As used herein, the term "framework region" or "FR" residues refers to the amino acid residues in the variable region of an antibody other than the CDR residues as defined above.

The term "antibody" is not limited by any particular method of producing antibodies. For example, it includes recombinant antibodies, monoclonal antibodies, and polyclonal antibodies. The antibody may be of different isotypes, such as IgG (*e.g.*, IgG1, IgG2, IgG3, or IgG4 subtypes), IgA1, IgA2, IgD, IgE, or IgM antibodies.

As used herein, the term "multispecific antibody" refers to an antibody having binding specificity for at least two (*e*.*g*., two, three, or four) different antigens (or epitopes). The multispecific antibody comprises multiple antigen-binding domains with binding specificity for different antigens (or epitopes), thereby enabling binding to at least two distinct binding sites and/or target molecules. Each antigen-binding domain contained in the multispecific antibody may be independently selected from full-length antibodies (*e.g.*, IgG antibodies) or antigen-binding fragments thereof (*e.g.*, Fv fragments, Fab fragments, F(ab')₂ fragments, or scFv). In some cases, the individual antigen-binding domains are linked via a peptide linker.

As used herein, the term "antigen-binding fragment" of an antibody refers to a polypeptide comprising a fragment of a full-length antibody that retains the ability to specifically bind to the same antigen as the full-length antibody, and/or competes with the full-length antibody to specifically bind to the antigen, which is also referred to as an "antigen-binding portion". Antigen-binding fragments of antibodies may be produced by recombinant DNA technology or by enzymatic or chemical cleavage of intact antibodies. Non-limiting examples of antigen-binding fragments include Fab fragments, Fab' fragments, F(ab')₂ fragments, Fd fragments, Fv fragments, complementarity-determining region (CDR) fragments, single-chain antibodies (*e.g.*, scFv or scFab), diabodies, single-domain antibodies, chimeric antibodies, linear antibodies, nanobodies (technology from Domantis), probodies, and polypeptides that contain at least a portion of an antibody sufficient to confer specific antigen-binding capability to the polypeptide.

As used herein, the term "full-length antibody" means an antibody consisting of two "full-length heavy chains" and two "full-length light chains". Specifically, a "full-length heavy chain" refers to a polypeptide chain consisting of a heavy chain variable region (VH), a heavy chain constant region CH1 domain, a hinge region (HR), a heavy chain constant region CH2 domain, and a heavy chain constant region CH3 domain from the N-terminus to the C-terminus; and optionally further comprises a heavy chain constant region CH4 domain when the full-length antibody is of the IgE or IgM isotype. Preferably, the "full-length heavy chain" is a polypeptide chain consisting of VH, CH1, HR, CH2, and CH3 from the N-terminus to the C-terminus. A "full-length light chain" is a polypeptide chain consisting of a light chain variable region (VL) and a light chain constant region (CL) from the N-terminus to the C-terminus. The two pairs of full-length antibody chains are linked together via a disulfide bond between CL and CH1 and a disulfide bond between the HRs of the two full-length heavy chains. The full-length antibody contains two antigen-binding sites formed by VH and VL pairs, respectively, which specifically recognize/bind to the same antigen.

The term "chimeric antibody" refers to an antibody obtained by fusing the variable region of a murine antibody with the constant region of a human antibody, which can alleviate the immune response induced by the murine antibody. To construct a chimeric antibody, a hybridoma secreting a specific murine monoclonal antibody is first established. A variable region gene is then cloned from murine hybridoma cells, followed by cloning of a constant region gene of a human antibody as desired. The murine variable region gene is ligated with the human constant region gene to form a chimeric gene, which is then inserted into a human vector. Finally, the chimeric antibody molecule is expressed in a eukaryotic or prokaryotic industrial system. The antibody may further comprise a light chain constant region derived from human κ or λ chain, or a variant thereof. The antibody further comprises a heavy chain constant region derived from human IgG1, IgG2, IgG3, or IgG4, or a variant thereof. The constant region of a human antibody may be selected from the heavy chain constant region derived from human IgG1, IgG2, IgG3, or IgG4, or a variant thereof, and preferably comprises a heavy chain constant region derived from human IgG2 or IgG4, or IgG4 without ADCC (antibody-dependent cell-mediated cytotoxicity) via amino acid mutations.

A "humanized antibody" refers to a type of engineered antibody having CDRs derived from a non-human donor immunoglobulin, the remaining immunoglobulin-derived portion of the humanized antibody being derived from one (or more) human immunoglobulin(s). Furthermore, framework support residues may be altered to preserve binding affinity (see, for example, Queen et al., Proc. Natl. Acad. Sci. USA, 86: 10029-10032 (1989); Hodgson et al., Bio/Technology, 9: 421 (1991)). A suitable human acceptor antibody may be one selected from a conventional database, such as the Los Alamos database and the Swiss Protein database, based on homology to the nucleotide and amino acid sequences of the donor antibody. A human antibody characterized by homology to the framework regions of the donor antibody (on an amino acid basis) may be suitable for providing a heavy chain constant region and/or a heavy chain variable framework region for insertion of the donor CDRs. A suitable acceptor antibody capable of providing light chain constant or variable framework regions may be selected in a similar manner. It should be noted that the heavy and light chains of an acceptor antibody are not required to be derived from the same acceptor antibody.

As used herein, the term "Fab fragment" means an antibody fragment consisting of the VL, VH, CL, and CH1 domains. The term "F(ab')₂ fragment" means an antibody fragment comprising two Fab fragments linked by a disulfide bridge on the hinge region. The term "Fab' fragment" means a fragment obtained by reducing the disulfide bond connecting the two heavy chain fragments in the F(ab')₂ fragment, consisting of an intact light chain and the Fd fragment (consisting of the VH and CH1 domains) of the heavy chain.

As used herein, the term "scFv" refers to a single polypeptide chain comprising the VL and VH domains, wherein the VL and VH are linked via a linker. Such scFv molecules may have a general structure: NH₂-VL-linker-VH-COOH or NH₂-VH-linker-VL-COOH. Suitable linkers known in the art consist of repeated GGGGS amino acid sequences or variants thereof. For example, a linker with the amino acid sequence (GGGGS)₂ may be used, and variants thereof may also be used. In some cases, a disulfide bond may also exist between the VH and VL of an scFv.

As used herein, the term "Fc domain" or "Fc region" means a portion of the heavy chain constant region comprising CH2 and CH3. The Fc fragment of an antibody has a variety of functions. "Effector functions" mediated by the Fc region include Fc receptor binding; Clq binding and complement-dependent cytotoxicity (CDC); antibody-dependent cell-mediated cytotoxicity (ADCC); phagocytosis; down-regulation of cell surface receptors (*e.g.*, B cell receptors); B cell activation, *etc*. In some embodiments, the Fc region comprises a hinge, CH2, and CH3. When the Fc region comprises a hinge, the hinge regulates dimerization between two Fc-containing polypeptides. The Fc region may be an isotype of any antibody heavy chain constant region, such as IgG1, IgG2, IgG3, or IgG4.

The Fc domain may comprise either a native Fc region or a variant Fc region. The native Fc region comprises an amino acid sequence identical to the amino acid sequence of the Fc region found in nature. For example, the native sequence human Fc region includes a native sequence human IgG1 Fc region (non-A and A allotypes); a native sequence human IgG2 Fc region; a native sequence human IgG3 Fc region; and a native sequence human IgG4 Fc region, as well as naturally occurring variants thereof. The variant Fc region comprises an amino acid sequence that differs from the amino acid sequence of the native sequence Fc region due to at least one amino acid modification. In some embodiments, the variant Fc region may have effector functions (*e.g.*, Fc receptor binding, antibody glycosylation, number of cysteine residues, effector cell function, or complement function) that are altered compared to the native Fc region.

The Fc domain may also comprise a modification that promotes dimerization of the Fc domain. In some embodiments, the modification comprises introducing amino acid mutations in one Fc domain monomer (*e.g.*, replacing a smaller amino acid with a larger amino acid) to form a "knob" modification; and simultaneously introducing amino acid mutations in the other Fc domain monomer (*e.g.*, replacing multiple larger amino acids with smaller amino acids) to form a "hole" modification, thereby altering the local spatial structure of the Fc domain. In such cases, the modified Fc domains are prone to undergo heterodimerization via a "knob-into-hole" interaction mechanism. The design facilitates the correct assembly of two heterologous antibody heavy chains, avoiding mismatches between heavy and light chains.

Each of the antibody fragments described above retains the ability to specifically bind to the same antigen as the full-length antibody, and/or competes with the full-length antibody to specifically bind to the antigen.

Antigen-binding fragments of an antibody (*e.g.*, the antibody fragments described above) may be obtained from a given antibody (*e.g.*, the antibodies provided in the present disclosure) using conventional techniques known to those skilled in the art (*e.g.*, recombinant DNA technology or enzymatic or chemical cleavage methods), and such antigen-binding fragments are screened for specificity in the same manner as for intact antibodies.

As used herein, the term "identity" refers to the matching of sequences between two polypeptides or between two nucleic acids. To determine the percent identity of two amino acid sequences or two nucleic acid sequences, the sequences are aligned for optimal comparison purposes (*e.g.*, a gap may be introduced in the first amino acid or nucleic acid sequence for optimal alignment with the second amino acid or nucleic acid sequence). The amino acid residues or nucleotides at corresponding amino acid or nucleotide positions are then compared. When a position in the first sequence is occupied by the same amino acid residue or nucleotide as the corresponding position in the second sequence, the molecules are identical at the position. The percent identity between two sequences is a function of the number of identical positions shared by the sequences (*i.e.*, percent identity = number of identical overlapping positions/total number of positions × 100%). In certain embodiments, the two sequences used for alignment are of the same length.

The determination of percent identity between two sequences can also be achieved using mathematical algorithms. One non-limiting example of a mathematical algorithm for the comparison of two sequences is the algorithm of Karlin and Altschul, 1990, Proc. Natl. Acad. Sci. U.S.A. 87: 2264-2268, as modified in Karlin and Altschul, 1993, Proc. Natl. Acad. Sci. U.S.A. 90: 5873-5877. Such algorithms are incorporated into the NBLAST and XBLAST programs of Altschul et al., 1990, J. Mol. Biol. 215: 403.

As used herein, the term "variant", in the context of polypeptides (including polypeptides), also refers to a polypeptide or peptide comprising an amino acid sequence that has been altered by the introduction of amino acid residue substitutions, deletions, or additions. In certain cases, the term "variant" also refers to a polypeptide or peptide that has been modified (*i.e.*, by covalently linking any type of molecule to the polypeptide or peptide). For example, but not by way of limitation, the polypeptide may be modified, such as by glycosylation, acetylation, pegylation, phosphorylation, amidation, derivatization with known protecting/blocking groups, proteolytic cleavage, or attachment to a cellular ligand or other proteins. Derivatized polypeptides or peptides may be produced by chemical modifications using techniques known to those skilled in the art, including, but not limited to, specific chemical cleavage, acetylation, formylation, metabolic synthesis of tunicamycin, *etc*. Furthermore, variants have similar, identical, or improved functions compared to the polypeptide or peptide from which they are derived. In certain embodiments, the variant has at least 90%, at least 91%, at least 92%, at least 93%, at least 94%, at least 95%, at least 96%, at least 97%, at least 98%, or at least 99% sequence identity compared to the sequence from which it is derived.

As used herein, the term "specific binding" refers to a non-random binding reaction between two molecules, such as the reaction between an antibody and its corresponding antigen. The strength or affinity of a specific binding interaction may be expressed by the equilibrium dissociation constant (K_{D}) of the interaction. In the present disclosure, the term "K_{D}" refers to the equilibrium dissociation constant of a specific antibody-antigen interaction, which is used to describe the binding affinity between an antibody and an antigen. The smaller the equilibrium dissociation constant, the tighter the antibody-antigen binding, and the higher the affinity between the antibody and the antigen.

The specific binding properties between two molecules may be determined using methods well known in the art. One method involves measuring the rates of formation and dissociation of an antigen-antibody complex. Both the "association rate constant" (kas or kon) and the "dissociation rate constant" (kdis or koff) may be calculated from the concentrations and the actual rates of association and dissociation (see Malmqvist M, Nature, 1993, 361: 186-187). The ratio of kdis/kon is equal to the dissociation constant K_{D} (see Davies et al., Annual Rev Biochem, 1990; 59: 439-473). K_{D}, kon, and kdis values may be measured by any effective method. In certain embodiments, the dissociation constant may be measured using surface plasmon resonance (SPR) in Biacore, and the dissociation constant may also be measured by bio-layer interferometry or KinExA. In some embodiments, the fusion protein of the present disclosure binds to human TIGIT protein with a K_{D} value of no greater than 1E-8 M, 2E-8 M, 3E-8 M, 4E-8 M, 5E-8 M, 6E-8 M, 7E-8 M, 8E-8 M, 9E-8 M, 1E-9 M, 2E-9 M, 3E-9 M, 4E-9 M, 5E-9 M, 6E-9 M, 7E-9 M, 8E-9 M, 9E-9 M, 1E-10 M, 2E-10 M, 3E-10 M, 4E-10 M, or 5E-10 M. In some embodiments, the fusion protein of the present disclosure binds to human PD-1 protein with a K_{D} value of no greater than 1E-8 M, 2E-8 M, 3E-8 M, 4E-8 M, 5E-8 M, 6E-8 M, 7E-8 M, 8E-8 M, 9E-8 M, 1E-9 M, 2E-9 M, 3E-9 M, 4E-9 M, 5E-9 M, 6E-9 M, 7E-9 M, 8E-9 M, 9E-9 M, 1E-10 M, 2E-10 M, 3E-10 M, 4E-10 M, or 5E-10 M.

As used herein, the term "vector" refers to a nucleic acid delivery vehicle into which a polynucleotide may be inserted. When a vector enables the expression of a protein encoded by the inserted polynucleotide, it is referred to as an expression vector. A vector may be transformed, transduced, or transfected into a host cell, allowing the genetic elements carried by the vector to be expressed in the host cell. Vectors are well known to those skilled in the art and include, but are not limited to: plasmids; phagemids; cosmids; artificial chromosomes, such as yeast artificial chromosomes (YACs), bacterial artificial chromosomes (BACs), or P1-derived artificial chromosomes (PACs); bacteriophages, such as lambda phage or M13 phage; and animal viruses. Animal viruses that may be used as vectors include, but are not limited to, retroviruses (including lentiviruses), adenoviruses, adeno-associated viruses, herpes viruses (*e.g.*, herpes simplex viruses), poxviruses, baculoviruses, papillomaviruses, and papovaviruses (*e.g.*, SV40). A vector may contain various elements that control expression, including, but not limited to, promoter sequences, transcription initiation sequences, enhancer sequences, selection elements, and reporter genes. Additionally, the vector may also contain an origin of replication.

As used herein, the term "host cell" refers to a cell that may be used for introducing a vector, including, but not limited to, prokaryotic cells such as *Escherichia coli* or *Bacillus subtilis*; fungal cells such as yeast cells or *Aspergillus*; insect cells such as S2 *Drosophila* cells or Sf9 cells; or human or animal cells such as fibroblasts, CHO cells, COS cells, NSO cells, HeLa cells, BHK cells, or HEK 293 cells.

The abbreviations for the twenty conventional amino acids mentioned herein follow conventional usage. See, for example, Immunology - A Synthesis (2nd Edition, E. S. Golub and D. R. Gren, Eds., Sinauer Associates, Sunderland, Mass. (1991)), which is incorporated herein by reference. In the present disclosure, the terms "polypeptide" and "protein" have the same meaning and are used interchangeably. In the present disclosure, amino acids are typically represented by one-letter and three-letter abbreviations well known in the art. For example, alanine may be represented by A or Ala.

As used herein, the term "pharmaceutically acceptable carrier and/or excipient" refers to a carrier and/or excipient that is pharmacologically and/or physiologically compatible with the subject and the active ingredient, which is well known in the art (see, for example, Remington's Pharmaceutical Sciences. Edited by Gennaro AR, 19th ed. Pennsylvania: Mack Publishing Company, 1995), and includes, but is not limited to: pH adjusters, surfactants, adjuvants, ionic strength enhancers, diluents, agents for maintaining osmotic pressure, agents for delaying absorption, and preservatives. Pharmaceutically acceptable carriers and/or excipients include any and all solvents, dispersion media, isotonic agents, absorption delaying agents, and the like that are physiologically compatible. Pharmaceutically acceptable carriers suitable for use in the present disclosure may be sterile liquids, such as water and oils, including those of petroleum, animal, vegetable, or synthetic origin, such as peanut oil, soybean oil, mineral oil, or sesame oil. Water is a preferred carrier when the pharmaceutical composition is administered intravenously. Saline solutions, aqueous dextrose solutions, and glycerol solutions may also be used as liquid carriers, particularly for injectable solutions. Suitable excipients include starch, glucose, lactose, sucrose, gelatin, malt, rice, flour, chalk, silica gel, sodium stearate, glyceryl monostearate, talc, sodium chloride, dried skim milk, glycerol, propylene glycol, water, ethanol, *etc*. For the use and application of excipients, also refer to "Handbook of Pharmaceutical Excipients" (5th Edition, R.C. Rowe, P.J. Seskey, and S.C. Owen, Pharmaceutical Press, London and Chicago). If desired, the composition may also contain minor amounts of wetting agents or emulsifying agents, or pH buffers. These compositions may be formulated in the form of solutions, suspensions, emulsions, tablets, pills, capsules, powders, sustained-release formulations, and the like. Oral formulations may include standard pharmaceutically acceptable carriers and/or excipients, such as pharmaceutical-grade mannitol, lactose, starch, magnesium stearate, or saccharin. The pharmaceutical preparation or pharmaceutical composition according to the present disclosure may be prepared by mixing the fusion protein or nucleic acid for expression of the present disclosure, having the desired purity, with one or more optional pharmaceutically acceptable excipients (Remington's Pharmaceutical Sciences, 16th Edition, Osol, A. ed. (1980)), preferably in the form of a lyophilized formulation or an aqueous solution. The pharmaceutical composition or preparation of the present disclosure may further comprise more than one active ingredient, wherein the active ingredients are required for a particular indication being treated, preferably those with complementary activities that do not adversely affect one another. For example, it may be desirable to further comprise other anti-infective active ingredients, such as other antibodies, anti-infective agents, small-molecule drugs, or immunomodulators. The active ingredients are suitably combined in amounts effective for the intended purpose. Sustained-release formulations may be prepared. Suitable examples of sustained-release formulations include semi-permeable matrices of solid hydrophobic polymers containing the antibody of the present disclosure or an antigen-binding fragment, wherein the matrices are in the form of shaped articles, such as films or microcapsules.

As used herein, the term "prevention" refers to a method implemented to inhibit or delay the onset of a disease or condition or symptom in a subject. As used herein, the term "treatment" refers to a method implemented to obtain beneficial or desired clinical outcomes. For the purpose of the present disclosure, beneficial or desired clinical outcomes include, but are not limited to, alleviation of symptoms, reduction in the extent of disease, stabilization (*i.e.*, no worsening) of the disease state, delay or slowing of disease progression, improvement or alleviation of the disease state, and remission of symptoms (whether partial or complete), whether detectable or undetectable. Furthermore, "treatment" may also refer to an extended survival period compared to the expected survival period (if not treated). As used herein, treatment may include neoadjuvant treatment and/or adjuvant treatment. "Neoadjuvant treatment" refers to a therapy that is administered to a patient prior to a planned surgery for treating the disease. "Adjuvant treatment" refers to a therapy that is administered to a patient after a surgery for treating the disease.

As used herein, the term "subject" refers to a mammal, such as a primate mammal, such as a human. In certain embodiments, the subject (*e.g.*, a human) suffers from a tumor, an inflammatory disease, or an autoimmune disease, or is at risk of developing the diseases.

As used herein, the term "effective amount" refers to an amount sufficient to achieve or at least partially achieve the desired effect. For example, an effective amount for preventing a disease (*e.g*., a tumor, an inflammatory disease, or an autoimmune disease) refers to an amount sufficient to prevent, inhibit, or delay the onset of a disease (*e.g*., a tumor, an inflammatory disease, or an autoimmune disease); an effective amount for treating a disease refers to an amount sufficient to cure or at least partially arrest a disease and its complications in a patient who already suffers from the disease. The determination of such an effective amount is well within the competence of those skilled in the art. For example, the effective amount for therapeutic use will depend on factors such as the severity of the disease to be treated, the overall state of the immune system of the patient, the general condition of the patient (*e.g.*, age, weight, and gender), the route of administration of the medicament, and any other therapies administered concurrently.

### Beneficial effects of the present disclosure

Compared to the limitations in efficacy of known immune checkpoint anti-tumor drugs, the bispecific antibody-cytokine fusion protein targeting TIGIT and PD-1 with immune effector cell activation provided by the present disclosure exhibits significant advantages. The bispecific antibody-cytokine fusion protein provided by the present disclosure can effectively relieve immunosuppression in T cells and NK cells, moderately promote the activation and proliferation of immune cells infiltrating tumors, overcome the poor efficacy associated with TIGIT monotherapy, and simultaneously combine with an engineered IL-15/IL-15Rα complex to broaden the therapeutic window while reducing the toxicity of IL-15, thereby providing good medication safety and anti-tumor efficacy.

The embodiments of the present disclosure will be described in detail below in conjunction with the accompanying drawings and examples. However, those skilled in the art will understand that the following drawings and examples are provided for illustrative purposes only and are not intended to limit the scope of the present disclosure. Various objects and advantageous aspects of the present disclosure will become apparent to those skilled in the art from the following detailed description of the drawings and preferred embodiments.

The present disclosure is now described with reference to the following examples, which are intended to illustrate, rather than limit, the present disclosure.

Those skilled in the art will appreciate that the examples describe the present disclosure by way of illustration and are not intended to limit the scope of protection claimed in the present disclosure. Unless otherwise specified, the experimental methods described in the examples are conventional methods. Experiments for which specific conditions are not specified in the examples are carried out according to conventional conditions or the conditions recommended by the manufacturer. Reagents or instruments for which the manufacturer is not specified are conventional products that are commercially available.

### Example 1: Construction of anti-TIGIT monoclonal antibodies and anti-PD-1 monoclonal antibodies

### 1.1 Acquisition of anti-TIGIT monoclonal antibodies and their encoding sequences

The anti-TIGIT antibodies are multiple anti-TIGIT monoclonal antibodies invented by the applicants. The preparation methods and amino acid sequences of these antibodies are disclosed in Chinese patent application No. CN202310994050.5, entitled "ANTI-TIGIT ANTIBODY AND USE THEREOF", filed on August 9, 2023.

Specifically, according to conventional methods, 100 µg of human TIGIT extracellular domain antigen huTIGIT-msIgG2a-Fc-tag (purchased from ACROBiosystems; TIT-H5253) was fully emulsified with Freund's complete adjuvant at a 1:1 ratio and administered to mice via intraperitoneal injection for immunization, followed by booster immunization via tail vein injection with the antigen without Freund's adjuvant. Splenocytes were then harvested from the immunized mice. The splenocytes were fused with Sp2/0 cells (Nanjing Cobioer Biosciences, CBP60881). After fusion, positive clones were screened by ELISA using human TIGIT-His (huTIGIT-His, KACTUS, TIG-HM110) and monkey TIGIT-His (cynoTIGIT-His, ACROBiosystems; TIT-C5223). Further screening was performed using Jurkat-huTIGIT cells (prepared by infecting Jurkat cells (Nanjing Cobioer Biosciences, CBP60520) at 60% confluence in a 6-well plate with 10 µL of lentivirus encoding the human TIGIT (huTIGIT) target gene (SEQ ID NO: 77) (customized by Hanbio), culturing for 24 hours, replacing the medium with fresh medium containing puromycin at a final working concentration of 10 µg/mL for 4-day antibiotic selection, followed by expansion and cryopreservation of the selected positive polyclonal cells) and 293T-cynoTIGIT cells (prepared similarly to Jurkat-huTIGIT cells by infecting 293T cells (Nanjing Cobioer Biosciences, CBP60439) with lentivirus encoding the cynomolgus monkey TIGIT (cynoTIGIT) target gene (SEQ ID NO: 78)). Positive clones with strong binding to both cell types were screened by FACS (flow cytometry). Clones positive in both ELISA and FACS were subcloned to obtain monoclonal antibodies. Similarly, positive monoclonal antibodies were screened by ELISA and flow cytometry, and their NF-κB signaling pathway activation was further evaluated to screen for functional anti-TIGIT positive clones, resulting in murine antibodies designated 3A03A03, 2IT1-10A3-2H6, and 41A07A03.

Total RNA was extracted from the hybridoma cells and reverse-transcribed. Using the resulting cDNA as a template, VH and VL sequences of antibodies 3A03A03, 2IT1-10A3-2H6, and 41A07A03 were amplified. The VH sequences were ligated to the human IgG1 heavy chain constant region (SEQ ID NO: 70), and the VL sequences were ligated to the human kappa light chain constant region (SEQ ID NO: 73), resulting in chimeric antibody 7TI-042 prepared from 3A03A03, chimeric antibody 7TI-027 prepared from 2IT1-10A3-2H6, and chimeric antibody 7TI-046 prepared from 41A07A03.

**Table 1: VH and VL amino acid sequences of murine/chimeric anti-TIGIT antibodies**

| Description | | SEQ ID NO: | Sequence |
|---|---|---|---|
| 3A03A03/7TI-04 2 heavy chain variable region | Full-length sequence | 1 | EVQLQQSGGGLVKPGGSLKLSCAASGFTFSDYGMHWFRQ APEKGLEWVAYISSGSSTIYYADTVKGRFTISRDNAKDTLFL QMTSLRSEDTAMYYCARYRFDAYAMDYWGQGTTVTVSS |
| | CDR-H1 | 3 | GFTFSDYGMH |
| | CDR-H2 | 4 | YISSGSSTIYYADTVKG |
| | CDR-H3 | 5 | ARYRFDAYAMDY |
| 3A03A03/7TI-04 2 light chain variable region | Full-length sequence | 2 | DIVMTQAASSLSASLGDRVTISCRASQDISNYLNWYQQKPD GAVKLLIYYKSRLHSGVPSRFSGSGSGTDYSLTISNLEPEDIA TYYCQHFRKVPWTFGGGTKLEIK |
| | CDR-L1 | 6 | RASQDISNYLN |
| | CDR-L2 | 7 | YKSRLHS |
| | CDR-L3 | 8 | QHFRKVPWT |
| 2IT1-10A3-2H6/ 7TI-027 heavy chain variable region | Full-length sequence | 9 | EVKLVESGAELLKPGASVKLSCKTSGYTFTSYWINWVKQR PGQGLEWIGNIYPALDSTNYNEKFKSKATLTVDTSSSTAYM QLNSLTSDDSAVYYCARLRQFGLFDQWGQGTTVTVSS |
| | CDR-H1 | 11 | GYTFTSYWIN |
| | CDR-H2 | 12 | NIYPALDSTNYNEKFKS |
| | CDR-H3 | 13 | ARLRQFGLFDQ |
| 2IT1-10A3-2H6/ 7TI-027 light chain variable region | Full-length sequence | 10 | DIVMTQSPTIMSASPGEKVTMTCSASSGVSYMHWYLQKSG TSPKLLIFDTSKLASGVPARFSGSGSGTSYSLTISSMEAEDAA TYYCQQWSNNPLTFGGGTKLEIK |
| | CDR-L1 | 14 | SASSGVSYMH |
| | CDR-L2 | 15 | DTSKLAS |
| | CDR-L3 | 16 | QQWSNNPLT |
| 41A07A03/7TI-0 46 heavy chain variable region | Full-length sequence | 17 | EVQLKESGGGLVKPGGSLKLSCAASGFTFSSYAMSWVRQT PERRLEWVAFISSGSSTIFYADTVEGRFTISRDNANNTLFLQ MTSLRSEDTAMYYCARRSLDYYAMDYWGQGTTVTVSS |
| | CDR-H1 | 19 | GFTFSSYAMS |
| | CDR-H2 | 20 | FISSGSSTIFYADTVEG |
| | CDR-H3 | 21 | ARRSLDYYAMDY |
| 41A07A03/7TI-0 46 light chain variable region | Full-length sequence | 18 | EIVLTQSPSSLSASLGDRVTISCRASQDISNFLNWYQQKPDG TVKLLIYYTSRLHSGVPSRFSGSGSGTDYSLTISNLEQEDIAT YFCQQNSKHPWTFGGGTKLEIK |
| | CDR-L1 | 22 | RASQDISNFLN |
| | CDR-L2 | 23 | YTSRLHS |
| | CDR-L3 | 24 | QQNSKHPWT |

The ability of chimeric anti-TIGIT antibodies to bind to huTIGIT-his and cynoTIGIT-his proteins was measured by SPR (surface plasmon resonance). The results demonstrated that 7TI-042, 7TI-027, and 7TI-046 were capable of binding to human TIGIT protein with K_{D} values of 1.5E-10 M, 9.26E-12 M, and 5.2E-11 M, respectively, and binding to monkey TIGIT protein with K_{D} values of 2.3E-08 M, 2.06E-10 M, and 5.8E-10 M, respectively.

The binding of chimeric anti-TIGIT antibodies to Jurkat-huTIGIT cells and 293T-cynoTIGIT cells was measured by flow cytometry. The results demonstrated that 7TI-042, 7TI-027, and 7TI-046 were capable of binding to Jurkat-huTIGIT cells with EC₅₀ values of 0.47 nM, 0.45 nM, and 0.66 nM, respectively, and binding to 293T-cynoTIGIT cells with EC₅₀ values of 0.28 nM, 0.14 nM, and 0.21 nM, respectively.

The ability of chimeric anti-TIGIT antibodies to compete with CD155 for binding to human TIGIT antigen and to activate downstream reporter genes was evaluated using Jurkat-NFAT-LUC-huTIGIT effector cells (Nanjing Cobioer Biosciences, CBP74020) and CHO-TCR-CD155 target cells (Nanjing Cobioer Biosciences, CBP74073). The results demonstrated that 7TI-042, 7TI-027, and 7TI-046 were capable of activating the reporter gene with EC₅₀ values of 2.21 nM, 3.17 nM, and 3.46 nM, respectively.

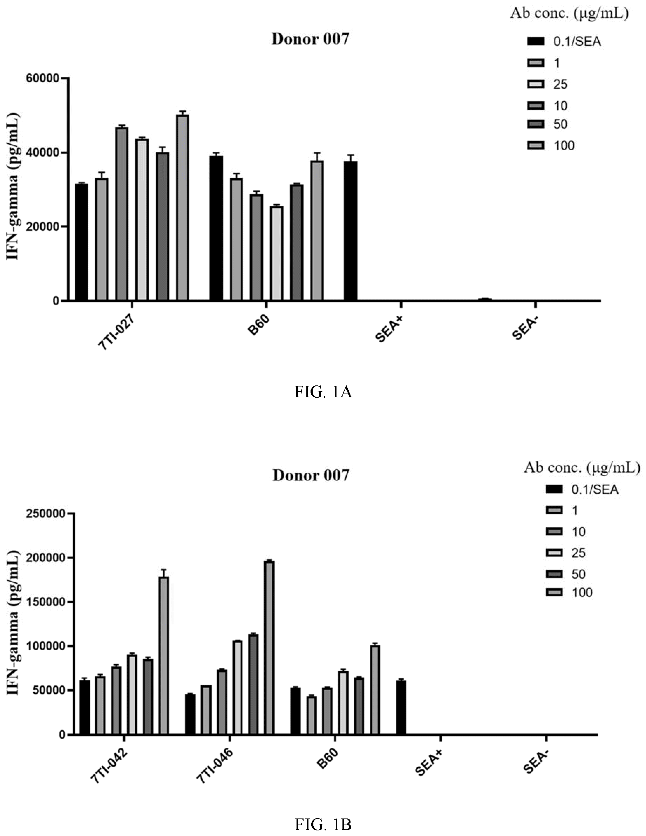

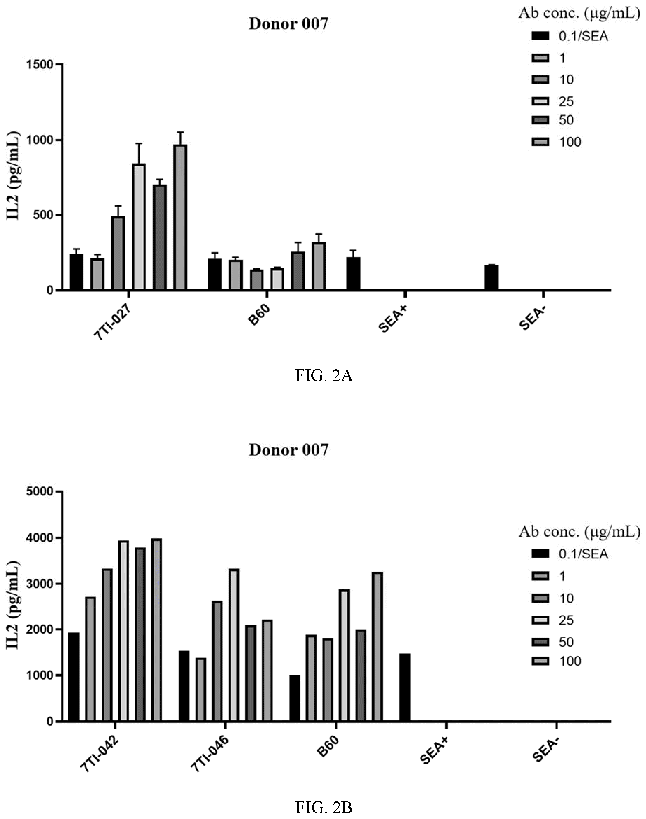

Human PBMCs (Donor: 007; SC12007) purchased from Sailybio were co-incubated with SEA (staphylococcal enterotoxin A, purchased from Toxin Technology, AT101) and chimeric anti-TIGIT antibodies, and the concentrations of IFN-γ and IL-2 were measured. The IFN-γ results are shown in FIG. 1A and FIG. 1B, and the IL-2 results are shown in FIG. 2A and FIG. 2B. As shown in the figures, the chimeric anti-TIGIT antibodies of the present disclosure can effectively promote IFN-γ and IL-2 secretion.

### 1.2 Humanization of murine anti-TIGIT antibodies

Murine antibodies 3A03A03, 2IT1-10A3-2H6, and 41A07A03 were humanized by comparing their amino acid sequences with the amino acid sequences of human germline antibodies. The sequences with high homology and favorable physicochemical properties were selected as humanized antibody framework sequences. The CDRs of the murine antibodies were grafted onto the humanized antibody framework sequences to obtain humanized anti-TIGIT antibodies with the following VH and VL sequences.

**Table 2: VH and VL amino acid sequences of humanized anti-TIGIT antibodies**

| Source of humanized antibody | Description of humanized antibody | SEQ ID NO: | Sequence |
|---|---|---|---|
| 3A03A03 | 7TI-208 heavy chain variable region | 41 | QVQLVESGGGLVKPGGSLRLSCAASGFTFSDYGMHWFRQ APGKGLEWVSYISSGSSTIYYADTVKGRFTISRDNAKNSLY LQMNSLRAEDTAVYYCARYRFDAYAMDYWGQGTLVTVSS |
| | 7TI-208 light chain variable region | 42 | DIQMTQSPSSLSASVGDRVTITCRASQDISNYLNWYQQKPG GAVKLLIYYKSRLHSGVPSRFSGSGSGTDYTLTISSLQPEDIA TYYCQHFRKVPWTFGQGTKVEIK |
| | 7TI-202 heavy chain variable region | 43 | QVQLVESGGGLVKPGGSLRLSCAASGFTFSDYGMHWIRQA PGKGLEWVSYISSGSSTIYYADTVKGRFTISRDNAKNSLYL QMNSLRAEDTAVYYCARYRFDAYAMDYWGQGTLVTVSS |
| | 7TI-202 light chain variable region | 44 | DIQMTQSPSSLSASVGDRVTITCRASQDISNYLNWYQQKPG GAPKLLIYYKSRLHSGVPSRFSGSGSGTDYTLTISSLQPEDIA TYYCQHFRKVPWTFGQGTKVEIK |
| 2IT1-10A 3-2H6 | 7TI-172/7TI-174 heavy chain variable region | 45 | QVQLVQSGAEVKKPGASVKVSCKASGYTFTSYWINWVRQ APGQGLEWMGNIYPALDSTNYNEKFKSRVTMTVDTSTSTA YMELRSLRSDDTAVYYCARLRQFGLFDQWGQGTLVTVSS |
| | 7TI-172 light chain variable region | 46 | EIVMTQSPATLSLSPGERATLSCSASSGVSYMHWYQQKPGQ APRLLIYDTSKLASGIPARFSGSGSGTDYTLTISSLEPEDFAV YYCQQWSNNPLTFGGGTKVEIK |
| | 7TI-174 light chain variable region | 47 | EIVMTQSPATLSLSPGERATLSCSASSGVSYMHWYQQKPGQ APRLLIFDTSKLASGIPARFSGSGSGTDYTLTISSMEPEDFAV YYCQQWSNNPLTFGGGTKVEIK |
| 41A07A03 | 7TI-225/7TI-224 heavy chain variable region | 48 | EVQLVESGGGLVQPGGSLRLSCAASGFTFSSYAMSWVRQA PGKGLEWVAFISSGSSTIFYADTVEGRFTISRDNAKNSLYLQ MNSLRDEDTAVYYCARRSLDYYAMDYWGQGTLVTVSS |
| | 7TI-225 light chain variable region | 49 | DIQLTQSPSSLSASVGDRVTITCRASQDISNFLNWYQQKPGG APKLLLYYTSRLHSGVPSRFSGSGSGTDYTLTISSLQPEDFAT YYCQQNSKHPWTFGQGTKVEIK |
| | 7TI-224 light chain variable region | 50 | DIQMTQSPSSLSASVGDRVTITCRASQDISNFLNWYQQKPG KAPKLLLYYTSRLHSGVPSRFSGSGSGTDYTLTISSLQPEDF ATYYCQQNSKHPWTFGQGTKVEIK |

The ability of humanized anti-TIGIT antibodies to bind to huTIGIT-his protein was measured by SPR (surface plasmon resonance). The results demonstrated that 7TI-208, 7TI-202, 7TI-172, 7TI-174, 7TI-224, and 7TI-225 were capable of binding to human TIGIT protein with K_{D} values of 1.1E-10 M, 1.7E-09 M, 3.80E-12 M, 2.70E-11 M, 1.99E-10 M, and 1.42E-10 M, respectively.

The binding of humanized anti-TIGIT antibodies to Jurkat-huTIGIT cells was measured by flow cytometry. The results demonstrated that 7TI-208, 7TI-202, 7TI-172, 7TI-174, 7TI-224, and 7TI-225 were capable of binding to Jurkat-huTIGIT cells with EC₅₀ values of 0.19 nM, 1.82 nM, 7.51 nM, 1.13 nM, 0.18 nM, and 0.18 nM, respectively.

The ability of humanized anti-TIGIT antibodies to compete with CD155 for binding and to activate reporter genes was evaluated using Jurkat-NFAT-LUC-huTIGIT effector cells and CHO-TCR-CD155 target cells. The results demonstrated that 7TI-208, 7TI-172, and 7TI-174 were capable of activating the reporter gene with EC₅₀ values of 3.96 nM, 7.25 nM, and 4.22 nM, respectively.

### 1.3 Acquisition of anti-PD-1 monoclonal antibodies and their encoding sequences

The anti-PD-1 antibodies are multiple anti-PD-1 monoclonal antibodies invented by the applicants. The preparation methods and amino acid sequences of these antibodies are disclosed in Chinese patent application No. CN202311267267.2, entitled "ANTI-PD-1 ANTIBODY AND USE THEREOF", filed on September 27, 2023.

Specifically, according to conventional methods, recombinant human PD1-hFc protein (KACTUS, PD1-HM101) was emulsified with an equal volume of Freund's complete adjuvant and subcutaneously injected into the bilateral inguinal regions of BALB/c mice aged 6 to 8 weeks. Subsequently, the mice were subcutaneously injected every two weeks with Freund's incomplete adjuvant containing PD1-hFc protein (the protein and adjuvant were emulsified at a volume ratio of 1:1). A total of four immunizations were performed. One week after the fourth immunization, mice with antibody titers meeting the fusion criteria were boosted by intravenous injection of recombinant human PD1-hFc protein without adjuvant. Splenocytes were then harvested from the boosted mice. The splenocytes were fused with Sp2/0 cells (Nanjing Cobioer Biosciences, CBP60881). After fusion, positive clones were screened by ELISA using human PD-1 extracellular domain protein (ACROBiosystems Group, PD1-H5221) or cynomolgus monkey PD-1 extracellular domain protein (ACROBiosystems Group, PD1-C5223). Further screening was performed by FACS (flow cytometry) using CHO-K1-hPD-1 cells (CHO-K1 cells overexpressing human PD-1, which is a stable cell line obtained by infecting CHO-K1 cells with a lentiviral vector (customized by Hanbio) carrying human PD-1 (Gene ID: NM_005018.3)) to identify positive clones with strong binding to CHO-K1 cells expressing human PD-1, which were then subcloned to obtain monoclonal antibodies. Similarly, positive monoclonal antibodies were screened by ELISA and flow cytometry, resulting in murine antibodies designated 1KP-3A6-3C6 and 1KP1-1E10-F6.

Total RNA was extracted from the hybridoma cells and reverse-transcribed. Using the resulting cDNA as a template, VH and VL sequences of antibodies 1KP-3A6-3C6 and 1KP1-1E10-F6 were amplified. The VH sequences were ligated to the human IgG1 heavy chain constant region, and the VL sequences were ligated to the human kappa light chain constant region, resulting in chimeric antibodies 1KP1-3A6-3C6-hz and 1KP1-1E10-F6-hz.

**Table 3: VH and VL amino acid sequences of murine/chimeric anti-PD-1 antibodies**

| Description | | SEQ ID NO: | Sequence |
|---|---|---|---|
| 1KP-3A6-3C6/1 KP1-3A6-3C6-hz heavy chain variable region | Full-length sequence | 25 | EVQLQQSGPELVKPGASVKISCKASGYTFTHYYINWVKQR PGQGLEWIGWISPGSVNTKYNEKFKGKATLTVDTSSSTAYM QLSSLTSDDSAVYFCATEVYDYGDYWGQGTTVTVSS |
| | CDR-H1 | 27 | GYTFTHYYIN |
| | CDR-H2 | 28 | WISPGSVNTKYNEKFKG |
| | CDR-H3 | 29 | ATEVYDYGDY |
| 1KP-3A6-3C6/1 KP1-3A6-3C6-hz light chain variable region | Full-length sequence | 26 | DIVMTQSPKFMSTSVGDRVSVTCKASQNVGTNVAWYQQK PGQSPKALIFSASYRYNGVPDRFTGSGSGTDFTLTITNVQSE DLAEYFCQQYNIYPYTFGGGTKLEIK |
| | CDR-L1 | 30 | KASQNVGTNVA |
| | CDR-L2 | 31 | SASYRYN |
| | CDR-L3 | 32 | QQYNIYPYT |
| 1KP1-1E10-F6/1 KP1-1E10-F6-hz heavy chain variable region | Full-length sequence | 33 | EVQLQQSGPELVKPGASVKISCKASGYTFTDYYMNWVKQS HGKSLEWIGDINPKYGDTTYNLRFQDKATLTVDKSSSTAY MELRSLTSEDFAVYFCARGIRLFDYWGQGTTVTVSS |
| | CDR-H1 | 35 | GYTFTDYYMN |
| | CDR-H2 | 36 | DINPKYGDTTYNLRFQD |
| | CDR-H3 | 37 | ARGIRLFDY |
| 1KP1-1E10-F6/1 KP1-1E10-F6-hz light chain variable region | Full-length sequence | 34 | DIQMTQTTASLSASVGETVTITCRASGNIHNYLAWYQQKQ GKSPQILVYNAKTLAEGVPSRFSGSVSGTQYSLKINSLQPED FGSYYCHHYYTSPFTFGGGTKLEIK |
| | CDR-L1 | 38 | RASGNIHNYLA |
| | CDR-L2 | 39 | NAKTLAE |
| | CDR-L3 | 40 | HHYYTSPFT |

The ability of chimeric anti-PD-1 antibodies to bind to human PD-1 protein and cynomolgus monkey PD-1 protein was measured by SPR (surface plasmon resonance). The results demonstrated that 1KP1-3A6-3C6-hz and 1KP1-1E10-F6-hz were capable of binding to human PD-1 protein with K_{D} values of 3.20E-10 M and 3.93E-10 M, respectively, and binding to cynomolgus monkey PD-1 protein with K_{D} values of 3.87E-09 M and 2.6E-09 M, respectively.

The antigen-binding ability of chimeric anti-PD-1 antibodies to CHO-K1-hPD-1 cells and CHO-K1-cynoPD-1 cells (CHO-K1 cells overexpressing cynomolgus monkey PD-1, which is a stable cell line obtained by infecting CHO-K1 cells with a lentiviral vector carrying cynomolgus monkey PD-1 (Gene ID: NM_001114358.1)) was measured by flow cytometry. The results demonstrated that 1KP1-3A6-3C6-hz and 1KP1-1E10-F6-hz were capable of binding to CHO-K1 cells expressing human PD-1 with EC₅₀ values of 2.558 nM and 9.13 nM, respectively, and binding to CHO-K1 cells expressing cynomolgus monkey PD-1 with EC₅₀ values of 2.075 nM and 1.208 nM, respectively.

The ability of chimeric anti-PD-1 antibodies to activate effector cells was evaluated using CHO-hPDL1-TCR cells overexpressing human PD-L1 (GenScript, M00613) as target cells and Jurkat/NFAT-luc/PD1 cells (GenScript, M00612) as effector cells. The results demonstrated that 1KP1-3A6-3C6-hz and 1KP1-1E10-F6-hz were capable of activating effector cells with EC₅₀ values of 0.192 nM and 0.43 nM, respectively.

The competitive binding effect of chimeric anti-PD-1 antibodies in blocking the binding of PD-L1/PD-L2 to PD-1 was evaluated using CHO-K1 cells overexpressing human PD-1, biotin-labeled PD-L1 (329703, Biolegend), mouse Fc-labeled PD-L2 (329604, Biolegend), AF647-labeled goat anti-mouse IgG Fc secondary antibody (Jackson ImmunoResearch, 115-605-008), and APC-labeled SA (streptavidin). The results demonstrated that 1KP1-3A6-3C6-hz and 1KP1-1E10-F6-hz were capable of blocking the binding of human PD-L1 to human PD-1 on the surface of CHO-K1 cells with EC₅₀ values of 1.868 nM and 1.27 nM, respectively; 1KP1-1E10-F6-hz was capable of blocking the binding of human PD-L2 to human PD-1 on the surface of CHO-K1 cells with an EC₅₀ of 1.209 nM.

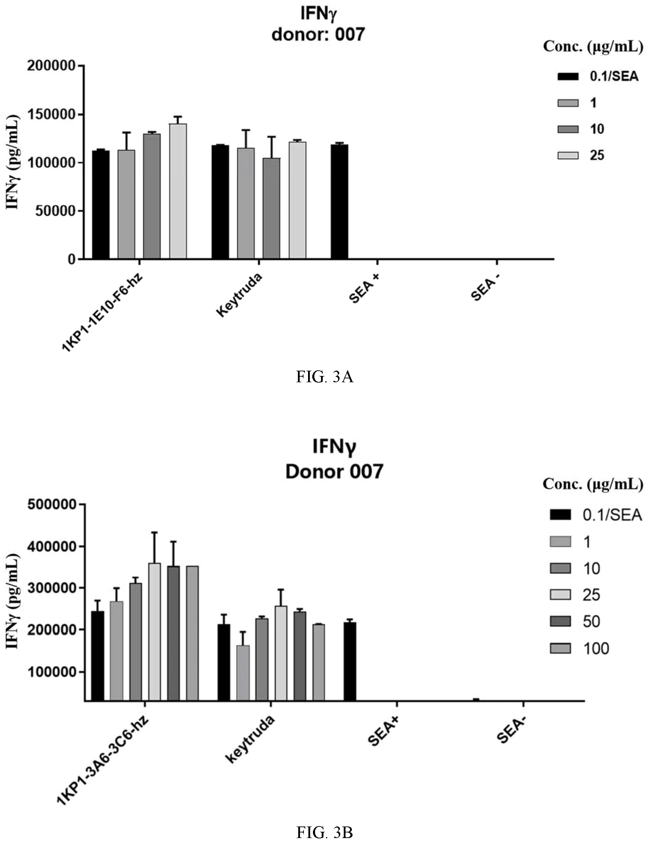

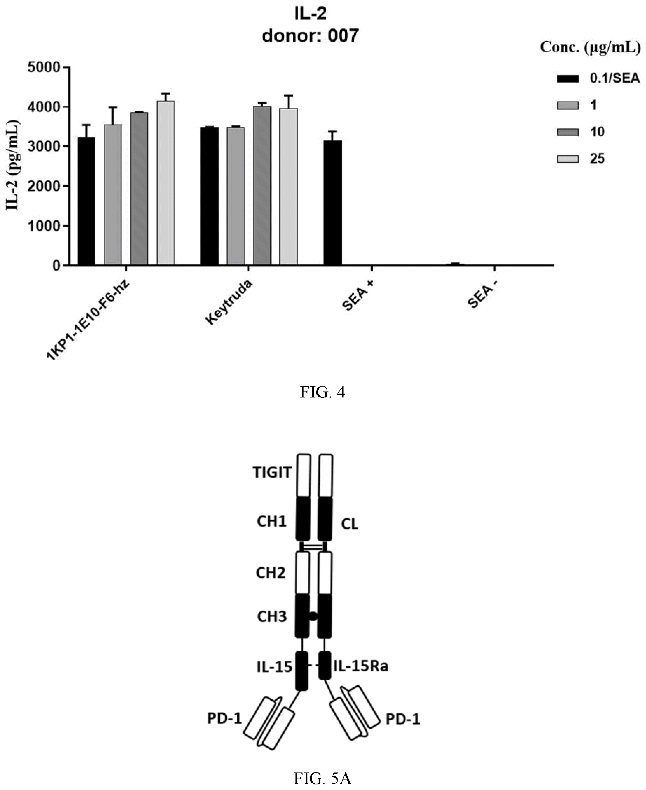

Human PBMCs (Donor: 007) purchased from Sailybio were co-incubated with SEA (staphylococcal enterotoxin A, purchased from Toxin Technology, AT101) and chimeric anti-PD-1 antibodies, and the concentrations of IFN-γ and IL-2 were measured. The IFN-γ results are shown in FIG. 3A and FIG. 3B, and the IL-2 results are shown in FIG. 4. As shown in the figures, the chimeric anti-PD-1 antibodies of the present disclosure can effectively promote IFN-γ and IL-2 secretion.

### 1.4 Humanization of murine anti-PD-1 antibodies

Murine antibodies 1KP1-3A6-3C6 and 1KP1-1E10-F6 were humanized by comparing their amino acid sequences with the amino acid sequences of human germline antibodies. The sequences with high homology and favorable physicochemical properties were selected as humanized antibody framework sequences. The CDRs of the murine antibodies were grafted onto the humanized antibody framework sequences to obtain humanized anti-PD-1 antibodies with the following VH and VL sequences.

**Table 4: VH and VL amino acid sequences of humanized anti-PD-1 antibodies**

| Source of humanized antibody | Description of humanized antibody | SEQ ID NO: | Sequence |
|---|---|---|---|
| 1KP1-1E10-F6 | 7PD-079 heavy chain variable region | 51 | QVQLVQSGAEVKKPGASVKVSCKASGYTFTDYYMNWVR QAPGQGLEWMGDINPKYGDTTYNLRFQDRVTMTRDTSTS TVYMELSSLRSEDTAVYYCARGIRLFDYWGQGTLVTVSS |
| | 7PD-079/7PD-082 light chain variable region | 52 | DIQMTQSPSSLSASVGDRVTITCRASGNIHNYLAWYQQKPG KAPKLLLYNAKTLAEGVPSRFSGSGSGTDYTLTISSLQPEDF ATYYCHHYYTSPFTFGGGTKVEIK |
| | 7PD-082 heavy chain variable region | 53 | QVQLVQSGAEVKKPGASVKVSCKASGYTFTDYYMNWVR QAPGQGLEWMGDINPKYGDTTYNLRFQDRVTMTVDTSTS TVYMELSSLRSEDTAVYYCARGIRLFDYWGQGTLVTVSS |
| 1KP1-3A6-3C6 | 7PD-111 heavy chain variable region | 54 | QVQLVQSGAEVKKPGASVKVSCKASGYTFTHYYINWVRQ APGQGLEWMGWISPGSVNTKYNEKFKGRVTMTVDTSISTA YMELSRLRSDDTAVYFCATEVYDYGDYWGQGTLVTVSS |
| | 7PD-111 light chain variable region | 55 | DIQMTQSPSSLSASVGDRVTITCKASQNVGTNVAWYQQKP EKSPKALIFSASYRYNGVPSRFSGSGSGTDFTLTISSLQPEDF ATYFCQQYNIYPYTFGQGTKLEIK |
| | 7PD-118/7PD-1 20 heavy chain variable region | 56 | QVQLVQSGAEVVKPGASVKVSCKASGYTFTHYYINWVRQ RPGQGLEWIGWISPGSVNTKYNEKFKGRVTLTVDTSISTAY MELSRLRSDDTAVYYCATEVYDGDYWGOGTLVTVSS |
| | 7PD-118/7PD-0 98 light chain variable region | 57 | DIQMTQSPSSLSASVGDRVTITCKASQNVGTNVAWYQQKP EKAPKSLIFSASYRYNGVPSRFSGSGSGTDFTLTISSLQPEDF ATYYCQQYNIYPYTFGQGTKLEIK |
| | 7PD-115 heavy chain variable region | 58 | QVQLVQSGAEVVKPGASVKVSCKASGYTFTHYYINWVRQ APGQGLEWIGWISPGSVNTKYNEKFKGRVTMTVDTSISTAY MELSRLRSDDTAVYYCATEVYDYGDYWGQGTLVTVSS |
| | 7PD-115/7PD-1 20 light chain variable region | 59 | DIQMTQSPSSLSASVGDRVTITCKASQNVGTNVAWYQQKP EKSPKALIFSASYRYNGVPSRFSGSGSGTDFTLTISSLQPEDF ATYYCQQYNIYPYTFGQGTKLEIK |
| | 7PD-098/7PD-099 heavy chain variable region | 60 | QVQLVQSGAEVKKPGASVKVSCKASGYTFTHYYINWVRQ APGQGLEWMGWISPGSVNTKYNEKFKGRVTMTRDTSISTA YMELSRLRSDDTAVYYCATEVYDYGDYWGQGTLVTVSS |
| | 7PD-099/7PD-104 light chain variable region | 61 | DIQMTQSPSSLSASVGDRVTITCKASQNVGTNVAWYQQKP EKAPKALIFSASYRYNGVPSRFSGSGSGTDFTLTISSLQPEDF ATYYCQQYNIYPYTFGQGTKLEIK |
| | 7PD-104 heavy chain variable region | 62 | QVQLVQSGAEVKKPGASVKVSCKASGYTFTHYYINWVRQ APGQGLEWMGWISPGSVNTKYNEKFKGRVTMTVDTSISTA YMELSRLRSDDTAVYYCATEVYDYGDYWGQGTLVTVSS |

The ability of humanized anti-PD-1 antibodies to bind to human PD-1 protein was measured by SPR (surface plasmon resonance). The results demonstrated that 7PD-079, 7PD-082, and 7PD-118 were capable of binding to human PD-1 protein with K_{D} values of 3.0E-09 M, 4.6E-09 M, and 6.47E-10 M, respectively; 7PD-118 was capable of binding to cynomolgus monkey PD-1 protein with a K_{D} of 1.03E-09 M.

The ability of chimeric anti-PD-1 antibodies to bind to CHO-K1 cells expressing human PD-1 and cynomolgus monkey PD-1 was measured by flow cytometry. The results demonstrated that 7PD-079, 7PD-082, 7PD-098, 7PD-099, 7PD-104, 7PD-111, 7PD-115, 7PD-118, and 7PD-120 were capable of binding to CHO-K1 cells expressing human PD-1 with EC₅₀ values of 1.363 nM, 1.597 nM, 1.131 nM, 1.248 nM, 1.029 nM, 1.207 nM, 1.189 nM, 1.133 nM, and 0.726 nM, respectively, and binding to CHO-K1 cells expressing cynomolgus monkey PD-1 with EC₅₀ values of 1.658 nM, 1.686 nM, 1.747 nM, 1.522 nM, 0.148 nM, 2.550 nM, 2.957 nM, 4.029 nM, and 1.694 nM, respectively.

### Example 2: Construction of expression vectors for TIGIT/PD-1/IL-15 bispecific antibody-cytokine fusion proteins

The TIGIT/PD-1/IL-15 bispecific antibody-cytokine fusion proteins described in this example have a total of four structures, namely structure I, structure II, structure III, and structure IV.

As shown in FIG. 5A, structure I consists of two chains: chain 1, comprising from the N-terminus to the C-terminus: an anti-TIGIT antibody heavy chain variable region, a human IgG1 heavy chain constant region CH1 (SEQ ID NO: 74), hinge region 1 (SEQ ID NO: 65), human IgG1 heavy chain constant regions CH2 and CH3 containing a Knob mutation (SEQ ID NO: 71), Linker 1 (SEQ ID NO: 67), an IL-15 polypeptide (SEQ ID NO: 63), Linker 1, an anti-PD-1 antibody light chain variable region, Linker 2 (SEQ ID NO: 68), and an anti-PD-1 antibody heavy chain variable region; chain 2, comprising from the N-terminus to the C-terminus: an anti-TIGIT antibody light chain variable region, a human IgG1 light chain constant region CL (SEQ ID NO: 73), hinge region 2 (SEQ ID NO: 66), human IgG1 heavy chain constant regions CH2 and CH3 containing a Hole mutation (SEQ ID NO: 72), Linker 1, an IL-15Rα polypeptide (SEQ ID NO: 64 or 75), Linker 1, an anti-PD-1 antibody light chain variable region, Linker 2, and an anti-PD-1 antibody heavy chain variable region.

Structure II is shown in a and b of FIG. 5B. As shown in a of FIG. 5B, structure II consists of two chains: chain 1, comprising from the N-terminus to the C-terminus: an anti-PD-1 antibody heavy chain variable region, a human IgG1 heavy chain constant region CH1, hinge region 1, human IgG1 heavy chain constant regions CH2 and CH3 containing a Knob mutation, Linker 1, an IL-15Rα polypeptide, Linker 3 (SEQ ID NO: 69), and an IL-15 polypeptide; chain 2, comprising from the N-terminus to the C-terminus: an anti-PD-1 antibody light chain variable region, a human IgG1 light chain constant region CL, hinge region 2, human IgG1 heavy chain constant regions CH2 and CH3 containing a Hole mutation, Linker 1, an anti-TIGIT antibody light chain variable region, Linker 2, and an anti-TIGIT heavy chain variable region. As shown in b of FIG. 5B, structure II consists of two chains: chain 1, comprising from the N-terminus to the C-terminus: an anti-TIGIT antibody heavy chain variable region, a human IgG1 heavy chain constant region CH1, hinge region 1, human IgG1 heavy chain constant regions CH2 and CH3 containing a Knob mutation, Linker 1, an IL-15Rα polypeptide, Linker 3, and an IL-15 polypeptide; chain 2, comprising from the N-terminus to the C-terminus: an anti-TIGIT antibody light chain variable region, a human IgG1 light chain constant region CL, hinge region 2, human IgG1 heavy chain constant regions CH2 and CH3 containing a Hole mutation, Linker 1, an anti-PD-1 antibody light chain variable region, Linker 2, and an anti-PD-1 heavy chain variable region.

Structure III is shown in a and b of FIG. 5C. As shown in a of FIG. 5C, structure III consists of four chains: chains 1 and 4, each comprising from the N-terminus to the C-terminus: an anti-PD-1 antibody light chain variable region and a human IgG1 light chain constant region CL; chains 2 and 3, each comprising a TIGIT/PD-1 antibody structural portion and an IL-15/IL-15Rα complex, wherein the TIGIT/PD-1 antibody structural portion comprises from the N-terminus to the C-terminus: an anti-PD-1 heavy chain variable region, a human IgG1-WT heavy chain constant region (SEQ ID NO: 70), Linker 1, an anti-TIGIT antibody light chain variable region, Linker 2, and an anti-TIGIT antibody heavy chain variable region; the IL-15/IL-15Rα complex comprises from the N-terminus to the C-terminus: IL-15Rα, Linker 3, and IL-15, and is linked to the N-terminus of the anti-PD-1 heavy chain variable region via Linker 1. As shown in b of FIG. 5C, structure III consists of four chains: chains 1 and 4, each comprising from the N-terminus to the C-terminus: an anti-TIGIT antibody light chain variable region and a human IgG1 light chain constant region CL; chains 2 and 3, each comprising a TIGIT/PD-1 antibody structural portion, which comprises from the N-terminus to the C-terminus: an anti-TIGIT heavy chain variable region, a human IgG1-WT heavy chain constant region, Linker 1, an anti-PD-1 antibody light chain variable region, Linker 2, and an anti-PD-1 antibody heavy chain variable region; the IL-15/IL-15Rα complex is linked to the C-terminus of the anti-PD-1 heavy chain variable region.

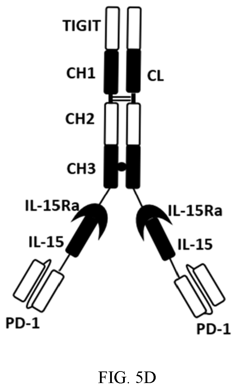

As shown in FIG. 5D, structure IV consists of two chains: chain 1, comprising from the N-terminus to the C-terminus: an anti-TIGIT antibody heavy chain variable region, a human IgG1 heavy chain constant region CH1, hinge region 1, human IgG1 heavy chain constant regions CH2 and CH3 containing a Knob mutation, Linker 1, IL-15Rα, Linker 3, IL-15, Linker 1, an anti-PD-1 antibody light chain variable region, Linker 2, and an anti-PD-1 antibody heavy chain variable region; chain 2, comprising from the N-terminus to the C-terminus: an anti-TIGIT antibody light chain variable region, a human IgG1 light chain constant region CL, hinge region 2, human IgG1 heavy chain constant regions CH2 and CH3 containing a Hole mutation, Linker 1, an IL-15Rα polypeptide, Linker 3, IL-15, Linker 1, an anti-PD-1 antibody light chain variable region, Linker 2, and an anti-PD-1 antibody heavy chain variable region.

Specifically, a total of seven chimeric anti-TIGIT/PD-1/IL-15 bispecific antibody-cytokine fusion proteins were obtained, among which 7Y2-102 and 7Y2-104 belong to structure I; 7Y2-072 (structure II shown in a of FIG. 5B) and 7Y2-079 (structure II shown in b of FIG. 5B) belong to structure II; 7Y2-094 (structure III shown in a of FIG. 5C) and 7Y2-078 (structure III shown in b of FIG. 5C) belong to structure III; and 7Y2-070 belongs to structure IV. Their specific structures are shown in Table 6.

A total of sixteen humanized anti-TIGIT/PD-1/IL-15 bispecific antibody-cytokine fusion proteins, all belonging to structure I, were obtained, namely 7Y2-120, 7Y2-123, 7Y2-133, 7Y2-136, 7Y2-137, 7Y2-126, 7Y2-127, 7Y2-132, 7Y2-134, 7Y2-135, 7Y2-128, 7Y2-129, 7Y2-124, 7Y2-125, 7Y2-130, and 7Y2-131. Their specific structures are shown in Table 7.

**Table 5: Amino acid sequences of IL-15, IL-15Rα, constant regions, hinge regions, and linkers**

| Description | SEQ ID NO: | Sequence |
|---|---|---|
| IL-15 | 63 | NWVNVISDLKKIEDLIQSMHIDATLYTESDVHPSCKVTAMKCFLLE LQVISLESGDASIHDTVENLIILANNSLSSNGNVTESGCKECEELEE KNIKEFLQSFVHIVQMFINTS |
| IL-15Rα_1 | 64 | PPPMSVEHADIWVKSYSLYSRERYICNSGFKRKAGTSSLTECVLNK ATNVAHWTTPSLKCIRDPALVHQRPAPPSTV |
| Hinge region 1 | 65 | EPKSCPPCP |
| Hinge region 2 | 66 | PPCP |
| Linker 1 | 67 | GGGGSGGGGS |
| Linker 2 | 68 | GGGGSGGGGSGGGGS |
| Linker 3 | 69 | SGGSGGGGSGGGSGGGGSLQ |
| IgG1-CH | 70 | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTYR VVSVLTVLHQDWLNGKEYKCKVSNKALPAPIEKTISKAKGQPREP QVYTLPPSREEMTKNQVSLTCLVKGFYPSDIAVEWESNGQPENNY KTTPPVLDSDGSFFLYSKLTVDKSRWQQGNVFSCSVMHEALHNHY TQKSLSLSPGK |
| IgG1-Knob | 71 | APELLGGPSVFLFPPKPKDTLMISRTPEVTCVVVDVSHEDPEVKFN WYVDGVEVHNAKTKPREEQYNSTYRVVSVLTVLHQDWLNGKEY KCKVSNKALPAPIEKTISKAKGQPREPQVYTLPPSREEMTKNQVSL WCLVKGFYPSDIAVEWESNGQPENNYKTTPPVLDSDGSFFLYSKLT VDKSRWQQGNVFSCSVMHEALHNHYTQKSLSLSPGK |
| IgG1-Hole | 72 | APELLGGPSVFLFPPKPKDTLMISRTPEVTCVVVDVSHEDPEVKFN WYVDGVEVHNAKTKPREEQYNSTYRVVSVLTVLHQDWLNGKEY KCKVSNKALPAPIEKTISKAKGQPREPQVYTLPPSREEMTKNQVSL SCAVKGFYPSDIAVEWESNGQPENNYKTTPPVLDSDGSFFLVSKLT VDKSRWQQGNVFSCSVMHEALHNHYTQKSLSLSPGK |
| IgG1-CL | 73 | |
| IgG1-CH1 | 74 | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKV |
| IL-15Rα_2 | 75 | ITCPPPMSVEHADIWVKSYSLYSRERYICNSGFKRKAGTSSLTECVL NKATNVAHWTTPSLKCIRDPALVHQRPAPPSTV |
| Signal peptide | 76 | METDTLLLWVLLLWVPGSTG |
| huTIGIT target gene | 77 | atgcgctggtgtctcctcctgatctgggcccaggggctgaggcaggctcccctcgcctcaggaatgatga caggcacaatagaaacaacggggaacatttctgcagagaaaggtggctctatcatcttacaatgtcacctc tcctccaccacggcacaagtgacccaggtcaactgggagcagcaggaccagcttctggccatttgtaatg ctgacttggggtggcacatctccccatccttcaaggatcgagtggccccaggtcccggcctgggcctcac cctccagtcgctgaccgtgaacgatacaggggagtacttctgcatctatcacacctaccctgatgggacgt acactgggagaatcttcctggaggtcctagaaagctcagtggctgagcacggtgccaggttccagattcc attgcttggagccatggccgcgacgctggtggtcatctgcacagcagtcatcgtggtggtcgcgttgacta gaaagaagaaagccctcagaatccattctgtggaaggtgacctcaggagaaaatcagctggacaggag gaatggagccccagtgctccctcacccccaggaagctgtgtccaggcagaagctgcacctgctgggctc tgtggagagcagcggggagaggactgtgccgagctgcatgactacttcaatgtcctgagttacagaagc ctgggtaactgcagcttcttcacagagactggttag |
| cynoTIGIT target gene | 78 | atggcctttcttgtggctccccctatgcagtttgtgtatttgttgaaaactttatgtgtttttaacatggtttttgcca aacctggtttttctgagaccgtcttttctcataggctcagttttaccgtcctatctgcagtcggctactttcggtg gcagaagaggccacatctgcttcctgtaagccctctgggcagaagcatgcggtggtgtctcttcctgatct gggcccaggggctgaggcaggctcccctcgcctcaggaatgatgacaggcacaatagaaacaacggg gaacatttctgcaaagaaaggtggctctgttatcttacaatgtcacctctcctccaccatggcacaagtgacc caggtcaactgggagcagcatgaccattcgcttctggccattcgtaatgctgagttggggtggcacatcta cccagccttcaaggatcgagtggccccgggtcctggcctgggcctcaccctccagtcgctgaccatgaat gatacaggggagtacttctgcacctatcacacctaccctgatgggacttacagagggagaatcttcctgga ggtcctagaaagctcagtggctgagcacagtgccaggttccagattccattgcttggagccatggccatg atgctggtggtcatctgcatagcagtcatcgtggtggtcgtgttggctagaaagaagaaatccctcagaatc cattctgtggaaagtggcctccagagaaaatcaactggacaggaagaacagattcccagtgctccctcac ccccaggaagctgtgtccaggcagaagctgcacctgctgggctctgtggagagcagcagggagatgac tgtgccgagctgcatgactacttcaatgtcctgagttacagaagcctggggagctgcagcttcttcacaga gactgggtag |
| Human IGKC nucleotide | 79 | CGCACCGTAGCTGCCCCGTCCGTGTTTATATTCCCACCAAGCGAT GAGCAACTCAAGTCCGGTACTGCTTCAGTCGTTTGTCTTCTTAAT AATTTCTATCCTAGAGAAGCTAAAGTACAATGGAAAGTAGATAAC GCCCTTCAATCCGGGAATTCACAGGAGAGCGTTACGGAGCAGGA TAGTAAGGACAGCACCTACAGCCTGAGTAGCACGCTCACTCTGT CAAAGGCAGATTATGAGAAACACAAAGTCTATGCGTGTGAGGTG ACTCATCAGGGACTCAGCAGCCCAGTAACAAAGAGTTTTAATAG GGGTGAGTGC |

**Table 6: Chimeric TIGIT/PD-1/IL-15 bispecific antibody-cytokine fusion proteins**

| Fusion protein name | | | 7Y2-102 | 7Y2-072 | 7Y2-070 | 7Y2-094 | 7Y2-104 | 7Y2-079 | 7Y2-078 |
|---|---|---|---|---|---|---|---|---|---|
| Structure type | | | Structure I | Structure II | Structure IV | Structure III | Structure I | Structure II | Structure III |
| Chimeric bispecific antibody-cytokine fusion protein sequence combination | TIGIT antibody VH | | 2IT1-10A3-2H6-VH (SEQ ID NO: 9) | | | | 3A03A03-VH (SEQ ID NO: 1) | | |
| | TIGIT antibody VL | | 2IT1-10A3-2H6-VL (SEQ ID NO: 10) | | | | 3A03A03-VL (SEQ ID NO: 2) | | |
| | PD-1 antibody VH | | 1KP-3A6-3C6-VH (SEQ ID NO: 25) | | | | | | |
| | PD-1 antibody VL | | 1KP-3A6-3C6-VL (SEQ ID NO: 26) | | | | | | |
| | IL-15 | | SEQ ID NO: 63 | | | | | | |
| | IL-15Rα_1 | | SEQ ID NO: 64 | | | | | | |
| | IgG1-WT CH | | / | | | SEQ ID NO: 70 | / | | SEQ ID NO: 70 |
| | IgG1-mutant CH sequence combination | CH1 | SEQ ID NO: 74 | | | / | SEQ ID NO: 74 | | / |
| | | Hole-mutant CH2 and CH3 | SEQ ID NO: 72 | | | / | SEQ ID NO: 72 | | / |
| | | Knob-mutant CH2 and CH3 | SEQ ID NO: 71 | | | / | SEQ ID NO: 71 | | / |
| | IgG1-CL | | SEQ ID NO: 73 | | | | | | |
| | Hinge region 1 | | SEQ ID NO: 65 | | | / | SEQ ID NO: 65 | | / |
| | Hinge region 2 | | SEQ ID NO: 66 | | | / | SEQ ID NO: 66 | | / |
| | Linker1 | | SEQ ID NO: 67 | | | | | | |
| | Linker2 | | SEQ ID NO: 68 | | | | | | |
| | Linker3 | | / | SEQ ID NO: 69 | | | / | SEQ ID NO: 69 | |

Note: The fusion protein 7Y2-072 belongs to structure II shown in a of FIG. 5B; the fusion protein 7Y2-079 belongs to structure II shown in b of FIG. 5B; the fusion protein 7Y2-094 belongs to structure III shown in a of FIG. 5C; the fusion protein 7Y2-078 belongs to structure III shown in b of FIG. 5C.

**Table 7: Humanized TIGIT/PD-1/IL-15 bispecific antibody-cytokine fusion proteins**

| Fusion protein name | Humanized bispecific antibody-cytokine fusion protein sequence combination | | | |
|---|---|---|---|---|
| | TIGIT antibody VH | TIGIT antibody VL | PD-1 antibody VH | PD-1 antibody VL |
| 7Y2-120 | 7TI-208-VH (SEQ ID NO: 41) | 7TI-208-VL (SEQ ID NO: 42) | 7PD-111-VH (SEQ ID NO: 54) | 7PD-111-VL (SEQ ID NO: 55) |
| 7Y2-123 | | | 7PD-118-VH (SEQ ID NO: 56) | 7PD-118-VL (SEQ ID NO: 57) |
| 7Y2-137 | | | 7PD-104-VH (SEQ ID NO: 62) | 7PD-104-VL (SEQ ID NO: 61) |
| 7Y2-124 | 7TI-174-VH (SEQ ID NO: 45) | 7TI-174-VL (SEQ ID NO: 47) | 7PD-079-VH (SEQ ID NO: 51) | 7PD-079-VL (SEQ ID NO: 52) |
| 7Y2-126 | | | 7PD-115-VH (SEQ ID NO: 58) | 7PD-115-VL (SEQ ID NO: 59) |
| 7Y2-127 | | | 7PD-118-VH (SEQ ID NO: 56) | 7PD-118-VL (SEQ ID NO: 57) |
| 7Y2-132 | | | 7PD-120-VH (SEQ ID NO: 56) | 7PD-120-VL (SEQ ID NO: 59) |
| 7Y2-135 | | | 7PD-104-VH (SEQ ID NO: 62) | 7PD-104-VL (SEQ ID NO: 61) |
| 7Y2-128 | 7TI-224-VH (SEQ ID NO: 48) | 7TI-224-VL (SEQ ID NO: 50) | 7PD-098-VH (SEQ ID NO: 60) | 7PD-098-VL (SEQ ID NO: 57) |
| 7Y2-130 | | | 7PD-079-VH (SEQ ID NO: 51) | 7PD-079-VL (SEQ ID NO: 52) |
| 7Y2-129 | 7TI-225-VH (SEQ ID NO: 48) | 7TI-225-VL (SEQ ID NO: 49) | 7PD-099-VH (SEQ ID NO: 60) | 7PD-099-VL (SEQ ID NO: 61) |
| 7Y2-131 | | | 7PD-082-VH (SEQ ID NO: 53) | 7PD-082-VL (SEQ ID NO: 52) |
| 7Y2-133 | 7TI-202-VH (SEQ ID NO: 43) | 7TI-202-VL (SEQ ID NO: 44) | 7PD-098-VH (SEQ ID NO: 60) | 7PD-098-VL (SEQ ID NO: 57) |
| 7Y2-136 | | | 7PD-099-VH (SEQ ID NO: 60) | 7PD-099-VL (SEQ ID NO: 61) |
| 7Y2-125 | 7TI-172-VH (SEQ ID NO: 45) | 7TI-172-VL (SEQ ID NO: 46) | 7PD-082-VH (SEQ ID NO: 53) | 7PD-082-VL (SEQ ID NO: 52) |
| 7Y2-134 | | | 7PD-098-VH (SEQ ID NO: 60) | 7PD-098-VL (SEQ ID NO: 57) |

| | | | | |
|---|---|---|---|---|
| Note: All sixteen humanized TIGIT/PD-1/IL-15 bispecific antibody-cytokine fusion proteins in Table 7 belong to structure I, in which the sequence of IL-15 is shown in SEQ ID NO: 63, the sequence of IL-15Rα_2 is shown in SEQ ID NO: 75, the sequence of human IgG1 heavy chain constant region CH1 is shown in SEQ ID NO: 74, the sequence of human IgG1 heavy chain constant regions CH2 and CH3 containing the Hole mutation is shown in SEQ ID NO: 72, the sequence of human IgG1 heavy chain constant regions CH2 and CH3 containing the Knob mutation is shown in SEQ ID NO: 71, the sequence of human IgG1 light chain constant region CL is shown in SEQ ID NO: 73, the sequence of hinge region 1 is shown in SEQ ID NO: 65, the sequence of hinge region 2 is shown in SEQ ID NO: 66, the sequence of Linker1 is shown in SEQ ID NO: 67, and the sequence of Linker2 is shown in SEQ ID NO: 68. | | | | |

### Example 3: Expression and purification of TIGIT/PD-1/IL-15 bispecific antibody-cytokine fusion proteins

### 3.1 Construction of vectors

(1) Based on the pTT5 vector (Hunan YouBio, VT2202), which contains a signal peptide (SEQ ID NO: 76) directing protein synthesis and secretion, restriction enzyme sites were introduced by primer-mediated mutagenesis. The synthesized human IGKC sequence (SEQ ID NO: 79) was inserted into the pTT5 vector using conventional biological enzymatic digestion and ligation methods to obtain the pTT5-IgG1-CL vector. Similarly, the synthesized human IgG1 heavy chain constant region sequence was inserted into the pTT5 vector to obtain the pTT5-IgG1-CH vector.
(2) A mutation was introduced into the pTT5-IgG1-CH vector using a forward primer, while a homologous fragment was incorporated using a reverse primer. PCR and homologous recombination were performed to obtain the pTT5-IgG1-Knob vector. Similarly, the pTT5-IgG1-Hole vector was constructed using the same method.

### 3.2 Expression of fusion proteins

The gene sequences encoding the chimeric TIGIT/PD-1/IL-15 bispecific antibody-cytokine fusion proteins listed in Table 6 and the humanized TIGIT/PD-1/IL-15 bispecific antibody-cytokine fusion proteins listed in Table 7 were respectively cloned into the pTT5-IgG1-CL, pTT5-IgG1-CH, pTT5-IgG1-Knob, or pTT5-IgG1-Hole vectors of human IgG1 containing a signal peptide directing protein synthesis and secretion. The expression vectors were transiently transfected into ExpiCHO-S cells (Gibco, A29127) for expression. Polyplus transfection reagent (Polyplus, REF #116-010/10 mL) was used for transfection. One day prior to transfection (D0-1), cells were diluted with culture medium to a density of 2.0 × 10⁶ cells/mL. On the day of transfection (D0), cells were counted (cell viability should be 95% or more) and adjusted to a density of 4.0 × 10⁶ cells/mL. Transfection was performed using 1 µg of plasmid, 1 µL of Polyplus FectoPRO^{®} DNA Transfection Reagent, and 1 µL of FectoPRO^{®} Booster per 4.0 × 10⁶ cells. Specifically, plasmid DNA encoding the bispecific antibody-cytokine fusion protein was diluted in 1/10 of the expression volume of culture medium in one centrifuge tube, while the required amount of Polyplus DNA Transfection Reagent was added to another centrifuge tube. The diluted plasmid was slowly added dropwise to the centrifuge tube containing the DNA Transfection Reagent, gently mixed by inversion, and incubated at room temperature for 10 minutes. After incubation, the transfection reagent-DNA complex was slowly added to the ExpiCHO-S cells with shaking, followed by addition of FectoPRO^{®} Booster within 0 to 4 hours. After transfection, the cells were cultured in a shaking incubator at 37°C and 120 rpm with 8% CO₂. On the first day after transfection (D1), pre-warmed CHOgro complete medium was supplemented at 1/5 of the expression volume, and the temperature was reduced to 32°C for further culture. On D2, D4, D6, and D8 post-transfection, Advanced CHO Feed1 was supplemented at 8%, 5%, 5%, and 5%, respectively. Cells were harvested on D10.

### 3.3 Quantification of expression levels of chimeric TIGIT/PD-1/IL-15 bispecific antibody-cytokine fusion proteins

The cells harvested in Example 3.2 were centrifuged to collect the supernatant. The bispecific antibody-cytokine fusion proteins were purified using a protein purification system (AKTA pure 150) by Protein-A affinity chromatography (AC). The purification procedure was as follows:
(1) The chromatography system was sterilized by soaking in 0.5 M NaOH for at least 30 minutes, and the chromatography column was sterilized by soaking in 0.1 M NaOH for 30 minutes.
(2) The system pH and conductivity baselines were equilibrated using 1× PBS buffer at pH 7.4.
(3) Samples were loaded at a flow rate corresponding to a retention time of 3 to 5 minutes.
(4) After sample loading, non-specifically bound proteins were removed by washing with a wash buffer containing 0.5 M arginine hydrochloride, 1 M NaCl, and 0.5% Triton X-100 at pH 5.0 ± 0.1, and binding levels of endotoxin to antibody were reduced.
(5) The target protein was eluted with 40 mM citric acid-sodium citrate buffer containing 0.15 M NaCl at pH 3.0 ± 0.1, and the pH of the eluted protein was adjusted to 6.0 ± 0.1 using a neutralization buffer of 1 M Tris at pH 8.6.
(6) A 2 to 5 µL aliquot of the protein solution was taken, and the protein concentration was measured using a NanoDrop spectrophotometer. Subsequently, the protein purity was measured by size-exclusion chromatography (SEC-HPLC): A 10 µg sample at 1 mg/mL was prepared and analyzed using a liquid chromatography system (Shimadzu, Japan, LC-2030C-3D Plus) at a flow rate of 0.5 mL/min with a run time of 35 minutes. After completion of the measurement, the percentage of the main peak area was calculated using the software (LabSolutions) provided with the instrument, yielding the purity of the bispecific antibody-cytokine fusion protein. The expression level of the fusion protein was calculated as: expression level = concentration × volume × purity ÷ expression volume.

The expression level data obtained for the chimeric TIGIT/PD-1/IL-15 bispecific antibody-cytokine fusion proteins were statistically analyzed, and the results are shown in Table 8.

**Table 8: Expression levels of chimeric TIGIT/PD-1/IL-15 bispecific antibody-cytokine fusion proteins**

| Fusion protein name | PD-1 antibody name | TIGIT antibody name | Structure type | Expression level (mg/L) |
|---|---|---|---|---|
| 7Y2-102 | | 2IT1-10A3-2H6 | Structure I | 42.6 |
| 7Y2-072 | | | Structure II | 17.98 |
| 7Y2-094 | | | Structure III | 5 |
| 7Y2-070 | 1KP-3A6-3C6 | | Structure IV | 13.43 |
| 7Y2-104 | | 3A03A03 | Structure I | 37.9 |
| 7Y2-079 | | | Structure II | 4.17 |
| 7Y2-078 | | | Structure III | 12.67 |

It was evident from the results in the table that: (1) among the chimeric bispecific antibody-cytokine fusion proteins prepared using the combination of 1KP-3A6-3C6 and 2IT1-10A3-2H6, the expression level of 7Y2-102 (structure I) was significantly higher than those of 7Y2-072 (structure II), 7Y2-094 (structure III), and 7Y2-070 (structure IV); (2) among the chimeric bispecific antibody-cytokine fusion proteins prepared using the combination of 1KP-3A6-3C6 and 3A03A03, the expression level of 7Y2-104 (structure I) was significantly higher than those of 7Y2-079 (structure II) and 7Y2-078 (structure III). Therefore, the TIGIT/PD-1/IL-15 bispecific antibody-cytokine fusion proteins prepared using structure I exhibited superior expression levels compared to the other three structures.

### 3.4 Purification by gel filtration chromatography

The bispecific antibody-cytokine fusion proteins obtained after affinity purification were further subjected to secondary purification by gel filtration chromatography (GFC) to obtain highly purified recombinant fusion proteins. The chromatography system and column were soaked in 0.5 M NaOH for at least 2 hours. The chromatography system was equilibrated to a stable pH using 1× PBS at pH 6.2 and conductivity baselines were stabilized. Samples were loaded using a sample loop, and intact bispecific antibody-cytokine fusion protein monomers were collected based on the principle of size exclusion.

### Example 4: Physicochemical characterization of chimeric TIGIT/PD-1/IL-15 bispecific antibody-cytokine fusion proteins

The chimeric TIGIT/PD-1/IL-15 bispecific antibody-cytokine fusion proteins purified in Example 3.4 were analyzed for their melting point (Tm), temperature of aggregation (Tagg), aggregation resistance, and thermostability.

Tm determination: Using differential scanning fluorimetry (DSF), 17.5 µL of the chimeric bispecific antibody-cytokine fusion protein at 1 mg/mL was prepared, followed by addition of 2.5 µL of 1000× Protein Thermal Shift Dye (Thermo, 4461146), and mixed in an EP tube. The resulting sample mixture was loaded into a Q-PCR system (QuantStudio) for reaction. The Q-PCR parameters were set as follows: Target (ROX); program (25°C for 3 minutes, followed by a temperature increase to 99°C at a rate of 0.05°C/s; 99°C for 2 minutes). The resulting data were imported into GraphPad Prism software to calculate the Tm values.

Tagg determination: A 9 µL aliquot of each chimeric bispecific antibody-cytokine fusion protein to be tested (1 mg/mL) was prepared and loaded into a testing instrument (Uncle-0734). After completion of the measurement, data analysis was performed using the software provided with the instrument.

High-temperature stability at 40°C: The chimeric bispecific antibody-cytokine fusion proteins to be tested were incubated in a constant-temperature incubator at 40°C for 1, 3, 7, and 14 days. The protein purity after high-temperature treatments for 1, 3, 7, and 14 days was determined by SEC-HPLC according to the method described in Example 3.3.

Freeze-thaw stability: The chimeric bispecific antibody-cytokine fusion proteins to be tested were frozen in a -20°C freezer and subsequently thawed at room temperature. The freeze-thaw cycle was repeated 1, 3, and 5 times, respectively. The protein purity after 1, 3, and 5 freeze-thaw cycles was determined by SEC-HPLC according to the method described in Example 3.3.

The assay results for the Tm values, Tagg values, high-temperature stability at 40°C, and freeze-thaw stability of the chimeric bispecific antibody-cytokine fusion proteins are shown in Table 9.

**Table 9: Physicochemical properties of chimeric bispecific antibody-cytokine fusion proteins**

| Fusion protein name | | 7Y2-102 | 7Y2-072 |
|---|---|---|---|
| PD-1 antibody name | | 1KP-3A6-3C6 | |
| TIGIT antibody name | | 2IT1-10A3-2H6 | |
| Structure type | | Structure I | Structure II |
| Tm (°C) | Tm1 | 56.26 | 50.05 |
| | Tm2 | 69.67 | 68.06 |
| | Tm3 | / | 78.29 |
| Tagg (°C) | | 61.9 | 48.3 |
| Freeze-thaw stability (SEC, %) | 0 cycles | 91.22 | 85.67 |
| | 1 cycle | 90.63 | 85.16 |
| | 3 cycles | 89.94 | N/A |
| | 5 cycles | N/A | 83.73 |
| High-temperature stability at 40°C (SEC, %) | Day 0 | 91.22 | 83.29 |
| | Day 3 | 84.38 | 13.33 |
| | Day 7 | N/A | 12.94 |
| | Day 14 | 75.02 | N/A |

| | | | |
|---|---|---|---|
| Note: "N/A" indicates none or not applicable. "/" indicates that the antibody structure remained stable without unfolding, and therefore no numerical value was obtained. | | | |

As shown by the results in Table 9, among the chimeric TIGIT/PD-1/IL-15 bispecific antibody-cytokine fusion proteins prepared using the combination of 1KP-3A6-3C6 and 2IT1-10A3-2H6, 7Y2-102 (structure I) exhibited superior performance compared to 7Y2-072 (structure II) in terms of Tm value, Tagg value, freeze-thaw stability, and high-temperature stability, indicating that the TIGIT/PD-1/IL-15 bispecific antibody-cytokine fusion proteins prepared using structure I possess more favorable physicochemical properties.

### Example 5: Determination of the affinity between chimeric TIGIT/PD-1/IL-15 bispecific antibody-cytokine fusion proteins and human TIGIT, cynomolgus monkey TIGIT, human PD-1, or cynomolgus monkey PD-1 antigens

The association rate constant (ka), dissociation rate constant (kd), and equilibrium dissociation constant (K_{D}) between the chimeric bispecific antibody-cytokine fusion proteins and human TIGIT (Hu-TIGIT, purchased from ACROBiosystems, TIT-H52H5), human PD-1 (Hu-PD-1, purchased from ACROBiosystems, PD1-H5221), cynomolgus monkey TIGIT (Cyno-TIGIT, purchased from ACROBiosystems, CAT: TIT-C5223), and cynomolgus monkey PD-1 (Cyno-PD-1, purchased from ACROBiosystems, PD1-C5223) were determined using a biomolecular interaction analyzer (Biacore, Cytiva). The specific procedure was as follows: An anti-human IgG secondary antibody was immobilized on an activated CM5 biosensor chip by amine coupling, followed by addition of the chimeric bispecific antibody-cytokine fusion protein to be tested at a final concentration of 5 µg/mL. Subsequently, serially diluted Hu-TIGIT, Cyno-TIGIT, Hu-PD-1, and Cyno-PD-1 antigens were sequentially added from low to high concentrations (starting from 90 nM, with 3-fold serial dilutions). Kinetic measurements were performed using the following program: Start up (60 s) -- Capture (60 s) -- Association (200 s) -- Dissociation (600 s) -- Regeneration (40 s). After the program was completed, the result file was read by Insight Evaluation Software, and kinetic fitting analysis was performed to obtain the binding affinities between the chimeric bispecific antibody-cytokine fusion proteins and the corresponding antigens. The results are shown in Table 10.

**Table 10: Affinity results of chimeric bispecific antibody-cytokine fusion proteins**

| Antibody name | | 7Y2-102 | 7Y2-072 | 7Y2-070 | 7Y2-104 | 7Y2-079 | 7Y2-078 |
|---|---|---|---|---|---|---|---|
| PD-1 antibody name | | 1KP-3A6-3C6 | | | | | |
| TIGIT antibody name | | 2IT1-10A3-2H6 | | | 3A03A03 | | |
| Structure type | | Structure I | Structure II | Structure IV | Structure I | Structure II | Structure III |
| Affinity to Hu-TIGIT | ka (1/Ms) | 9.27E+05 | 2.51E+05 | 1.56E+06 | 1.02E+06 | 7.29E+05 | 3.01E+06 |
| | kd (1/s) | 2.71E-04 | 1.27E-04 | 2.75E-04 | 1.17E-03 | 2.13E-04 | 1.40E-03 |
| | K_{D} (M) | 2.92E-10 | 5.09E-10 | 1.77E-10 | 1.14E-09 | 2.93E-10 | 4.66E-10 |
| Affinity to Cyno-TIGIT | ka (1/Ms) | 1.55E+06 | 4.79E+05 | 1.05E+06 | 1.00E+05 | N/A | N/A |
| | kd (1/s) | 1.08E-03 | 1.36E-04 | 3.10E-04 | 1.15E-03 | N/A | N/A |
| | K_{D} (M) | 6.97E-10 | 2.85E-10 | 2.96E-10 | 1.14E-08 | 2.90E-08 | 3.98E-08 |
| Affinity to Hu-PD-1 | ka (1/Ms) | 2.88E+05 | 4.15E+05 | 2.04E+05 | 2.88E+05 | 1.31E+05 | 1.83E+05 |
| | kd (1/s) | 7.02E-04 | 3.74E-04 | 5.40E-04 | 5.09E-04 | 3.01E-04 | 6.33E-04 |
| | K_{D} (M) | 2.44E-09 | 9.01E-10 | 2.65E-09 | 1.77E-09 | 2.29E-09 | 3.46E-09 |
| Affinity to Cyno-PD-1 | ka (1/Ms) | 2.17E+05 | 1.80E+05 | 1.02E+05 | 2.63E+05 | 1.18E+05 | 8.75E+04 |
| | kd (1/s) | 1.07E-03 | 8.36E-04 | 9.49E-04 | 1.34E-03 | 7.06E-04 | 8.05E-04 |
| | K_{D} (M) | 4.94E-09 | 4.64E-09 | 9.27E-09 | 5.09E-09 | 5.99E-09 | 9.19E-09 |

| | | | | | | | |
|---|---|---|---|---|---|---|---|
| Note: N/A indicates none or not applicable. | | | | | | | |

As shown in Table 10, the chimeric TIGIT/PD-1/IL-15 bispecific antibody-cytokine fusion proteins listed in the table exhibited good binding activity to human TIGIT, human PD-1, cynomolgus monkey TIGIT, and cynomolgus monkey PD-1 proteins.

### Example 6: Binding assay of chimeric TIGIT/PD-1/IL-15 bispecific antibody-cytokine fusion proteins to cells expressing human TIGIT, cynomolgus monkey TIGIT, human PD-1, or cynomolgus monkey PD-1 antigens

The binding ability of the chimeric bispecific antibody-cytokine fusion proteins to CHO-K1-hPD-1 and CHO-K1-cynoPD-1 cells was evaluated by flow cytometry (FACS). The specific experimental procedure was as follows: CHO-K1-hPD-1 cells overexpressing human PD-1 and CHO-K1-cynoPD-1 cells overexpressing cynomolgus monkey PD-1 in logarithmic growth phase were collected separately and washed once with Ham's F-12 K medium containing 2% FBS (ExCell Bio) (referred to as H2 medium, BasalMedia, L450KJ). The cells were then resuspended in H2 medium and adjusted to a density of 1 × 10⁶ cells/mL, followed by addition of Fc blocker (BD, Cat. No. 564220) at a volume ratio of 1:100, and incubated at room temperature for 30 minutes. CHO-K1-hPD-1 or CHO-K1-cynoPD-1 cell suspensions were added to a 96-well U-bottom plate at 100 µL per well (containing 1 × 10⁵ cells per well), centrifuged at 300 g for 3 minutes, and the supernatant was discarded. The chimeric TIGIT/PD-1/IL-15 bispecific antibody-cytokine fusion proteins were diluted in H2 medium to an initial working concentration of 240 µg/mL, followed by 3-fold serial dilutions. Wells containing H2 medium alone were used as blank controls. Subsequently, the chimeric bispecific antibody-cytokine fusion proteins at various concentrations were added to the plated CHO-K1-hPD-1 and CHO-K1-cynoPD-1 cells at 50 µL per well, gently mixed, and incubated at 37°C for 1 hour. After incubation, the plate was centrifuged at 300 g for 3 minutes, and the supernatant was discarded. The cells were collected and washed three times with FACS buffer (PBS containing 2% FBS and 0.002 M EDTA), then resuspended by adding a fluorescently conjugated goat anti-human IgG Fc secondary antibody (diluted 1:800 in FACS buffer, Biolegend, 410712) at 100 µL per well, and incubated at 4°C for 30 minutes. After incubation, the plate was centrifuged at 300 g for 3 minutes, and the supernatant was discarded. The cells were washed three times with FACS buffer, resuspended in 100 µL of FACS buffer, and analyzed by flow cytometry. The flow cytometry data were analyzed and calculated using FlowJo, and the mean fluorescent intensity (MFI) values of AF647 for cells in each sample well were exported. Nonlinear regression fitting of MFI values versus corresponding antibody concentrations was performed using the Log(agonist) vs. response - Variable slope (four parameters) model in GraphPad Prism software, with the x-axis representing Log[Ab concentration] and the y-axis representing MFI values, to generate binding curves of the chimeric bispecific antibody-cytokine fusion proteins to PD-1 antigens. EC₅₀ values of the binding curves were calculated to evaluate the binding ability of the fusion proteins to cells overexpressing PD-1 antigens.

Using the same procedure as described above for CHO-K1-hPD-1 cells, the binding ability of the chimeric bispecific antibody-cytokine fusion proteins to Jurkat-huTIGIT cells overexpressing human TIGIT and 293T-cynoTIGIT cells overexpressing cynomolgus monkey TIGIT was determined, wherein the Jurkat cells were cultured in RPMI 1640 medium (Shanghai BasalMedia, L210KJ), and the 293T cells were cultured in DMEM high-glucose medium (Shanghai BasalMedia, L110KJ). The binding affinity results of the chimeric bispecific antibody-cytokine fusion proteins to cells expressing the corresponding antigens are shown in Table 11.

**Table 11: Binding results of chimeric bispecific antibody-cytokine fusion proteins to cell surface targets**

| Fusion protein name | | 7Y2-102 | 7Y2-072 | 7Y2-070 | 7Y2-104 | 7Y2-079 | 7Y2-078 |
|---|---|---|---|---|---|---|---|
| PD-1 antibody name | | 1KP-3A6-3C6 | | | | | |
| TIGIT antibody name | | 2IT1-10A3-2H6 | | | 3A03A03 | | |
| Structure type | | I | II | IV | I | II | III |
| Binding to Jurkat-huTIGIT cells | EC₅₀ (nM) | 2.81 | 18.59 | 2.39 | 1.96 | 18.17 | 0.59 |
| Binding to 293T-cynoTIGIT cells | EC₅₀ (nM) | 1.42 | 5.82 | 1.45 | 6.08 | 100.69 | 241.09 |
| Binding to CHO-K1-hPD-1 cells | EC₅₀ (nM) | 5.16 | 2.77 | 7.04 | 5.89 | 23.49 | 2.06 |
| Binding to CHO-K1-cynoPD-1 cells | EC₅₀ (nM) | 2.14 | 1.99 | 2.10 | 2.31 | 14.92 | 2.31 |

As shown in Table 11, the chimeric TIGIT/PD-1/IL-15 bispecific antibody-cytokine fusion proteins listed in the table exhibited good binding ability to cells expressing human TIGIT, cynomolgus monkey TIGIT, human PD-1, or cynomolgus monkey PD-1 antigens.

### Example 7: Activation assay of chimeric TIGIT/PD-1/IL-15 bispecific antibody-cytokine fusion proteins on reporter cell pairs overexpressing human TIGIT/CD155 and overexpressing human PD-1/PD-L1

### 7.1 Activation assay of chimeric TIGIT/PD-1/IL-15 bispecific antibody-cytokine fusion proteins on reporter cell pairs overexpressing human PD-1/PD-L1

CHO-K1/hPDL1/TCR cells overexpressing human PD-L1 (M00613, Nanjing GenScript Biotech Co., Ltd.) in logarithmic growth phase were used as target cells. The cells were digested with trypsin, resuspended in fresh Ham's F-12 K medium containing 10% FBS (referred to as H10 medium), and adjusted to a density of 5 × 10⁵ cells/mL. The resuspended cells were seeded into a white-walled, clear-bottom 96-well cell culture plate at 5 × 10⁴ cells per well in a volume of 100 µL per well, and cultured overnight in an incubator at 37°C with 5% CO₂. On the following day, the plate was centrifuged and the supernatant was discarded. The chimeric TIGIT/PD-1/IL-15 bispecific antibody-cytokine fusion proteins were diluted in RPMI 1640 medium containing 2% FBS (referred to as R2 medium) to a 2× initial working concentration of 240 µg/mL, followed by 3-fold serial dilutions across 11 concentrations. Wells containing R2 medium alone were used as blank controls. Subsequently, the fusion proteins at various concentrations were added to the plated CHO-K1/hPDL1/TCR cells at 50 µL per well. Jurkat/NFAT-luc/PD1 cells (M00612, Nanjing GenScript Biotech Co., Ltd.), which stably express human PD-1 and a luciferase reporter gene under the control of an NFAT transcriptional response element, were used as effector cells. The cells were resuspended in R2 medium and adjusted to a density of 3 × 10⁶ cells/mL. The resuspended cells were added to a 96-well plate at 50 µL per well, followed by incubation in an incubator at 37°C with 5% CO₂ for 6 hours. The 96-well plate was removed from the incubator, and 100 µL of Bright-Lite^{™} Luciferase Assay Reagent (Vazyme) was added to each well. After reacting at room temperature for 3 to 5 minutes, luminescence was measured using a microplate reader. Nonlinear regression fitting was performed using the Log(agonist) vs. response - Variable slope (four parameters) model in GraphPad Prism software, with the fluorescence values corresponding to each concentration as the y-axis and the logarithm (base 10) of the sample concentration as the x-axis, to generate activation curves of the antibodies on the reporter cells. EC₅₀ values of the activation curves were calculated to evaluate the activation capability of the antibodies.

### 7.2 Activation assay of chimeric TIGIT/PD-1/IL-15 bispecific antibody-cytokine fusion proteins on reporter cell pairs expressing human TIGIT/CD155

Following the same method as described above in Example 7.1, the activation of the chimeric bispecific antibody-cytokine fusion proteins was evaluated using CHO-K1/CD155/TCR cells overexpressing human CD155 (CBP74073, Nanjing Cobioer Biosciences Co., Ltd.), and Jurkat-TIGIT-NFAT-Luc cells (CBP74020, Nanjing Cobioer Biosciences Co., Ltd.), which stably express human TIGIT and a luciferase reporter gene under the control of an NFAT transcriptional response element; wherein the Jurkat-TIGIT-NFAT-Luc cells were adjusted to a density of 1 × 10⁶ cells/mL.

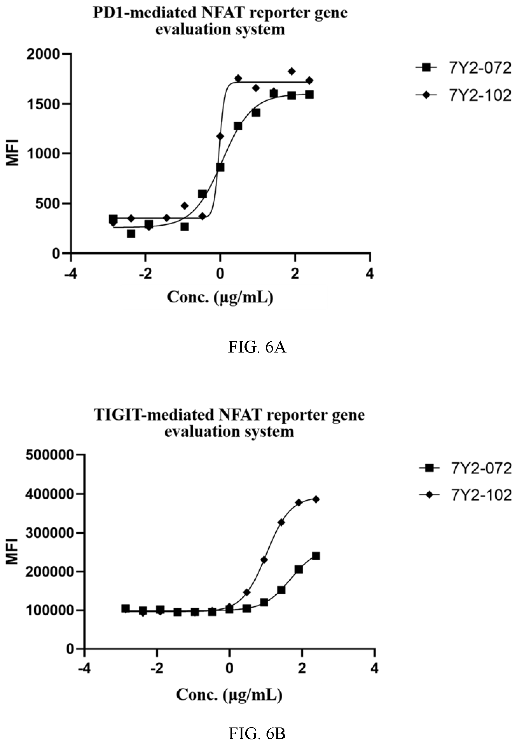

The results are shown in Table 12, FIG. 6A, and FIG. 6B. The chimeric TIGIT/PD-1/IL-15 bispecific antibody-cytokine fusion proteins listed in the table were capable of recognizing and activating Jurkat-TIGIT-NFAT-Luc or Jurkat/NFAT-luc/PD1 reporter cells. 7Y2-102 (structure I) exhibited a stronger activation effect on both cell types compared to 7Y2-072 (structure II), indicating that the TIGIT/PD-1/IL-15 bispecific antibody-cytokine fusion proteins prepared using structure I possess more favorable activation capability for reporter cells.

**Table 12: Activation results of chimeric bispecific antibody-cytokine fusion proteins on reporter cells**

| Fusion protein name | | 7Y2-102 | 7Y2-072 |
|---|---|---|---|
| PD-1 antibody name | | 1KP-3A6-3C6 | |
| TIGIT antibody name | | 2IT1-10A3-2H6 | |
| Structure type | | Structure I | Structure II |
| Jurkat-TIGIT-NFAT-Luc | EC₅₀ (nM) | 59.53 | 324.69 |
| | MAX RLU | 393200 | 261861 |
| Jurkat/NFAT-luc/PD1 | EC₅₀ (nM) | 5.4 | 7.78 |
| | MAX RLU | 1721 | 1603 |

### Example 8: Functional assay of chimeric TIGIT/PD-1/IL-15 bispecific antibody-cytokine fusion proteins in promoting immune cell proliferation

CTLL-2 cells (China National Institutes for Food and Drug Control, 1102MOU-NIFDC00051) and Mo7e cells (sourced from the National Infrastructure of Cell Line Resource) in logarithmic growth phase were harvested separately. The cells were washed twice with PBS, resuspended in assay medium (RPMI 1640 (ATCC modified) (Shanghai BasalMedia, L240KJ) + 10% FBS), and adjusted to a density of 8 × 10⁵ cells/mL. Mo7e cells and CTLL-2 cells were seeded separately into a 96-well flat-bottom plate at 50 µL per well, followed by incubation in an incubator at 37°C with 5% CO₂ for 4 hours, resulting in cytokine-starved cells.

The chimeric bispecific antibody-cytokine fusion proteins were diluted in assay medium to a final concentration of 1080 nM, followed by one 2-fold serial dilution and then nine 3-fold serial dilutions. Each dilution of the chimeric bispecific antibody-cytokine fusion proteins was added to the wells containing Mo7e cells or CTLL-2 cells at 50 µL per well. The CTLL-2 cells or Mo7e cells treated with the chimeric bispecific antibody-cytokine fusion proteins were further cultured for 72 hours. Finally, the number of viable cells was determined using CCK-8 reagent (Beyotime, C0040). Proliferation curves were generated, and the EC₅₀ values of the chimeric bispecific antibody-cytokine fusion proteins in promoting the proliferation of Mo7e cells and CTLL-2 cells were calculated.

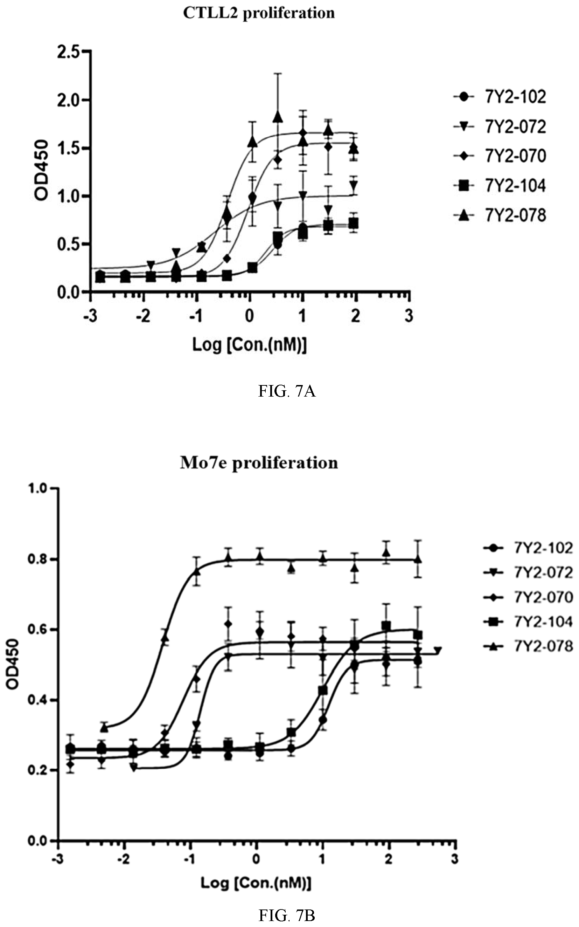

The results are shown in Table 13, FIG. 7A, and FIG. 7B. All chimeric bispecific antibody-cytokine fusion proteins were capable of stimulating the proliferation of CTLL-2 cells and Mo7e cells. Among the chimeric TIGIT/PD-1/IL-15 bispecific antibody-cytokine fusion proteins prepared using the combination of 1KP-3A6-3C6 and 3A03A03, the fusion protein 7Y2-078 (structure III) exhibited higher proliferative activity than 7Y2-104 (structure I). Among the chimeric TIGIT/PD-1/IL-15 bispecific antibody-cytokine fusion proteins prepared using the combination of 1KP-3A6-3C6 and 2IT1-10A3-2H6, 7Y2-072 (structure II) and 7Y2-070 (structure IV) exhibited higher proliferative activity than 7Y2-102 (structure I). These results indicate that structure I fusion proteins can activate immune cells in a more moderate manner, and exhibit better safety profiles compared to structure II, structure III, and structure IV.

**Table 13: Cytokine activity of IL-15 in chimeric bispecific antibody-cytokine fusion proteins**

| Fusion protein name | | 7Y2-102 | 7Y2-072 | 7Y2-070 | 7Y2-104 | 7Y2-078 |
|---|---|---|---|---|---|---|
| PD-1 antibody name | | 1KP-3A6-3C6 | | | | |
| TIGIT antibody name | | 2IT1-10A3-2H6 | | | 3A03A03 | |
| Structure type | | Structure I | Structure II | Structure IV | Structure I | Structure III |
| CTLL-2 cells | EC₅₀ (nM) | 2.685 | 0.217 | 0.917 | 2.006 | 0.377 |
| | MAX OD | 0.709 | 1.005 | 1.554 | 0.683 | 1.663 |
| Mo7e cells | EC₅₀ (nM) | 12.28 | 0.141 | 0.078 | 10.040 | 0.038 |
| | MAX OD | 0.514 | 0.530 | 0.564 | 0.601 | 0.799 |

### Example 9: Functional assay of chimeric TIGIT/PD-1/IL-15 bispecific antibody-cytokine fusion proteins in activating human PBMCs

Peripheral blood mononuclear cells (PBMCs) obtained from a healthy donor (Shanghai OriBiotech Co., Ltd., Donor ID: Z0395) were thawed from liquid nitrogen and resuspended in complete medium (RPMI 1640 + 10% FBS) to a density of 1 × 10⁶ cells/mL. SEA was added to the cell suspension at 10 ng/mL, and the resulting mixture was added to a 96-well flat-bottom plate at 100 µL per well. The chimeric bispecific antibody-cytokine fusion proteins were diluted in complete medium to 30 µg/mL. For the combination therapy control group (Combo (P + T)), sample P (Keytruda) was diluted to 20 µg/mL and sample T (Tiragolumab) was diluted to 10 µg/mL. Both samples were then subjected to 5-fold serial dilutions and added to the corresponding PBMC wells at 100 µL/well. The cells were cultured in an incubator at 37°C with 5% CO₂ for 3 to 4 days, and cell culture supernatants were collected on days 3 and 4, respectively.

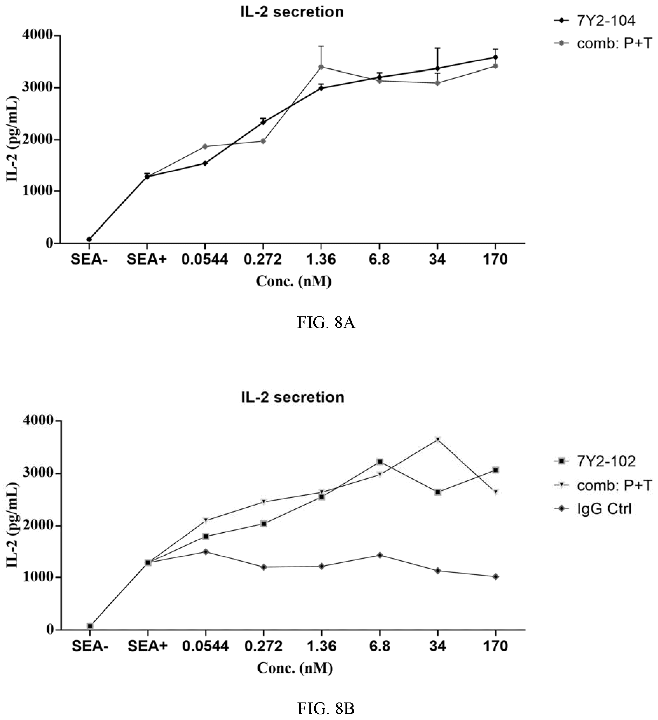

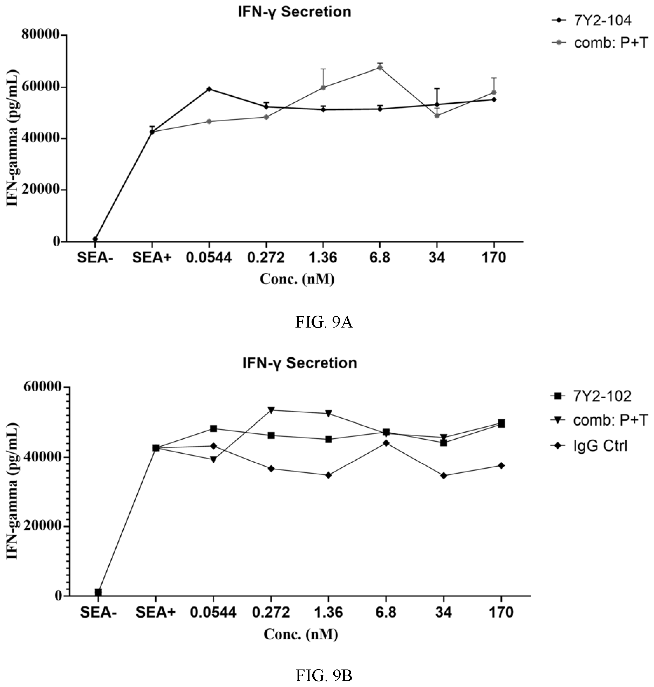

Human IFN-γ and IL-2 levels in the collected culture supernatants were measured using a homogeneous time-resolved fluorescence (HTRF) assay. The experimental procedures were performed according to the kit instructions. The IL-2 assay kit was purchased from Cisbio Bioassays SAS (Cat. No. 62HIL02PEH), and the IFN-γ assay kit was purchased from Cisbio Bioassays SAS (Cat. No. 62HIFNGPEH). The assay results were calculated according to the kit instructions. As shown in FIG. 8A, FIG. 8B, FIG. 9A, and FIG. 9B, with increasing concentrations of the chimeric bispecific fusion proteins, IFN-γ levels did not exhibit a significant trend of change, whereas IL-2 secretion markedly increased, indicating that the chimeric bispecific fusion proteins exhibited an activation effect on human PBMCs.

The mean values obtained at each tested concentration for the samples were compared with the control group (Combo (P + T)) as follows: $Ratio = \left({\sum }_{n = 1}^{∞} \left(\frac{Sample \left(conc. 1\right)}{LCombo \left(conc. 1\right)}+\frac{Sample \left(conc. 2\right)}{LCombo \left(conc. 2\right)}+⋯+\frac{Sample \left(conc. n\right)}{LCombo \left(conc. n\right)}\right)\right) \div n$

The results are shown in Table 14, demonstrating that the chimeric bispecific antibody-cytokine fusion proteins 7Y2-102 and 7Y2-104 exhibited an activation effect on human PBMCs comparable to that of the control group.

**Table 14: Activation results of chimeric bispecific antibody-cytokine fusion proteins on PBMCs**

| Fusion protein name | | 7Y2-102 | 7Y2-104 |
|---|---|---|---|
| PD-1 antibody name | | 1KP-3A6-3C6 | |
| TIGIT antibody name | | 2IT1-10A3-2H6 | 3A03A03 |
| Structure type | | Structure I | |
| PBMC activation ratio: (sample/combo) | IL-2 | 1.000 | 1.009 |
| | IFN-γ | 0.973 | 0.980 |

### Example 10: Long-term toxicity evaluation of chimeric TIGIT/PD-1/IL-15 bispecific antibody-cytokine fusion proteins in mice

PD-1 and TIGIT humanized Balb/c mice aged 4 to 6 weeks were purchased from Jiangsu GemPharmatech Co., Ltd. The mice were housed in an SPF animal facility and allowed to acclimate to the environment for one week. At the start of the experiment, the mice weighed 19 to 25 g were randomly divided into four groups: a 7Y2-102 group, a 7Y2-104 group, a 7Y2-070 group, and a 7Y2-078 group, with six mice per group. The chimeric bispecific antibody-cytokine fusion proteins were administered intraperitoneally twice weekly at the following doses: 3.0 mg/kg for the 7Y2-102 group, 1.5 mg/kg for the 7Y2-070 group, 2.7 mg/kg for the 7Y2-104 group, and 2.0 mg/kg for the 7Y2-078 group. Body weight was measured twice weekly, and general condition of the mice were monitored. After two weeks of dosing, treatment was discontinued, and the mice were observed for an additional week.

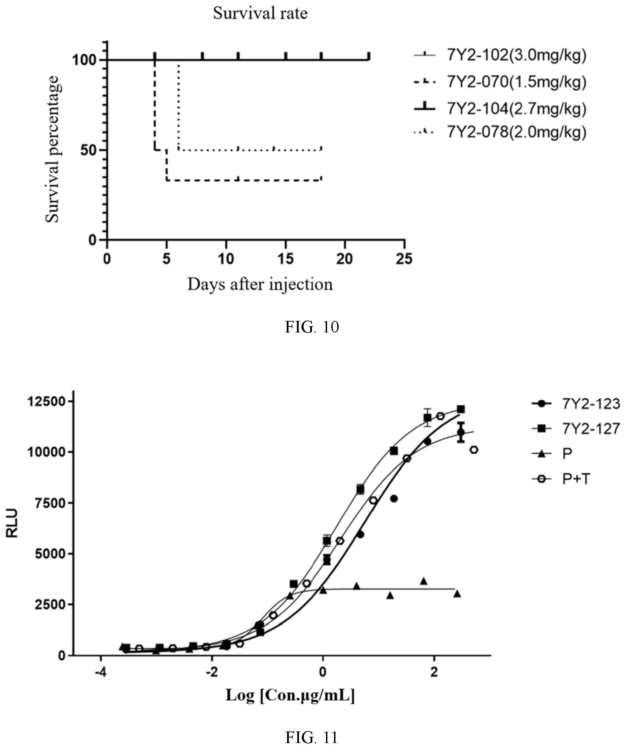

The dosing regimen, time to onset of mortality, and mortality rate at the experimental endpoint were recorded and analyzed for each group of mice. The results are shown in Table 15, and the corresponding survival curves are shown in FIG. 10. For the structure I fusion proteins (7Y2-102 and 7Y2-104), no mice died up to the experimental endpoint despite higher doses. In contrast, the mortality rate was 66.67% for mice in the structure IV fusion protein (7Y2-070) group and 50% for mice in the structure III fusion protein (7Y2-078) group.

**Table 15: Toxicity evaluation of chimeric bispecific antibody-cytokine fusion proteins**

| Fusion protein name | 7Y2-102 | 7Y2-070 | 7Y2-104 | 7Y2-078 |
|---|---|---|---|---|
| PD-1 antibody name | 1KP-3A6-3C6 | | | |
| TIGIT antibody name | 2IT1-10A3-2H6 | | 3A03A03 | |
| Structure type | Structure I | Structure IV | Structure I | Structure III |
| Number of IL-15_IL-15Rα | 1 | 2 | 1 | 2 |
| Dose (mg/kg) | 3.0 | 1.5 | 2.7 | 2.0 |
| Time to onset of mortality (day) | / | 4 | / | 6 |
| Mortality rate (%) | 0 | 66.67 | 0 | 50.00 |

### Example 11: Physicochemical characterization of humanized TIGIT/PD-1/IL-15 bispecific antibody-cytokine fusion proteins

Following the physicochemical characterization method described in Example 4 for the chimeric bispecific antibody-cytokine fusion proteins, the physicochemical properties of the humanized TIGIT/PD-1/IL-15 bispecific antibody-cytokine fusion proteins were evaluated. The assay results are shown in Tables 16 to 18, demonstrating that the humanized bispecific antibody-cytokine fusion proteins listed in the tables exhibited favorable properties, including Tm values, freeze-thaw stability, and high-temperature stability.

**Table 16: Tm values of humanized bispecific antibody-cytokine fusion proteins**

| Fusion protein name | Tm (°C) | | |
|---|---|---|---|
| | Tm1 | Tm2 | Tm3 |
| 7Y2-123 | 68.14 | 82.96 | / |
| 7Y2-126 | 67 | / | / |
| 7Y2-127 | 68.1 | / | / |
| 7Y2-128 | 66.2 | 66.2 | / |
| 7Y2-129 | 58.79 | 70.36 | / |
| 7Y2-130 | 66.49 | 79.46 | / |
| 7Y2-131 | 69.47 | 82.06 | / |
| 7Y2-132 | 68.5 | / | / |
| 7Y2-133 | 70.42 | 86.27 | / |
| 7Y2-134 | 57.83 | 70.21 | / |
| 7Y2-135 | 53.31 | 69.68 | / |
| 7Y2-136 | 58.72 | 70.21 | 85.92 |
| 7Y2-137 | 58.05 | 58.05 | 68.65 |

| | | | |
|---|---|---|---|
| Note: "/" indicates that the antibody structure remained stable without unfolding, and therefore no numerical value was obtained. | | | |

**Table 17: Freeze-thaw stability testing of humanized bispecific antibody-cytokine fusion proteins**

| Fusion protein name | Freeze-thaw stability (SEC, %) | | |
|---|---|---|---|
| | 0 cycles | 2 cycles | 4 cycles |
| 7Y2-120 | 92.42 | 89.78 | 89.47 |
| 7Y2-123 | 96.31 | 95.06 | 94.24 |
| 7Y2-126 | 88.28 | 85.32 | 80.8 |
| 7Y2-127 | 94.98 | 94.83 | 94.76 |
| 7Y2-128 | 92.25 | 91.11 | 90.24 |
| 7Y2-129 | 99.17 | 97.38 | 96.30 |
| 7Y2-132 | 89.83 | 88.23 | 86.79 |
| 7Y2-133 | 96.76 | 96.87 | 95.91 |
| 7Y2-134 | 93.94 | 90.65 | 88.37 |
| 7Y2-135 | 87.82 | 82.47 | 80.41 |
| 7Y2-136 | 99.13 | 97.42 | 96.26 |
| 7Y2-137 | 91.14 | 89.55 | 88.22 |

**Table 18: High-temperature stability testing of humanized bispecific antibody-cytokine fusion proteins**

| Fusion protein name | High-temperature stability at 40°C (SEC, %) | | | |
|---|---|---|---|---|
| | Day 0 | Day 3 | Day 7 | Day 14 |
| 7Y2-123 | 96.31 | 94.11 | 84.00 | 84.26 |
| 7Y2-126 | 88.28 | 80.04 | 77.71 | 60.21 |
| 7Y2-127 | 94.98 | 93.78 | 92.58 | 90.38 |
| 7Y2-128 | 92.27 | 81.41 | 78.92 | ND |
| 7Y2-129 | 99.17 | 94.32 | 90.97 | 88.58 |
| 7Y2-130 | 86.38 | 86.21 | 80.82 | ND |
| 7Y2-132 | 89.83 | 91.34 | 87.12 | 76.44 |
| 7Y2-133 | 96.76 | 92.07 | 88.59 | 90.16 |
| 7Y2-134 | 93.94 | 82.23 | 83.97 | 79.93 |
| 7Y2-135 | 88.57 | 80.1 | 61.59 | ND |
| 7Y2-136 | 99.13 | 92.38 | 91.02 | 87.18 |
| 7Y2-137 | 92.85 | 86.38 | 82.35 | ND |

| | | | | |
|---|---|---|---|---|
| Note: ND indicates no data or not done. | | | | |

### Example 12: Determination of the affinity between humanized TIGIT/PD-1/IL-15 bispecific antibody-cytokine fusion proteins and human TIGIT, cynomolgus monkey TIGIT, human PD-1, or cynomolgus monkey PD-1 antigens

Following the method described in Example 5 for measuring the affinities between the chimeric bispecific antibody-cytokine fusion proteins and human or cynomolgus monkey TIGIT antigens as well as human or cynomolgus monkey PD-1 antigens, the affinities between the humanized bispecific antibody-cytokine fusion proteins and the corresponding target antigens were measured. The results are shown in Table 19 and Table 20, demonstrating that the humanized bispecific antibody-cytokine fusion proteins listed in Tables 19 and 20 exhibited good affinities to human TIGIT, cynomolgus monkey TIGIT, human PD-1, and cynomolgus monkey PD-1 antigens, respectively.

**Table 19: Affinity testing results of humanized bispecific antibody-cytokine fusion proteins to TIGIT antigens**

| Fusion protein name | Affinity to Hu-TIGIT | | | Affinity to Cyno-TIGIT | | |
|---|---|---|---|---|---|---|
| | ka (1/Ms) | kd (1/s) | K_{D} (M) | ka (1/Ms) | kd (1/s) | K_{D} (M) |
| 7Y2-120 | 2.68E+06 | 8.14E-04 | 3.03E-10 | 8.37E+05 | 4.98E-02 | 5.95E-08 |
| 7Y2-123 | 2.53E+06 | 5.88E-04 | 2.32E-10 | 1.56E+06 | 2.06E-02 | 1.32E-08 |
| 7Y2-124 | 1.25E+06 | 5.95E-04 | 4.75E-10 | 1.04E+06 | 5.62E-05 | 5.41E-11 |
| 7Y2-125 | 7.52E+05 | 1.94E-04 | 2.57E-10 | 1.08E+06 | 4.86E-04 | 4.49E-10 |
| 7Y2-126 | 1.49E+06 | 1.42E-03 | 9.54E-10 | 1.69E+06 | 5.82E-04 | 3.45E-10 |
| 7Y2-127 | 5.53E+05 | 2.94E-04 | 5.31E-10 | 1.09E+06 | 3.77E-04 | 3.44E-10 |
| 7Y2-128 | 9.93E+05 | 3.30E-04 | 3.33E-10 | 8.53E+05 | 2.43E-03 | 2.85E-09 |
| 7Y2-129 | 1.53E+06 | 4.85E-04 | 3.16E-10 | 1.24E+06 | 2.08E-03 | 1.68E-09 |
| 7Y2-130 | 8.65E+05 | 4.49E-04 | 5.18E-10 | 9.99E+05 | 2.48E-03 | 2.48E-09 |
| 7Y2-131 | 1.61E+06 | 4.36E-04 | 2.71E-10 | 1.22E+06 | 2.05E-03 | 1.68E-09 |
| 7Y2-132 | 9.83E+05 | 3.78E-04 | 3.85E-10 | 1.38E+06 | 1.75E-04 | 1.28E-10 |
| 7Y2-133 | 1.27E+06 | 1.40E-03 | 1.10E-09 | NA | NA | NA |
| 7Y2-134 | 1.02E+06 | 7.23E-04 | 7.08E-10 | 8.96E+05 | 1.00E-03 | 1.12E-09 |
| 7Y2-135 | 1.14E+06 | 1.22E-03 | 1.07E-09 | 1.38E+06 | 3.95E-04 | 2.87E-10 |
| 7Y2-136 | 5.25E+05 | 6.97E-04 | 1.33E-09 | NA | NA | NA |
| 7Y2-137 | 1.85E+06 | 4.69E-04 | 2.53E-10 | NA | NA | 2.66E-08 |

| | | | | | | |
|---|---|---|---|---|---|---|
| Note: N/A indicates none or not applicable. | | | | | | |

**Table 20: Affinity testing results of humanized bispecific antibody-cytokine fusion proteins to PD-1 antigens**

| Fusion protein name | Affinity to Hu-PD-1 | | | Affinity to Cyno-PD-1 | | |
|---|---|---|---|---|---|---|
| | ka (1/Ms) | kd (1/s) | K_{D} (M) | ka (1/Ms) | kd (1/s) | K_{D} (M) |
| 7Y2-120 | 1.66E+05 | 2.25E-04 | 1.36E-09 | 2.42E+05 | 6.88E-04 | 2.84E-09 |
| 7Y2-123 | 2.09E+05 | 1.70E-04 | 8.15E-10 | 1.88E+05 | 8.16E-04 | 4.33E-09 |
| 7Y2-124 | 9.77E+04 | 3.98E-03 | 4.07E-08 | 1.44E+05 | 1.19E-02 | 8.31E-08 |
| 7Y2-125 | 1.07E+05 | 2.33E-03 | 2.18E-08 | 1.17E+05 | 7.52E-03 | 6.42E-08 |
| 7Y2-126 | 1.66E+05 | 2.09E-04 | 1.26E-09 | 1.50E+05 | 4.05E-04 | 2.69E-09 |
| 7Y2-127 | 2.07E+05 | 1.06E-04 | 5.11E-10 | 2.80E+05 | 1.84E-04 | 6.59E-10 |
| 7Y2-128 | 4.92E+05 | 7.50E-04 | 1.53E-09 | 3.68E+05 | 4.07E-04 | 1.11E-09 |
| 7Y2-129 | 1.00E+05 | 4.52E-04 | 4.51E-09 | 1.08E+05 | 5.24E-04 | 4.86E-09 |
| 7Y2-130 | 5.12E+05 | 1.29E-03 | 2.52E-09 | 3.79E+05 | 3.39E-03 | 8.94E-09 |
| 7Y2-131 | 9.10E+04 | 2.33E-03 | 2.56E-08 | 1.33E+05 | 6.84E-03 | 5.12E-08 |
| 7Y2-132 | 7.99E+04 | 8.40E-04 | 1.05E-08 | 5.70E+04 | 9.07E-04 | 1.59E-08 |
| 7Y2-133 | 1.85E+05 | 1.26E-04 | 6.81E-10 | 1.29E+05 | 5.44E-04 | 4.22E-09 |
| 7Y2-134 | 1.52E+05 | 4.78E-04 | 3.15E-09 | 1.76E+05 | 5.28E-04 | 2.99E-09 |
| 7Y2-135 | 1.24E+05 | 5.05E-04 | 4.07E-09 | 9.51E+04 | 4.27E-04 | 4.49E-09 |
| 7Y2-136 | 9.73E+04 | 4.99E-04 | 5.13E-09 | 1.46E+05 | 7.08E-04 | 4.84E-09 |
| 7Y2-137 | 1.41E+05 | 5.01E-04 | 3.55E-09 | 8.10E+04 | 4.02E-04 | 4.97E-09 |

### Example 13: Binding assay of humanized TIGIT/PD-1/IL-15 bispecific antibody-cytokine fusion proteins to cells expressing human TIGIT, cynomolgus monkey TIGIT, human PD-1, or cynomolgus monkey PD-1 antigens

Following the method described in Example 6 for evaluating the binding ability of fusion proteins to cells overexpressing human TIGIT, cynomolgus monkey TIGIT, human PD-1, or cynomolgus monkey PD-1 antigens, the binding ability of the humanized TIGIT/PD-1/IL-15 antibodies to cells expressing the corresponding target antigens were evaluated. The results are shown in Table 21 and Table 22, demonstrating that the humanized TIGIT/PD-1/IL-15 antibodies listed in Tables 21 and 22 exhibited good binding ability to cells expressing human TIGIT, cynomolgus monkey TIGIT, human PD-1, or cynomolgus monkey PD-1 antigens.

**Table 21: Binding results of humanized bispecific antibody-cytokine fusion proteins to cell surface human or cynomolgus monkey TIGIT antigens**

| Fusion protein name | Binding to Jurkat-huTIGIT cells | | Binding to 293T-cynoTIGIT cells | |
|---|---|---|---|---|
| | MAX MFI | EC₅₀ (nM) | MAX MFI | EC₅₀ (nM) |
| 7Y2-120 | 50516 | 1.26 | 894.5 | 8.8 |
| 7Y2-123 | 34717 | 1.07 | 3087 | 4.89 |
| 7Y2-125 | 44082 | 1.15 | 4326 | 0.37 |
| 7Y2-126 | 48916 | 1.89 | 3546 | 0.71 |
| 7Y2-127 | 42796 | 1.46 | 29047 | 0.88 |
| 7Y2-128 | 42568 | 0.77 | 4342 | 0.64 |
| 7Y2-129 | 43255 | 1.36 | 7341 | 2.01 |
| 7Y2-131 | 26808 | 2.45 | 10619 | 118.74 |
| 7Y2-132 | 43520 | 0.82 | 10274 | 0.82 |
| 7Y2-133 | 30654 | 4.22 | N/A | N/A |
| 7Y2-134 | 27126 | 2.08 | 4889 | 1.01 |
| 7Y2-135 | 57336 | 1.19 | 12700 | 1.82 |
| 7Y2-136 | 17248 | 4.41 | 1337 | 217.88 |
| 7Y2-137 | 51651 | 0.73 | 2956 | 7.54 |

| | | | | |
|---|---|---|---|---|
| Note: N/A indicates none or not applicable. | | | | |

**Table 22: Binding results of humanized bispecific antibody-cytokine fusion proteins to cell surface human or cynomolgus monkey PD-1 antigens**

| Fusion protein name | Binding to CHO-K1-hPD-1 cells | | Binding to CHO-K1-cynoPD-1 cells | |
|---|---|---|---|---|
| | MAX MFI | EC₅₀ (nM) | MAX MFI | EC₅₀ (nM) |
| 7Y2-120 | 6891 | 3.41 | 7704 | 2.39 |
| 7Y2-123 | 33420 | 3.4 | 23510 | 1.16 |
| 7Y2-125 | 2152 | 1.78 | 3290 | 2.3 |
| 7Y2-126 | 6405 | 3.78 | 6573 | 2.27 |
| 7Y2-127 | 51965 | 2.79 | 38781 | 3.15 |
| 7Y2-128 | 39738 | 2.08 | 64815 | 1.74 |
| 7Y2-129 | 48957 | 23.89 | 27130 | 7.21 |
| 7Y2-131 | 2721 | 8.84 | 4870 | 7.64 |
| 7Y2-132 | 36112 | 4.28 | 43192 | 1.65 |
| 7Y2-133 | 5073 | 7.24 | 4809 | 4.67 |
| 7Y2-134 | 5019 | 15.66 | 5238 | 8.86 |
| 7Y2-135 | 34859 | 4.65 | 35496 | 1.4 |
| 7Y2-136 | 4683 | 15.94 | 5580 | 14.61 |
| 7Y2-137 | 39228 | 5.08 | 37020 | 1.28 |

### Example 14: Activation assay of humanized TIGIT/PD-1/IL-15 bispecific antibody-cytokine fusion proteins on reporter cell pairs overexpressing human TIGIT/CD155 and overexpressing human PD-1/PD-L1

Following the method described in Example 7 for evaluating the activation of fusion proteins on reporter cell pairs overexpressing human TIGIT/CD155 and overexpressing human PD-1/PD-L1, respectively, the activation capability of the humanized TIGIT/PD-1/IL-15 bispecific antibody-cytokine fusion proteins on the corresponding reporter cell pairs was evaluated. The results are shown in Table 23, demonstrating that the humanized bispecific antibody-cytokine fusion protein molecules listed in Table 23 exhibited a good activation effect on reporter cells via TCR pathway responses in Jurkat cells mediated by the TIGIT or PD-1 signaling pathways.

**Table 23: Activation results of humanized bispecific antibody-cytokine fusion proteins on reporter cells**

| Fusion protein name | Jurkat-TIGIT-NFAT-Luc | | Jurkat/NFAT-luc/PD1 | |
|---|---|---|---|---|
| | MAX (RLU) | EC₅₀ (nM) | MAX (RLU) | EC₅₀ (nM) |
| 7Y2-120 | 278670 | 107.36 | 1744 | 0.17 |
| 7Y2-123 | 152854 | 61.86 | 1998 | 0.35 |
| 7Y2-125 | 1.203 | 18.54 | 1.563 | 13.2 |
| 7Y2-126 | 281747 | 22.81 | 1658 | 0.4 |
| 7Y2-127 | 200271 | 12.77 | 5616 | 0.72 |
| 7Y2-128 | 156747 | 71.09 | 36609 | 9.04 |
| 7Y2-129 | 119670 | 145.94 | 1977 | 3.89 |
| 7Y2-132 | 738365 | 12.48 | 4047 | 1 |
| 7Y2-133 | N/A | N/A | 2047 | 1.46 |
| 7Y2-134 | 147817 | 19.53 | 4120 | 5.61 |
| 7Y2-135 | 166492 | 13.34 | N/A | N/A |
| 7Y2-137 | 173410 | 116.89 | 3465 | 0.83 |

| | | | | |
|---|---|---|---|---|
| Note: N/A indicates none or not applicable. | | | | |

### Example 15: Activation assay of humanized TIGIT/PD-1/IL-15 bispecific antibody-cytokine fusion proteins on reporter cell pairs co-overexpressing human TIGIT/CD155 and human PD-1/PD-L1

PDL1/CD155/TCR Activator/CHO cells (Nanjing Cobioer Biosciences, CBP74127) in logarithmic growth phase were digested with trypsin, resuspended in fresh F12K medium containing 10% FBS (Shanghai BasalMedia, L450KJ), and adjusted to a density of 4 × 10⁵ cells/mL. The resuspended cells were seeded into a white-walled, clear-bottom 96-well cell culture plate at 100 µL per well, and cultured overnight in an incubator at 37°C. On the following day, the F12K medium in the 96-well plate containing the PDL1/CD155/TCR Activator/CHO cells was aspirated. Samples were then serially diluted in RPMI 1640 medium containing 10% serum. Samples 7Y2-123 and 7Y2-127 were diluted starting from a highest concentration of 600 µg/mL (2× samples). Sample P (Keytruda) was diluted starting from a highest concentration of 512.8 µg/mL (2× samples). For the P + T combination group, sample P was diluted starting from a highest concentration of 684.4 µg/mL (2× samples), and sample T (Tiragolumab) was diluted starting from a highest concentration of 342.2 µg/mL (2× samples). All samples were subjected to 4-fold serial dilutions. The serially diluted 2× samples were added to the seeded cells in the 96-well plate at 50 µL/well. In addition, wells containing medium only were used as blank controls.

Human PD1/TIGIT dual effector reporter cells (Nanjing Cobioer Biosciences, CBP74126) in logarithmic growth phase were centrifuged, and the supernatant was discarded. The cells were resuspended in fresh RPMI 1640 medium containing 10% FBS and adjusted to a density of 4 × 10⁵ cells/mL. Subsequently, The cells were added to the previously prepared 96-well plate containing the fusion proteins and PDL1/CD155/TCR Activator/CHO cells at 50 µL per well, followed by further culture in an incubator at 37°C for 6 hours. The 96-well plate was removed from the incubator, and 100 µL of Bright-Glo^{™} Luciferase Assay Reagent was added to each well. After standing for 3 to 5 minutes, luminescence was measured using a microplate reader. Based on the luminescence values corresponding to each concentration, GraphPad Prism software was used to fit dose-response curves of sample activation on the cells and calculate the half-maximal effective concentration (EC₅₀) of each sample. The results are shown in Table 24 and FIG. 11, demonstrating that 7Y2-123 and 7Y2-127 exhibited an activation effect comparable to that of the combination therapy of the control antibodies Keytruda and Tiragolumab, and were more potent than Keytruda monotherapy.

**Table 24: Activation results of humanized bispecific antibody-cytokine fusion proteins on reporter cells**

| Fusion protein/antibody name | EC₅₀ (µg/mL) | Max signal |
|---|---|---|
| 7Y2-123 | 3.27 | 10975 |
| 7Y2-127 | 1.76 | 12104 |
| Keytruda | 0.079 | 3043 |
| Keytruda + Tiragolumab | 2.1 | 10117 |

### Example 16: Functional assay of humanized TIGIT/PD-1/IL-15 bispecific antibody-cytokine fusion proteins in promoting immune cell proliferation

Following the method described in Example 8 for evaluating the ability of fusion proteins to promote immune cell proliferation, the ability of humanized TIGIT/PD-1/IL-15 bispecific antibody-cytokine fusion proteins to promote immune cell proliferation was evaluated.

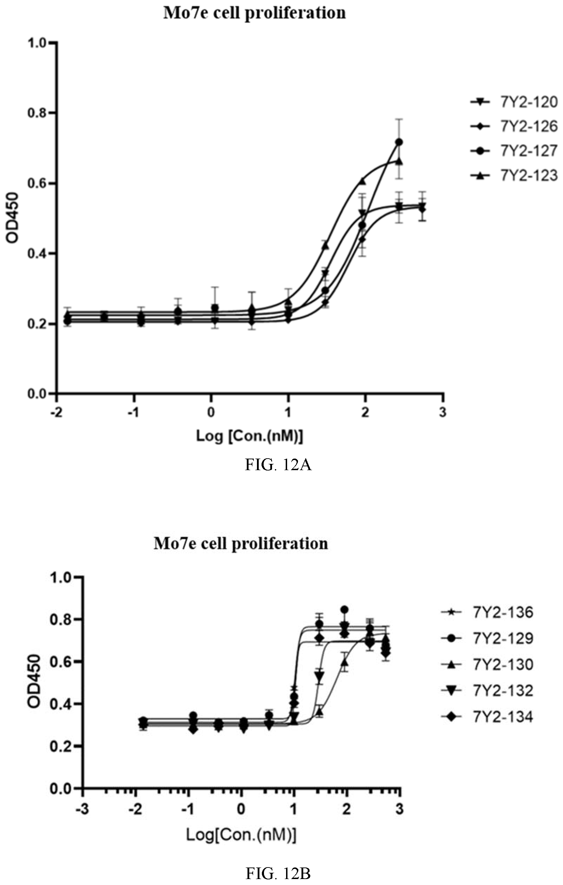

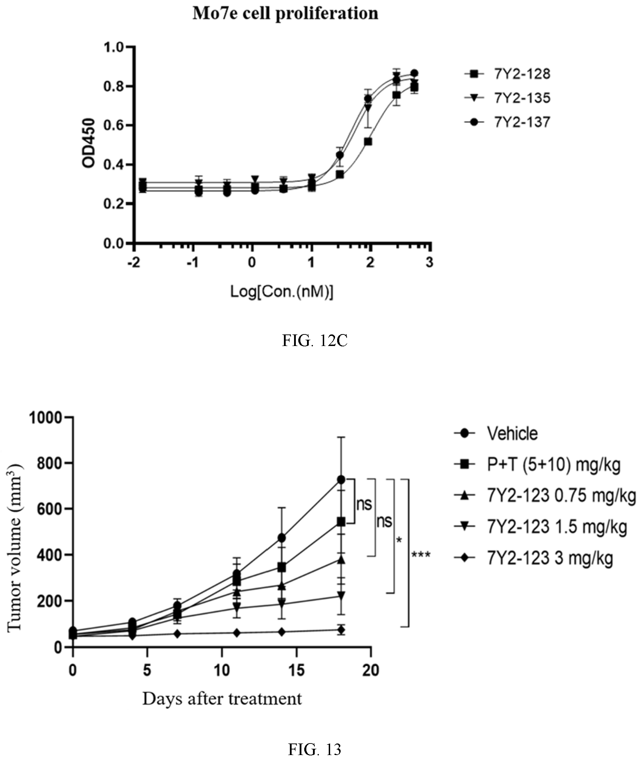

The results are shown in Table 25, FIG. 12A, FIG. 12B, and FIG. 12C, demonstrating that the humanized TIGIT/PD-1/IL-15 bispecific antibody-cytokine fusion proteins shown in the figures were able to stimulate the proliferation of the Mo7e cell line.

**Table 25: Proliferation results of humanized bispecific antibody-cytokine fusion proteins**

| Fusion protein name | Mo7e cell proliferation | |
|---|---|---|
| | EC₅₀ (nM) | Max signal |
| 7Y2-120 | 35.04 | 0.538 |
| 7Y2-123 | 34.93 | 0.6732 |
| 7Y2-126 | 59.92 | 0.534 |
| 7Y2-127 | 65.62 | 0.631 |
| 7Y2-128 | 103.2 | 0.834 |
| 7Y2-129 | 11.01 | 0.765 |
| 7Y2-130 | 64.51 | 0.735 |
| 7Y2-132 | 28.97 | 0.698 |
| 7Y2-134 | 10.64 | 0.694 |
| 7Y2-135 | 55.47 | 0.846 |
| 7Y2-136 | 10.49 | 0.75 |
| 7Y2-137 | 46.08 | 0.868 |

### Example 17: Functional assay of humanized TIGIT/PD-1/IL-15 bispecific antibody-cytokine fusion proteins in activating human PBMCs

Following the method described in Example 9 for evaluating the activation of human PBMCs by fusion proteins, the *in vitro* activation activity of humanized TIGIT/PD-1/IL-15 bispecific antibody-cytokine fusion proteins on peripheral lymphocytes was evaluated. The calculated ratios of IL-2 and IFN-γ secretion levels between the sample groups and the combination therapy control group (Keytruda and Tiragolumab) are shown in Table 26. A ratio greater than 1 indicates that the activation effect of the sample is stronger than that of the control group. The results demonstrated that the humanized bispecific antibody-cytokine fusion proteins listed in the table were able to effectively activate PBMCs.

**Table 26: Activation results of humanized bispecific antibody-cytokine fusion proteins on PBMCs**

| Fusion protein name | PBMC activation: (sample/Keytruda + Tiragolumab) | |
|---|---|---|
| | IL-2 (≥1) | IFN-γ (≥1) |
| 7Y2-123 | 1.125462 | 1.047792 |
| 7Y2-127 | 1.028205 | 1.035334 |
| 7Y2-128 | 1.101005 | 1.100722 |
| 7Y2-129 | 0.934478 | 0.833648 |
| 7Y2-130 | 0.931154 | 0.854587 |
| 7Y2-132 | 1.098781 | 0.968428 |
| 7Y2-134 | 0.877339 | 0.97948 |
| 7Y2-135 | 1.17543 | 1.05864 |
| 7Y2-136 | 0.92578 | 0.971947 |
| 7Y2-137 | 1.161616 | 0.951679 |

### Example 18: In vivo anti-tumor efficacy evaluation of humanized TIGIT/PD-1/IL-15 bispecific antibody-cytokine fusion proteins

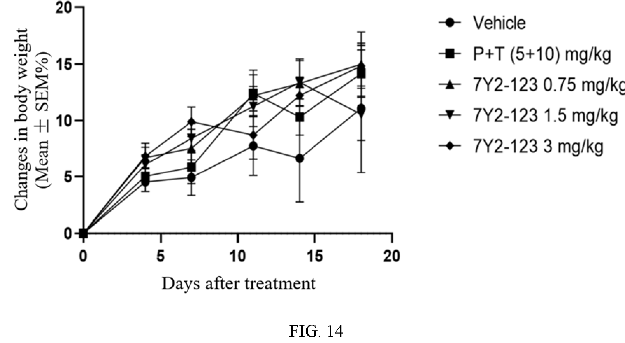

A total of 56 NOG-dKO mice (purchased from Vital River) were housed in an SPF animal facility and allowed to acclimate to the environment for 7 days. During the acclimation period, human bladder cancer 5637 cells (Nanjing Cobioer Biosciences, CBP60309) were thawed and cultured. After the acclimation period, human PBMCs (OriBiotech, ID: Z0406) were injected into the mice via the tail vein at a dose of 5 × 10⁶ cells per mouse. After normal housing for 4 days, 5637 cells were inoculated into the left axilla of the mice at a dose of 5 × 10⁶ cells per mouse. After normal housing for 3 days, tumor size was measured, and the mice were randomly divided into five groups: vehicle group, P + T (Keytruda 5 mg/kg + Tiragolumab 10 mg/kg) group, 7Y2-123_0.75 mg/kg group, 7Y2-123_1.5 mg/kg group, and 7Y2-123_3 mg/kg group. The first three groups contained 10 mice each, and the latter two groups contained 13 mice each. Administration was initiated on Day 6 after tumor cell inoculation. Each group received intraperitoneal injections at the corresponding dosing levels, twice weekly, for a total of 6 doses. The body weight and tumor volume of the mice were monitored twice weekly, for a total observation period of 27 days starting from the first administration. The results are shown in FIG. 13. 7Y2-123 exhibited a dose-dependent tumor inhibitory effect among the groups of different dosing levels, demonstrating good anti-tumor efficacy. Changes in body weight for each group of mice are shown in FIG. 14. During the dosing period, there was no significant difference in body weight change among the groups, indicating that 7Y2-123 exhibits a favorable safety profile.

Although the specific embodiments of the present disclosure have been described in detail, those skilled in the art will understand that various modifications and changes may be made to the details based on all the teachings that have been disclosed, and all such alterations fall within the scope of protection of the present disclosure. The entire scope of the present disclosure is defined by the appended claims and any equivalents thereof.

## Claims

1. A fusion protein, comprising:
a first antigen-binding domain targeting TIGIT,
a second antigen-binding domain targeting PD-1,
an IL-15 polypeptide or a functional fragment thereof, and
an IL-15Rα polypeptide or a functional fragment thereof, wherein:
(i) the first antigen-binding domain comprises a heavy chain variable region (VH) and a light chain variable region (VL) selected from any one of the following groups, wherein
(i-a) a VH comprising the following three CDRs: CDR-H1 comprising the sequence as set forth in SEQ ID NO: 3, CDR-H2 comprising the sequence as set forth in SEQ ID NO: 4, and CDR-H3 comprising the sequence as set forth in SEQ ID NO: 5; and
a VL comprising the following three CDRs: CDR-L1 comprising the sequence as set forth in SEQ ID NO: 6, CDR-L2 comprising the sequence as set forth in SEQ ID NO: 7, and CDR-L3 comprising the sequence as set forth in SEQ ID NO: 8;
or
(i-b) a VH comprising the following three CDRs: CDR-H1 comprising the sequence as set forth in SEQ ID NO: 11, CDR-H2 comprising the sequence as set forth in SEQ ID NO: 12, and CDR-H3 comprising the sequence as set forth in SEQ ID NO: 13; and
a VL comprising the following three CDRs: CDR-L1 comprising the sequence as set forth in SEQ ID NO: 14, CDR-L2 comprising the sequence as set forth in SEQ ID NO: 15, and CDR-L3 comprising the sequence as set forth in SEQ ID NO: 16;
or
(i-c) a VH comprising the following three CDRs: CDR-H1 comprising the sequence as set forth in SEQ ID NO: 19, CDR-H2 comprising the sequence as set forth in SEQ ID NO: 20, and CDR-H3 comprising the sequence as set forth in SEQ ID NO: 21; and
a VL comprising the following three CDRs: CDR-L1 comprising the sequence as set forth in SEQ ID NO: 22, CDR-L2 comprising the sequence as set forth in SEQ ID NO: 23, and CDR-L3 comprising the sequence as set forth in SEQ ID NO: 24;
and/or, (ii) the second antigen-binding domain comprises a VH and a VL selected from any one of the following groups, wherein
(ii-a) a VH comprising the following three CDRs: CDR-H1 comprising the sequence as set forth in SEQ ID NO: 27, CDR-H2 comprising the sequence as set forth in SEQ ID NO: 28, and CDR-H3 comprising the sequence as set forth in SEQ ID NO: 29; and
a VL comprising the following three CDRs: CDR-L1 comprising the sequence as set forth in SEQ ID NO: 30, CDR-L2 comprising the sequence as set forth in SEQ ID NO: 31, and CDR-L3 comprising the sequence as set forth in SEQ ID NO: 32;
or
(ii-b) a VH comprising the following three CDRs: CDR-H1 comprising the sequence as set forth in SEQ ID NO: 35, CDR-H2 comprising the sequence as set forth in SEQ ID NO: 36, and CDR-H3 comprising the sequence as set forth in SEQ ID NO: 37; and
a VL comprising the following three CDRs: CDR-L1 comprising the sequence as set forth in SEQ ID NO: 38, CDR-L2 comprising the sequence as set forth in SEQ ID NO: 39, and CDR-L3 comprising the sequence as set forth in SEQ ID NO: 40.

2. The fusion protein according to claim 1, wherein
(i) the first antigen-binding domain comprises a VH and a VL selected from any one of the following groups, wherein
(i-a) a VH comprising the sequence as set forth in SEQ ID NO: 1 and a VL comprising the sequence as set forth in SEQ ID NO: 2;
(i-b) a VH comprising the sequence as set forth in SEQ ID NO: 9 and a VL comprising the sequence as set forth in SEQ ID NO: 10;
(i-c) a VH comprising the sequence as set forth in SEQ ID NO: 17 and a VL comprising the sequence as set forth in SEQ ID NO: 18;
(i-d) a VH comprising the sequence as set forth in SEQ ID NO: 41 and a VL comprising the sequence as set forth in SEQ ID NO: 42;
(i-e) a VH comprising the sequence as set forth in SEQ ID NO: 43 and a VL comprising the sequence as set forth in SEQ ID NO: 44;
(i-f) a VH comprising the sequence as set forth in SEQ ID NO: 45 and a VL comprising the sequence as set forth in SEQ ID NO: 46;
(i-g) a VH comprising the sequence as set forth in SEQ ID NO: 45 and a VL comprising the sequence as set forth in SEQ ID NO: 47;
(i-h) a VH comprising the sequence as set forth in SEQ ID NO: 48 and a VL comprising the sequence as set forth in SEQ ID NO: 49;
(i-i) a VH comprising the sequence as set forth in SEQ ID NO: 48 and a VL comprising the sequence as set forth in SEQ ID NO: 50;
and/or,
(ii) the second antigen-binding domain comprises a VH and a VL selected from any one of the following groups, wherein
(ii-a) a VH comprising the sequence as set forth in SEQ ID NO: 25 and a VL comprising the sequence as set forth in SEQ ID NO: 26;
(ii-b) a VH comprising the sequence as set forth in SEQ ID NO: 33 and a VL comprising the sequence as set forth in SEQ ID NO: 34;
(ii-c) a VH comprising the sequence as set forth in SEQ ID NO: 54 and a VL comprising the sequence as set forth in SEQ ID NO: 55;
(ii-d) a VH comprising the sequence as set forth in SEQ ID NO: 56 and a VL comprising the sequence as set forth in SEQ ID NO: 57;
(ii-e) a VH comprising the sequence as set forth in SEQ ID NO: 58 and a VL comprising the sequence as set forth in SEQ ID NO: 59;
(ii-f) a VH comprising the sequence as set forth in SEQ ID NO: 56 and a VL comprising the sequence as set forth in SEQ ID NO: 59;
(ii-g) a VH comprising the sequence as set forth in SEQ ID NO: 60 and a VL comprising the sequence as set forth in SEQ ID NO: 57;
(ii-h) a VH comprising the sequence as set forth in SEQ ID NO: 60 and a VL comprising the sequence as set forth in SEQ ID NO: 61;
(ii-i) a VH comprising the sequence as set forth in SEQ ID NO: 62 and a VL comprising the sequence as set forth in SEQ ID NO: 61;
(ii-j) a VH comprising the sequence as set forth in SEQ ID NO: 51 and a VL comprising the sequence as set forth in SEQ ID NO: 52;
(ii-k) a VH comprising the sequence as set forth in SEQ ID NO: 53 and a VL comprising the sequence as set forth in SEQ ID NO: 52;
optionally, for the VH and VL, compared to the VH and VL in any one of groups (i-a) to (i-i) and (ii-a) to (ii-k), the VH has at least 70%, at least 80%, at least 85%, at least 90%, at least 91%, at least 92%, at least 93%, at least 94%, at least 95%, at least 96%, at least 97%, at least 98%, or at least 99% sequence identity; and, the VL has at least 70%, at least 80%, at least 85%, at least 90%, at least 91%, at least 92%, at least 93%, at least 94%, at least 95%, at least 96%, at least 97%, at least 98%, or at least 99% sequence identity;
and/or,
(iii) the IL-15 polypeptide comprises the native amino acid sequence as set forth in SEQ ID NO: 63, or comprises an IL-15 variant that differs from the native amino acid sequence as set forth in SEQ ID NO: 63; preferably, the amino acid sequence of the IL-15 variant has at least one amino acid substitution, deletion, or addition (*e.g.*, 1, 2, 3, 4, or 5 amino acid substitutions, deletions, or additions) compared to the native amino acid sequence as set forth in SEQ ID NO: 63;
and/or, (iv) the IL-15Rα polypeptide comprises the native amino acid sequence as set forth in SEQ ID NO: 64, or comprises an IL-15Rα variant that differs from the native amino acid sequence as set forth in SEQ ID NO: 64; preferably, the amino acid sequence of the IL-15Rα variant has at least one amino acid substitution, deletion, or addition (*e.g.*, 1, 2, 3, 4, or 5 amino acid substitutions, deletions, or additions) compared to the native amino acid sequence as set forth in SEQ ID NO: 64; further preferably, the IL-15Rα variant comprises the sequence as set forth in SEQ ID NO: 75.

3. A fusion protein, comprising: a first antigen-binding domain targeting TIGIT or PD-1, a second antigen-binding domain targeting TIGIT or PD-1, an IL-15 polypeptide or a functional fragment thereof, and an IL-15Rα polypeptide or a functional fragment thereof, wherein the first antigen-binding domain and the second antigen-binding domain respectively bind to different targets; the first antigen-binding domain is a Fab fragment; the second antigen-binding domain is a single-chain antibody; the IL-15 polypeptide and the IL-15Rα polypeptide are located on different peptide chains;
preferably, the first antigen-binding domain is an antigen-binding domain targeting TIGIT; the second antigen-binding domain is an antigen-binding domain targeting PD-1;
preferably, the single-chain antibody is an scFv.

4. The fusion protein according to claim 3, wherein the fusion protein further comprises an Fc domain, the Fc domain comprising a first monomer and a second monomer; wherein:
the N-terminus of the first monomer is optionally linked, via a linker, to one domain of the Fab fragment (*e.g.*, a heavy chain CH1 domain thereof), and the C-terminus of the first monomer is optionally linked, via a linker, to the IL-15 polypeptide or a functional fragment thereof, or to the IL-15Rα polypeptide or a functional fragment thereof; preferably, the N-terminus of the first monomer is optionally linked, via a linker, to the heavy chain CH1 domain of the Fab fragment, and the C-terminus of the first monomer is optionally linked, via a linker, to the IL-15 polypeptide or a functional fragment thereof;
the N-terminus of the second monomer is optionally linked, via a linker, to another domain of the Fab fragment (*e.g.*, a light chain CL domain thereof), and the C-terminus of the second monomer is optionally linked, via a linker, to the IL-15Rα polypeptide or a functional fragment thereof, or to the IL-15 polypeptide or a functional fragment thereof; preferably, the N-terminus of the second monomer is optionally linked, via a linker, to the light chain CL domain of the Fab fragment, and the C-terminus of the second monomer is optionally linked, via a linker, to the IL-15Rα polypeptide or a functional fragment thereof;
preferably, the Fc domain comprises a modification that promotes dimerization of the first monomer and the second monomer;
more preferably, the modification comprises a "knob" modification in one of the first monomer and the second monomer and a "hole" modification in the other of the first monomer and the second monomer, thereby forming a "knob-into-hole" modification.

5. The fusion protein according to claim 3 or 4, wherein
the IL-15 polypeptide comprises the amino acid sequence of the native IL-15 polypeptide of SEQ ID NO: 63, or comprises an IL-15 variant having an amino acid sequence different from that of the native IL-15 polypeptide, wherein the IL-15 variant is still capable of maintaining the function of the native IL-15 polypeptide in promoting effector cell activation; preferably, the amino acid sequence of the IL-15 variant has at least one amino acid substitution, deletion, or addition (*e.g.*, 1, 2, 3, 4, or 5 amino acid substitutions, deletions, or additions) compared to the amino acid sequence of the native IL-15 polypeptide; and/or,
the IL-15Rα polypeptide comprises the amino acid sequence of the native IL-15Rα polypeptide of SEQ ID NO: 64, or comprises an IL-15Rα variant having an amino acid sequence different from that of the native IL-15Rα polypeptide, wherein the IL-15Rα variant is still capable of maintaining the function of the native IL-15Rα polypeptide in promoting effector cell activation; preferably, the amino acid sequence of the IL-15Rα variant has at least one amino acid substitution, deletion, or addition (*e.g.*, 1, 2, 3, 4, or 5 amino acid substitutions, deletions, or additions) compared to the amino acid sequence of the native IL-15Rα polypeptide; further preferably, the IL-15Rα variant comprises the sequence as set forth in SEQ ID NO: 75.

6. The fusion protein according to any one of claims 3 to 5, wherein the fusion protein comprises:
(i) a first peptide chain comprising a VH of the first antigen-binding domain, a heavy chain constant region 1 (CH1), an Fc domain monomer, an IL-15 polypeptide or an IL-15Rα polypeptide, and a single-chain antibody of the second antigen-binding domain; preferably, the CH1 is a human IgG1 heavy chain constant region CH1; preferably, the N-terminus of the Fc domain monomer is linked, via a first hinge region (*e.g.*, a hinge region comprising a PPCP peptide), to the C-terminus of the CH1, and the C-terminus of the Fc domain monomer is linked, via a linker (*e.g.*, a flexible peptide comprising (G4S)n), to the N-terminus of the IL-15 polypeptide or the IL-15Rα polypeptide; preferably, the C-terminus of the IL-15 polypeptide or the IL-15Rα polypeptide is linked, via a linker (*e.g.*, a flexible peptide comprising (G4S)n), to the N-terminus of the single-chain antibody; preferably, the single-chain antibody is an scFv;
and
(ii) a second peptide chain comprising a VL of the first antigen-binding domain, a light chain constant region (CL), an Fc domain monomer, an IL-15 polypeptide or an IL-15Rα polypeptide, and a single-chain antibody of the second antigen-binding domain; preferably, the CL is a human kappa light chain constant region; preferably, the N-terminus of the Fc domain monomer is linked, via a second hinge region (*e.g.*, a hinge region comprising a PPCP peptide), to the C-terminus of the CL, and the C-terminus of the Fc domain monomer is linked, via a linker (*e.g.*, a flexible peptide comprising (G4S)n), to the N-terminus of the IL-15 polypeptide or the IL-15Rα polypeptide; preferably, the C-terminus of the IL-15 polypeptide or the IL-15Rα polypeptide is linked, via a linker (*e.g.*, a flexible peptide comprising (G4S)n), to the N-terminus of the single-chain antibody; preferably, the single-chain antibody is an scFv;
preferably, the Fc domain monomer of the first peptide chain is capable of forming a dimer with the Fc domain monomer of the second peptide chain;
more preferably, the IL-15 polypeptide is located on the first peptide chain, and the IL-15Rα polypeptide is located on the second peptide chain.

7. The fusion protein according to any one of claims 3 to 6, wherein the Fc domain monomer of the first peptide chain and the Fc domain monomer of the second peptide chain comprise a modification that promotes dimerization;
preferably, the modification comprises an amino acid substitution in the CH3 domain of the Fc domain;
preferably, the modification comprises a "knob" modification in one of the two Fc domains and a "hole" modification in the other of the two Fc domains, thereby forming a "knob-into-hole" modification;
preferably, the Fc domain monomer of the first peptide chain comprises the amino acid sequence as set forth in SEQ ID NO: 71;
preferably, the Fc domain monomer of the second peptide chain comprises the amino acid sequence as set forth in SEQ ID NO: 72;
preferably, the two Fc domain monomers comprise the amino acid sequences of SEQ ID NOs: 71 and 72, respectively;
preferably, the CH1 in the first peptide chain comprises the amino acid sequence as set forth in SEQ ID NO: 74, and/or the CL in the second peptide chain comprises the amino acid sequence as set forth in SEQ ID NO: 73;
preferably, the first hinge region comprises the amino acid sequence as set forth in SEQ ID NO: 65, and the second hinge region comprises the amino acid sequence as set forth in SEQ ID NO: 66; and/or
preferably, in the first peptide chain and/or the second peptide chain, the linker connecting the C-terminus of the Fc domain monomer to the N-terminus of the IL-15 polypeptide or the IL-15Rα polypeptide comprises the sequence as set forth in SEQ ID NO: 67;
preferably, in the first peptide chain and/or the second peptide chain, the linker connecting the C-terminus of the IL-15 polypeptide or the IL-15Rα polypeptide to the N-terminus of the single-chain antibody comprises the sequence as set forth in SEQ ID NO: 68.

8. The fusion protein according to any one of claims 3 to 7, wherein the first antigen-binding domain targeting TIGIT comprises a VH and a VL selected from any one of the following groups, wherein
(a) a VH comprising the following three CDRs: CDR-H1 comprising the sequence as set forth in SEQ ID NO: 3, CDR-H2 comprising the sequence as set forth in SEQ ID NO: 4, and CDR-H3 comprising the sequence as set forth in SEQ ID NO: 5; and
a VL comprising the following three CDRs: CDR-L1 comprising the sequence as set forth in SEQ ID NO: 6, CDR-L2 comprising the sequence as set forth in SEQ ID NO: 7, and CDR-L3 comprising the sequence as set forth in SEQ ID NO: 8;
or
(b) a VH comprising the following three CDRs: CDR-H1 comprising the sequence as set forth in SEQ ID NO: 11, CDR-H2 comprising the sequence as set forth in SEQ ID NO: 12, and CDR-H3 comprising the sequence as set forth in SEQ ID NO: 13; and
a VL comprising the following three CDRs: CDR-L1 comprising the sequence as set forth in SEQ ID NO: 14, CDR-L2 comprising the sequence as set forth in SEQ ID NO: 15, and CDR-L3 comprising the sequence as set forth in SEQ ID NO: 16;
or
(c) a VH comprising the following three CDRs: CDR-H1 comprising the sequence as set forth in SEQ ID NO: 19, CDR-H2 comprising the sequence as set forth in SEQ ID NO: 20, and CDR-H3 comprising the sequence as set forth in SEQ ID NO: 21; and
a VL comprising the following three CDRs: CDR-L1 comprising the sequence as set forth in SEQ ID NO: 22, CDR-L2 comprising the sequence as set forth in SEQ ID NO: 23, and CDR-L3 comprising the sequence as set forth in SEQ ID NO: 24;
preferably, the first antigen-binding domain targeting TIGIT comprises a VH and a VL selected from any one of the following groups, wherein
(i-a) a VH comprising the sequence as set forth in SEQ ID NO: 1 and a VL comprising the sequence as set forth in SEQ ID NO: 2;
(i-b) a VH comprising the sequence as set forth in SEQ ID NO: 9 and a VL comprising the sequence as set forth in SEQ ID NO: 10;
(i-c) a VH comprising the sequence as set forth in SEQ ID NO: 17 and a VL comprising the sequence as set forth in SEQ ID NO: 18;
(i-d) a VH comprising the sequence as set forth in SEQ ID NO: 41 and a VL comprising the sequence as set forth in SEQ ID NO: 42;
(i-e) a VH comprising the sequence as set forth in SEQ ID NO: 43 and a VL comprising the sequence as set forth in SEQ ID NO: 44;
(i-f) a VH comprising the sequence as set forth in SEQ ID NO: 45 and a VL comprising the sequence as set forth in SEQ ID NO: 46;
(i-g) a VH comprising the sequence as set forth in SEQ ID NO: 45 and a VL comprising the sequence as set forth in SEQ ID NO: 47;
(i-h) a VH comprising the sequence as set forth in SEQ ID NO: 48 and a VL comprising the sequence as set forth in SEQ ID NO: 49;
(i-i) a VH comprising the sequence as set forth in SEQ ID NO: 48 and a VL comprising the sequence as set forth in SEQ ID NO: 50;
optionally, for the VH and VL, compared to the VH and VL in any one of groups (i-a) to (i-i), the VH has at least 70%, at least 80%, at least 85%, at least 90%, at least 91%, at least 92%, at least 93%, at least 94%, at least 95%, at least 96%, at least 97%, at least 98%, or at least 99% sequence identity; and, the VL has at least 70%, at least 80%, at least 85%, at least 90%, at least 91%, at least 92%, at least 93%, at least 94%, at least 95%, at least 96%, at least 97%, at least 98%, or at least 99% sequence identity.

9. The fusion protein according to any one of claims 3 to 8, wherein the second antigen-binding domain targeting PD-1 comprises a VH and a VL selected from any one of the following groups, wherein
(A) a VH comprising the following three CDRs: CDR-H1 comprising the sequence as set forth in SEQ ID NO: 27, CDR-H2 comprising the sequence as set forth in SEQ ID NO: 28, and CDR-H3 comprising the sequence as set forth in SEQ ID NO: 29; and
a VL comprising the following three CDRs: CDR-L1 comprising the sequence as set forth in SEQ ID NO: 30, CDR-L2 comprising the sequence as set forth in SEQ ID NO: 31, and CDR-L3 comprising the sequence as set forth in SEQ ID NO: 32;
or
(B) a VH comprising the following three CDRs: CDR-H1 comprising the sequence as set forth in SEQ ID NO: 35, CDR-H2 comprising the sequence as set forth in SEQ ID NO: 36, and CDR-H3 comprising the sequence as set forth in SEQ ID NO: 37; and
a VL comprising the following three CDRs: CDR-L1 comprising the sequence as set forth in SEQ ID NO: 38, CDR-L2 comprising the sequence as set forth in SEQ ID NO: 39, and CDR-L3 comprising the sequence as set forth in SEQ ID NO: 40;
preferably, the second antigen-binding domain targeting PD-1 comprises a VH and a VL selected from any one of the following groups, wherein
(ii-a) a VH comprising the sequence as set forth in SEQ ID NO: 25 and a VL comprising the sequence as set forth in SEQ ID NO: 26;
(ii-b) a VH comprising the sequence as set forth in SEQ ID NO: 33 and a VL comprising the sequence as set forth in SEQ ID NO: 34;
(ii-c) a VH comprising the sequence as set forth in SEQ ID NO: 54 and a VL comprising the sequence as set forth in SEQ ID NO: 55;
(ii-d) a VH comprising the sequence as set forth in SEQ ID NO: 56 and a VL comprising the sequence as set forth in SEQ ID NO: 57;
(ii-e) a VH comprising the sequence as set forth in SEQ ID NO: 58 and a VL comprising the sequence as set forth in SEQ ID NO: 59;
(ii-f) a VH comprising the sequence as set forth in SEQ ID NO: 56 and a VL comprising the sequence as set forth in SEQ ID NO: 59;
(ii-g) a VH comprising the sequence as set forth in SEQ ID NO: 60 and a VL comprising the sequence as set forth in SEQ ID NO: 57;
(ii-h) a VH comprising the sequence as set forth in SEQ ID NO: 60 and a VL comprising the sequence as set forth in SEQ ID NO: 61;
(ii-i) a VH comprising the sequence as set forth in SEQ ID NO: 62 and a VL comprising the sequence as set forth in SEQ ID NO: 61;
(ii-j) a VH comprising the sequence as set forth in SEQ ID NO: 51 and a VL comprising the sequence as set forth in SEQ ID NO: 52;
(ii-k) a VH comprising the sequence as set forth in SEQ ID NO: 53 and a VL comprising the sequence as set forth in SEQ ID NO: 52;
optionally, for the VH and VL, compared to the VH and VL in any one of groups (ii-a) to (ii-k), the VH has at least 70%, at least 80%, at least 85%, at least 90%, at least 91%, at least 92%, at least 93%, at least 94%, at least 95%, at least 96%, at least 97%, at least 98%, or at least 99% sequence identity; and, the VL has at least 70%, at least 80%, at least 85%, at least 90%, at least 91%, at least 92%, at least 93%, at least 94%, at least 95%, at least 96%, at least 97%, at least 98%, or at least 99% sequence identity.

10. The fusion protein according to any one of claims 3 to 9, wherein the fusion protein comprises:
(i) a first peptide chain having a structure of [VH1]-[CH1]-[hinge region 1]-[Fc monomer 1]-[L1]-[IL-15]-[L1]-[VL2]-[L2]-[VH2]; and
(ii) a second peptide chain having a structure of [VL1]-[CL]-[hinge region 2]-[Fc monomer 2]-[L1]-[IL-15Rα]-[L1]-[VL2]-[L2]-[VH2];
wherein any one of the following is selected:
(1) the VH1 comprises the sequence as set forth in SEQ ID NO: 9; the VL1 comprises the sequence as set forth in SEQ ID NO: 10; the VH2 comprises the sequence as set forth in SEQ ID NO: 25; the VL2 comprises the sequence as set forth in SEQ ID NO: 26; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 64; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(2) the VH1 comprises the sequence as set forth in SEQ ID NO: 1; the VL1 comprises the sequence as set forth in SEQ ID NO: 2; the VH2 comprises the sequence as set forth in SEQ ID NO: 25; the VL2 comprises the sequence as set forth in SEQ ID NO: 26; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 64; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(3) the VH1 comprises the sequence as set forth in SEQ ID NO: 41; the VL1 comprises the sequence as set forth in SEQ ID NO: 42; the VH2 comprises the sequence as set forth in SEQ ID NO: 54; the VL2 comprises the sequence as set forth in SEQ ID NO: 55; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(4) the VH1 comprises the sequence as set forth in SEQ ID NO: 41; the VL1 comprises the sequence as set forth in SEQ ID NO: 42; the VH2 comprises the sequence as set forth in SEQ ID NO: 56; the VL2 comprises the sequence as set forth in SEQ ID NO: 57; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(5) the VH1 comprises the sequence as set forth in SEQ ID NO: 41; the VL1 comprises the sequence as set forth in SEQ ID NO: 42; the VH2 comprises the sequence as set forth in SEQ ID NO: 62; the VL2 comprises the sequence as set forth in SEQ ID NO: 61; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(6) the VH1 comprises the sequence as set forth in SEQ ID NO: 45; the VL1 comprises the sequence as set forth in SEQ ID NO: 47; the VH2 comprises the sequence as set forth in SEQ ID NO: 51; the VL2 comprises the sequence as set forth in SEQ ID NO: 52; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(7) the VH1 comprises the sequence as set forth in SEQ ID NO: 45; the VL1 comprises the sequence as set forth in SEQ ID NO: 47; the VH2 comprises the sequence as set forth in SEQ ID NO: 58; the VL2 comprises the sequence as set forth in SEQ ID NO: 59; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(8) the VH1 comprises the sequence as set forth in SEQ ID NO: 45; the VL1 comprises the sequence as set forth in SEQ ID NO: 47; the VH2 comprises the sequence as set forth in SEQ ID NO: 56; the VL2 comprises the sequence as set forth in SEQ ID NO: 57; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(9) the VH1 comprises the sequence as set forth in SEQ ID NO: 45; the VL1 comprises the sequence as set forth in SEQ ID NO: 47; the VH2 comprises the sequence as set forth in SEQ ID NO: 56; the VL2 comprises the sequence as set forth in SEQ ID NO: 59; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(10) the VH1 comprises the sequence as set forth in SEQ ID NO: 45; the VL1 comprises the sequence as set forth in SEQ ID NO: 46; the VH2 comprises the sequence as set forth in SEQ ID NO: 60; the VL2 comprises the sequence as set forth in SEQ ID NO: 57; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(11) the VH1 comprises the sequence as set forth in SEQ ID NO: 45; the VL1 comprises the sequence as set forth in SEQ ID NO: 47; the VH2 comprises the sequence as set forth in SEQ ID NO: 62; the VL2 comprises the sequence as set forth in SEQ ID NO: 61; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(12) the VH1 comprises the sequence as set forth in SEQ ID NO: 48; the VL1 comprises the sequence as set forth in SEQ ID NO: 50; the VH2 comprises the sequence as set forth in SEQ ID NO: 60; the VL2 comprises the sequence as set forth in SEQ ID NO: 57; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(13) the VH1 comprises the sequence as set forth in SEQ ID NO: 48; the VL1 comprises the sequence as set forth in SEQ ID NO: 50; the VH2 comprises the sequence as set forth in SEQ ID NO: 51; the VL2 comprises the sequence as set forth in SEQ ID NO: 52; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(14) the VH1 comprises the sequence as set forth in SEQ ID NO: 48; the VL1 comprises the sequence as set forth in SEQ ID NO: 49; the VH2 comprises the sequence as set forth in SEQ ID NO: 60; the VL2 comprises the sequence as set forth in SEQ ID NO: 61; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(15) the VH1 comprises the sequence as set forth in SEQ ID NO: 48; the VL1 comprises the sequence as set forth in SEQ ID NO: 49; the VH2 comprises the sequence as set forth in SEQ ID NO: 53; the VL2 comprises the sequence as set forth in SEQ ID NO: 52; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(16) the VH1 comprises the sequence as set forth in SEQ ID NO: 43; the VL1 comprises the sequence as set forth in SEQ ID NO: 44; the VH2 comprises the sequence as set forth in SEQ ID NO: 60; the VL2 comprises the sequence as set forth in SEQ ID NO: 57; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(17) the VH1 comprises the sequence as set forth in SEQ ID NO: 43; the VL1 comprises the sequence as set forth in SEQ ID NO: 44; the VH2 comprises the sequence as set forth in SEQ ID NO: 60; the VL2 comprises the sequence as set forth in SEQ ID NO: 61; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66;
(18) the VH1 comprises the sequence as set forth in SEQ ID NO: 45; the VL1 comprises the sequence as set forth in SEQ ID NO: 46; the VH2 comprises the sequence as set forth in SEQ ID NO: 53; the VL2 comprises the sequence as set forth in SEQ ID NO: 52; the CL comprises the sequence as set forth in SEQ ID NO: 73; the CH1 comprises the sequence as set forth in SEQ ID NO: 74; the Fc monomer 1 comprises the sequence as set forth in SEQ ID NO: 71; the Fc monomer 2 comprises the sequence as set forth in SEQ ID NO: 72; the IL-15 comprises the sequence as set forth in SEQ ID NO: 63; the IL-15Rα comprises the sequence as set forth in SEQ ID NO: 75; the L1 and the L2 are peptide linkers, preferably each independently selected from peptide linkers comprising one or more glycine residues and/or one or more serine residues (*e.g.*, peptide linkers represented by (G4S)n); preferably, the L1 is the sequence as set forth in SEQ ID NO: 67, and the L2 is the sequence as set forth in SEQ ID NO: 68; the hinge region 1 and the hinge region 2 are peptide hinge regions, preferably each independently selected from peptide hinge regions comprising PPCP; preferably, the hinge region 1 is the sequence as set forth in SEQ ID NO: 65, and the hinge region 2 is the sequence as set forth in SEQ ID NO: 66.

11. An isolated nucleic acid molecule encoding the fusion protein according to any one of claims 1 to 10.

12. A vector comprising the isolated nucleic acid molecule according to claim 11;
preferably, the vector comprises nucleotide sequences encoding respective peptide chains of the fusion protein, and the nucleotide sequences encoding respective peptide chains are present in the same vector or in different vectors.

13. A host cell comprising the isolated nucleic acid molecule according to claim 11 or the vector according to claim 12.

14. A method of preparing a fusion protein, comprising culturing the host cell according to claim 13 under conditions that allow protein expression, and collecting the fusion protein from the culture of the cultured host cell.

15. A conjugate comprising the fusion protein according to any one of claims 1 to 10, or the isolated nucleic acid molecule according to claim 11, and a conjugation moiety linked thereto;
preferably, the conjugation moiety is a detectable label (*e.g.*, a radioisotope, a fluorescent substance, a luminescent substance, a chromogenic substance, or an enzyme) or a therapeutic agent (*e.g.*, a cytotoxic agent, a cytokine, a toxin, a radionuclide, an immunostimulant, an immunosuppressant, and other active substances that inhibit tumor cell growth or promote tumor cell apoptosis or necrosis).

16. A pharmaceutical composition comprising the fusion protein according to any one of claims 1 to 10, the isolated nucleic acid molecule according to claim 11, the vector according to claim 12, the host cell according to claim 13, or the conjugate according to claim 15, and a pharmaceutically acceptable carrier and/or excipient.

17. A kit comprising the fusion protein according to any one of claims 1 to 10, the isolated nucleic acid molecule according to claim 11, the vector according to claim 12, the host cell according to claim 13, the conjugate according to claim 15, or the pharmaceutical composition according to claim 16.

18. Use of the fusion protein according to any one of claims 1 to 10, the isolated nucleic acid molecule according to claim 11, the vector according to claim 12, the host cell according to claim 13, the conjugate according to claim 15, or the pharmaceutical composition according to claim 16, in the manufacture of a medicament for the prevention and/or treatment and/or neoadjuvant treatment and/or adjuvant treatment of a disease.

19. A method for the prevention and/or treatment and/or neoadjuvant treatment and/or adjuvant treatment of a disease in a subject, comprising administering to the subject in need thereof an effective amount of the fusion protein according to any one of claims 1 to 10, the isolated nucleic acid molecule according to claim 11, the vector according to claim 12, the host cell according to claim 13, the conjugate according to claim 15, or the pharmaceutical composition according to claim 16.

20. The use according to claim 18 or the method according to claim 19, wherein the disease is a tumor;
preferably, the tumor is a solid tumor or a hematological tumor;
more preferably, the solid tumor is melanoma, lung cancer, renal cancer, bladder cancer, head and neck tumor, gastric cancer, esophageal cancer, liver cancer, cervical cancer, breast cancer, or skin cancer.

21. The fusion protein according to any one of claims 1 to 10, the isolated nucleic acid molecule according to claim 11, the vector according to claim 12, the host cell according to claim 13, the conjugate according to claim 15, or the pharmaceutical composition according to claim 16, for use in the prevention and/or treatment and/or neoadjuvant treatment and/or adjuvant treatment of a disease in a subject;
preferably, the disease is a tumor;
more preferably, the tumor is a solid tumor or a hematological tumor;
further preferably, the solid tumor is melanoma, lung cancer, renal cancer, bladder cancer, head and neck tumor, gastric cancer, esophageal cancer, liver cancer, cervical cancer, breast cancer, or skin cancer.
